# EUROPEAN PATENT SPECIFICATION

(11) **EP 3 003 352 B1**
(45) Date of publication and mention of the grant of the patent: **03.07.2019**
(21) Application number: 14731168.2
(22) Date of filing: 02.06.2014
(51) Int. Cl.: A61K 38/53, A61K 38/17, C07K 14/47, C12N 15/113, C12N 9/00

(54) **PROTEIN INVOLVED IN DNA REPLICATION, AND MODULATION OF ITS ACTIVITY**
IN DER DNA-REPLIKATION BETEILIGTES PROTEIN UND MODULATION SEINER AKTIVITÄT
PROTÉINE IMPLIQUÉE DANS LA RÉPLICATION DE L'ADN ET MODULATION DE SON ACTIVITÉ

(30) Priority: 31.05.2013 EP 13305725
(43) Date of publication of application: 13.04.2016
(73) Proprietor: Centre National de la Recherche Scientifique (C.N.R.S.), 75794 Paris Cedex 16 (FR)
(72) Inventor: MECHALI, Marcel, 34980 Montferrier Sur Lez (FR); PHILIPPE, Coulombe, 34080 Montpellier (FR)
(74) Representative: Monni, Richard
(86) International application number: PCT/EP2014/061369
(87) International publication number: WO 2014/191575

(56) References cited:
- WO-A1-01/55381
- WO-A2-03/047526
- Z. SHEN ET AL: "Dynamic Association of ORCA with Prereplicative Complex Components Regulates DNA Replication Initiation", MOLECULAR AND CELLULAR BIOLOGY, vol. 32, no. 15, 1 August 2012 (2012-08-01), pages 3107-3120, XP055079883, ISSN: 0270-7306, DOI: 10.1128/MCB.00362-12
- ZHEN SHEN ET AL: "A WD-Repeat Protein Stabilizes ORC Binding to Chromatin", MOLECULAR CELL, vol. 40, no. 1, 1 October 2010 (2010-10-01), pages 99-111, XP055080438, ISSN: 1097-2765, DOI: 10.1016/j.molcel.2010.09.021
- SONJA A DE MUNNIK ET AL: "Meier-Gorlin syndrome genotype-phenotype studies: 35 individuals with pre-replication complex gene mutations and 10 without molecular diagnosis", EUROPEAN JOURNAL OF HUMAN GENETICS, vol. 20, no. 6, 1 June 2012 (2012-06-01), pages 598-606, XP055080076, ISSN: 1018-4813, DOI: 10.1038/ejhg.2011.269
- A. KLINGSEISEN ET AL: "Mechanisms and pathways of growth failure in primordial dwarfism", GENES & DEVELOPMENT, vol. 25, no. 19, 1 October 2011 (2011-10-01), pages 2011-2024, XP055080071, ISSN: 0890-9369, DOI: 10.1101/gad.169037
- HELEN WALDEN ET AL: "Regulation of Parkin E3 ubiquitin ligase activity", CMLS CELLULAR AND MOLECULAR LIFE SCIENCES, BIRKHÄUSER-VERLAG, BA, vol. 69, no. 18, 19 April 2012 (2012-04-19), pages 3053-3067, XP035103750, ISSN: 1420-9071, DOI: 10.1007/S00018-012-0978-5
- L. JIA ET AL: "RBX1 (RING Box Protein 1) E3 Ubiquitin Ligase Is Required for Genomic Integrity by Modulating DNA Replication Licensing Proteins", JOURNAL OF BIOLOGICAL CHEMISTRY, vol. 286, no. 5, 4 February 2011 (2011-02-04), pages 3379-3386, XP055080439, ISSN: 0021-9258, DOI: 10.1074/jbc.M110.188425
- HELLE D. ULRICH ET AL: "Ubiquitin signalling in DNA replication and repair", NATURE REVIEWS MOLECULAR CELL BIOLOGY, vol. 11, no. 7, 16 June 2010 (2010-06-16), pages 479-489, XP055080463, ISSN: 1471-0072, DOI: 10.1038/nrm2921
- DATABASE UniProt [Online] 7 December 2004 (2004-12-07), "RecName: Full=RING finger protein 219;", XP002713423, retrieved from EBI accession no. UNIPROT:Q5W0B1 Database accession no. Q5W0B1
- DEREKF CECCARELLI ET AL: "An Allosteric Inhibitor of the Human Cdc34Ubiquitin-Conjugating Enzyme", CELL, CELL PRESS, US, vol. 145, no. 7, 31 May 2011 (2011-05-31), pages 1075-1087, XP028230595, ISSN: 0092-8674, DOI: 10.1016/J.CELL.2011.05.039 [retrieved on 2011-06-07]

### SEQUENCE LISTING

<110> CENTRE NATIONAL DE LA RECHERCHE SCIENTIFIQUE MECHALI, Marcel COULOMBE, Philippe
<120> Protein involved in DNA replication, and modulation of its activity
<130> BR71245
<150> EP 13305725.7
   <151> 2013-05-31
<160> 109
<170> PatentIn version 3.5
<210> 1
   <211> 726
   <212> PRT
   <213> Homo sapiens
<400> 1
   Met Ala Gln Thr Val Gln Asn Val Thr Leu Ser Leu Thr Leu Pro Ile
   1               5                   10                  15
   Thr Cys His Ile Cys Leu Gly Lys Val Arg Gln Pro Val Ile Cys Ile
               20                  25                  30
   Asn Asn His Val Phe Cys Ser Ile Cys Ile Asp Leu Trp Leu Lys Asn
           35                  40                  45
   Asn Ser Gln Cys Pro Ala Cys Arg Val Pro Ile Thr Pro Glu Asn Pro
       50                  55                  60
   Cys Lys Glu Ile Ile Gly Gly Thr Ser Glu Ser Glu Pro Met Leu Ser
   65                  70                  75                  80
   His Thr Val Arg Lys His Leu Arg Lys Thr Arg Leu Glu Leu Leu His
                   85                  90                  95
   Lys Glu Tyr Glu Asp Glu Ile Asp Cys Leu Gln Lys Glu Val Glu Glu
               100                 105                 110
   Leu Lys Ser Lys Asn Leu Ser Leu Glu Ser Gln Ile Lys Thr Ile Leu
           115                 120                 125
   Asp Pro Leu Thr Leu Val Gln Gly Asn Gln Asn Glu Asp Lys His Leu
       130                 135                 140
   Val Thr Asp Asn Pro Ser Lys Ile Asn Pro Glu Thr Val Ala Glu Trp
   145                 150                 155                 160
   Lys Lys Lys Leu Arg Thr Ala Asn Glu Ile Tyr Glu Lys Val Lys Asp
   165 170 175
   Asp Val Asp Lys Leu Lys Glu Ala Asn Lys Lys Leu Lys Leu Glu Asn
   180 185 190
   Gly Gly Leu Val Arg Glu Asn Leu Arg Leu Lys Ala Glu Val Asp Asn
   195 200 205
   Arg Ser Pro Gln Lys Phe Gly Arg Phe Ala Val Ala Ala Leu Gln Ser
   210 215 220
   Lys Val Glu Gln Tyr Glu Arg Glu Thr Asn Arg Leu Lys Lys Ala Leu
   225 230 235 240
   Glu Arg Ser Asp Lys Tyr Ile Glu Glu Leu Glu Ser Gln Val Ala Gln
   245 250 255
   Leu Lys Asn Ser Ser Glu Glu Lys Glu Ala Met Asn Ser Ile Cys Gln
   260 265 270
   Thr Ala Leu Ser Ala Asp Gly Lys Gly Ser Lys Gly Ser Glu Glu Asp
   275 280 285
   Val Val Ser Lys Asn Gln Gly Asp Ser Ala Arg Lys Gln Pro Gly Ser
   290 295 300
   Ser Thr Ser Ser Ser Ser His Leu Ala Lys Pro Ser Ser Ser Arg Leu
   305 310 315 320
   Cys Asp Thr Ser Ser Ala Arg Gln Glu Ser Thr Ser Lys Ala Asp Leu
   325 330 335
   Asn Cys Ser Lys Asn Lys Asp Leu Tyr Gln Glu Gln Val Glu Val Met
   340 345 350
   Leu Asp Val Thr Asp Thr Ser Met Asp Thr Tyr Leu Glu Arg Glu Trp
   355 360 365
   Gly Asn Lys Pro Ser Asp Cys Val Pro Tyr Lys Asp Glu Glu Leu Tyr
   370 375 380
   Asp Leu Pro Ala Pro Cys Thr Pro Leu Ser Leu Ser Cys Leu Gln Leu
   385 390 395 400
   Ser Thr Pro Glu Asn Arg Glu Ser Ser Val Val Gln Ala Gly Gly Ser
                   405                 410                 415
   Lys Lys His Ser Asn His Leu Arg Lys Leu Val Phe Asp Asp Phe Cys
               420                 425                 430
   Asp Ser Ser Asn Val Ser Asn Lys Asp Ser Ser Glu Asp Asp Ile Ser
           435                 440                 445
   Arg Ser Glu Asn Glu Lys Lys Ser Glu Cys Phe Ser Ser Pro Lys Thr
       450                 455                 460
   Gly Phe Trp Asp Cys Cys Ser Thr Ser Tyr Ala Gln Asn Leu Asp Phe
   465                 470                 475                 480
   Glu Ser Ser Glu Gly Asn Thr Ile Ala Asn Ser Val Gly Glu Ile Ser
                   485                 490                 495
   Ser Lys Leu Ser Glu Lys Ser Gly Leu Cys Leu Ser Lys Arg Leu Asn
               500                 505                 510
   Ser Ile Arg Ser Phe Glu Met Asn Arg Thr Arg Thr Ser Ser Glu Ala
           515                 520                 525
   Ser Met Asp Ala Ala Tyr Leu Asp Lys Ile Ser Glu Leu Asp Ser Met
       530                 535                 540
   Met Ser Glu Ser Asp Asn Ser Lys Ser Pro Cys Asn Asn Gly Phe Lys
   545                 550                 555                 560
   Ser Leu Asp Leu Asp Gly Leu Ser Lys Ser Ser Gln Gly Ser Glu Phe
                   565                 570                 575
   Leu Glu Glu Pro Asp Lys Leu Glu Glu Lys Thr Glu Leu Asn Leu Ser
               580                 585                 590
   Lys Gly Ser Leu Thr Asn Asp Gln Leu Glu Asn Gly Ser Glu Trp Lys
           595                 600                 605
   Pro Thr Ser Phe Phe Leu Leu Ser Pro Ser Asp Gln Glu Met Asn Glu
       610                 615                 620
   Asp Phe Ser Leu His Ser Ser Ser Cys Pro Val Thr Asn Glu Ile Lys
   625                 630                 635                 640
   Pro Pro Ser Cys Leu Phe Gln Thr Glu Phe Ser Gln Gly Ile Leu Leu
                   645                 650                 655
   Ser Ser Ser His Arg Leu Phe Glu Asp Gln Arg Phe Gly Ser Ser Leu
   660 665 670
   Phe Lys Met Ser Ser Glu Met His Ser Leu His Asn His Leu Gln Ser
   675 680 685
   Pro Trp Ser Thr Ser Phe Val Pro Glu Lys Arg Asn Lys Asn Val Asn
   690 695 700
   Gln Ser Thr Lys Arg Lys Ile Gln Ser Ser Leu Ser Ser Ala Ser Pro
   705 710 715 720
   Ser Lys Ala Thr Lys Ser
   725
<210> 2
   <211> 726
   <212> PRT
   <213> Pan troglodytes
<400> 2
   Met Ala Gln Thr Val Gln Asn Val Thr Leu Ser Leu Thr Leu Pro Ile
   1 5 10 15
   Thr Cys His Ile Cys Leu Gly Lys Val Arg Gln Pro Val Ile Cys Ile
   20 25 30
   Asn Asn His Val Phe Cys Ser Ile Cys Ile Asp Leu Trp Leu Lys Asn
   35 40 45
   Asn Ser Gln Cys Pro Ala Cys Arg Val Pro Ile Thr Pro Glu Asn Pro
   50 55 60
   Cys Lys Glu Ile Ile Gly Gly Thr Ser Glu Ser Glu Pro Met Leu Ser
   65 70 75 80
   His Thr Val Arg Lys His Leu Arg Lys Thr Arg Leu Glu Leu Leu His
   85 90 95
   Lys Glu Tyr Glu Asp Glu Ile Asp Cys Leu Gln Lys Glu Val Glu Glu
   100 105 110
   Leu Lys Ser Lys Asn Leu Ser Leu Glu Ser Gln Ile Lys Thr Ile Leu
   115 120 125
   Asp Pro Leu Thr Leu Val Gln Gly Asn Gln Asn Glu Asp Lys His Leu
   130 135 140
   Val Thr Asp Asn Pro Ser Lys Ile Asn Pro Glu Thr Val Ala Glu Trp
   145                 150                 155                 160
   Lys Lys Lys Leu Arg Thr Ala Asn Glu Ile Tyr Glu Lys Val Lys Asp
                   165                 170                 175
   Asp Val Asp Lys Leu Lys Glu Ala Asn Lys Lys Leu Lys Leu Glu Asn
               180                 185                 190
   Gly Gly Leu Val Arg Glu Asn Leu Arg Leu Lys Ala Glu Val Asp Asn
           195                 200                 205
   Arg Ser Pro Gln Lys Phe Gly Arg Phe Ala Val Ala Ala Leu Gln Ser
       210                 215                 220
   Lys Val Glu Gln Tyr Glu Arg Glu Thr Asn Arg Leu Lys Lys Ala Leu
   225                 230                 235                 240
   Glu Arg Ser Asp Lys Tyr Ile Glu Glu Leu Glu Ser Gln Val Ala Gln
                   245                 250                 255
   Leu Lys Asn Ser Ser Glu Glu Lys Glu Ala Met Asn Ser Ile Cys Lys
               260                 265                 270
   Thr Ala Leu Ser Ala Asp Gly Lys Gly Ser Lys Gly Ser Glu Glu Asp
           275                 280                 285
   Val Ala Ser Lys Asn Gln Gly Asp Ser Ala Arg Lys Gln Pro Gly Ser
       290                 295                 300
   Ser Thr Ser Ser Ser Ser His Leu Ala Lys Pro Ser Ser Ser Arg Leu
   305                 310                 315                 320
   Cys Asp Thr Ser Ser Ala Arg Gln Glu Ser Thr Ser Lys Ala Asp Leu
                   325                 330                 335
   Asn Cys Ser Lys Asn Lys Asp Leu Tyr Gln Glu Gln Val Glu Val Met
               340                 345                 350
   Leu Asp Val Thr Asp Thr Ser Met Asp Thr Tyr Leu Glu Arg Glu Trp
           355                 360                 365
   Gly Asn Lys Pro Ser Asp Cys Val Pro Tyr Lys Asp Glu Glu Leu Tyr
       370                 375                 380
   Asp Leu Pro Ala Pro Cys Thr Pro Leu Ser Leu Ser Cys Leu Gln Leu
   385                 390                 395                 400
   Ser Thr Pro Glu Asn Arg Glu Ser Ser Val Val Gln Ala Gly Gly Ser
                   405                 410                 415
   Lys Lys His Ser Asn His Leu Arg Lys Leu Val Phe Asp Asp Phe Cys
               420                 425                 430
   Asp Ser Ser Asn Val Ser Asn Lys Asp Ser Ser Glu Asp Asp Ile Ser
           435                 440                 445
   Arg Ser Glu Asn Glu Lys Lys Ser Glu Cys Phe Ser Ser Pro Lys Thr
       450                 455                 460
   Gly Phe Trp Asp Cys Cys Ser Thr Ser Tyr Ala Gln Asn Leu Asp Phe
   465                 470                 475                 480
   Glu Ser Ser Glu Gly Asn Thr Ile Ala Asn Ser Val Gly Glu Ile Ser
                   485                 490                 495
   Ser Lys Leu Ser Glu Lys Ser Gly Leu Cys Leu Ser Lys Arg Leu Asn
               500                 505                 510
   Ser Ile Arg Ser Phe Glu Met Asn Arg Thr Arg Thr Ser Ser Glu Ala
           515                 520                 525
   Ser Met Asp Ala Ala Tyr Leu Asp Lys Ile Ser Glu Leu Asp Ser Met
       530                 535                 540
   Met Ser Glu Ser Asp Asn Ser Lys Ser Pro Cys Asn Asn Gly Phe Lys
   545                 550                 555                 560
   Ser Leu Asp Leu Asp Gly Leu Ser Lys Ser Ser Gln Gly Ser Glu Phe
                   565                 570                 575
   Leu Glu Glu Pro Asp Lys Leu Glu Glu Lys Thr Glu Leu Asn Leu Ser
               580                 585                 590
   Lys Gly Ser Leu Thr Asn Asp Gln Leu Glu Asn Gly Ser Glu Trp Lys
           595                 600                 605
   Pro Thr Ser Phe Phe Leu Leu Ser Pro Ser Asp Gln Glu Met Asn Glu
       610                 615                 620
   Asp Phe Ser Leu His Ser Ser Ser Cys Pro Val Thr Asn Glu Ile Lys
   625                 630                 635                 640
   Pro Pro Ser Cys Leu Phe Gln Thr Glu Phe Ser Gln Gly Ile Leu Leu
                   645                 650                 655
   Ser Ser Ser His Arg Leu Phe Glu Asp Gln Arg Phe Gly Ser Ser Leu
               660                 665                 670
   Phe Lys Met Ser Ser Glu Met His Ser Leu His Asn His Leu Gln Ser
           675                 680                 685
   Pro Trp Ser Thr Ser Phe Val Pro Glu Lys Arg Asn Lys Asn Val Asn
       690                 695                 700
   Gln Ser Thr Lys Arg Lys Ile Gln Ser Ser Leu Ser Asn Ala Ser Pro
   705                 710                 715                 720
   Ser Lys Ala Thr Lys Ser
                   725
<210> 3
   <211> 726
   <212> PRT
   <213> Nomascus leucogenys
<400> 3
   Met Ala Gln Thr Val Gln Asn Val Thr Leu Ser Leu Thr Leu Pro Ile
   1               5                   10                  15
   Thr Cys His Ile Cys Leu Gly Lys Val Arg Gln Pro Val Ile Cys Ile
               20                  25                  30
   Asn Asn His Val Phe Cys Ser Ile Cys Ile Asp Leu Trp Leu Lys Asn
           35                  40                  45
   Asn Ser Gln Cys Pro Ala Cys Arg Val Pro Ile Thr Pro Glu Asn Pro
       50                  55                  60
   Cys Lys Glu Ile Ile Gly Gly Thr Ser Glu Ser Glu Pro Met Leu Ser
   65                  70                  75                  80
   His Thr Val Arg Lys His Leu Arg Lys Thr Arg Leu Glu Leu Leu His
                   85                  90                  95
   Lys Glu Tyr Glu Asp Glu Ile Asp Cys Leu Gln Lys Glu Val Glu Glu
               100                 105                 110
   Leu Lys Ser Lys Asn Leu Ser Leu Glu Ser Gln Ile Lys Thr Ile Leu
           115                 120                 125
   Asp Pro Leu Thr Leu Val Gln Gly Asn Gln Asn Glu Asp Lys His Leu
       130                 135                 140
   Val Thr Asp Asn Pro Ser Lys Ile Asn Pro Glu Thr Val Ala Glu Trp
   145 150 155 160
   Lys Lys Lys Leu Arg Thr Ala Asn Glu Ile Tyr Glu Lys Val Lys Asp
   165 170 175
   Asp Val Asp Lys Leu Lys Glu Ala Asn Lys Lys Leu Lys Leu Glu Asn
   180 185 190
   Gly Gly Leu Val Arg Glu Asn Leu Arg Leu Lys Ala Glu Val Asp Asn
   195 200 205
   Arg Ser Pro Gln Lys Phe Gly Arg Phe Ala Val Ala Ala Leu Gln Ser
   210 215 220
   Lys Val Glu Gln Tyr Glu Arg Glu Thr Asn Arg Leu Lys Lys Ala Leu
   225 230 235 240
   Glu Arg Ser Asp Lys Tyr Ile Glu Glu Leu Glu Ser Gln Val Ala Gln
   245 250 255
   Leu Lys Asn Ser Ser Glu Glu Lys Glu Ala Met Asn Ser Ile Cys Gln
   260 265 270
   Thr Ala Leu Ser Ala Asp Gly Lys Gly Ser Lys Gly Ser Glu Glu Asp
   275 280 285
   Val Ala Ser Lys Asn Gln Gly Asp Ser Ala Arg Lys Gln Pro Gly Ser
   290 295 300
   Ser Thr Ser Ser Ser Ser His Leu Ala Lys Pro Ser Ser Ser Arg Leu
   305 310 315 320
   Cys Asp Thr Ser Ser Ala Arg Gln Glu Ser Thr Ser Lys Ala Glu Leu
   325 330 335
   Asn Cys Ser Lys Asn Lys Asp Leu Tyr Gln Glu Gln Val Glu Val Met
   340 345 350
   Leu Asp Val Thr Asp Thr Ser Met Asp Thr Tyr Leu Glu Arg Glu Trp
   355 360 365
   Gly Asn Lys Pro Ser Asp Cys Val Pro Tyr Lys Asp Glu Glu Leu Tyr
   370 375 380
   Asp Leu Pro Ala Pro Cys Thr Pro Leu Ser Leu Ser Cys Leu Gln Leu
   385 390 395 400
   Ser Thr Pro Glu Asn Arg Glu Ser Ser Val Val Gln Ala Gly Gly Ser
   405 410 415
   Lys Lys His Ser Asn His Leu Arg Lys Leu Val Phe Asp Asp Phe Cys
   420 425 430
   Asp Ser Ser Asn Val Ser Asn Lys Asp Ser Ser Glu Asp Asp Ile Ser
   435 440 445
   Arg Ser Glu Asn Glu Lys Lys Ser Glu Cys Phe Ser Ser Pro Lys Thr
   450 455 460
   Gly Phe Trp Asp Cys Cys Ser Thr Ser Tyr Ala Gln Asn Leu Asp Phe
   465 470 475 480
   Glu Ser Ser Glu Gly Asn Thr Ile Ala Asn Ser Val Gly Glu Ile Ser
   485 490 495
   Ser Lys Leu Ser Glu Lys Ser Gly Ser Cys Val Ser Lys Arg Leu Asn
   500 505 510
   Ser Ile Arg Ser Phe Glu Met Asn Arg Thr Arg Thr Ser Ser Glu Ala
   515 520 525
   Ser Met Asp Ala Ala Tyr Leu Asp Lys Ile Ser Glu Leu Asp Ser Met
   530 535 540
   Met Ser Glu Ser Asp Asn Ser Lys Ser Pro Cys Asn Asn Gly Phe Lys
   545 550 555 560
   Ser Leu Asp Leu Asp Gly Leu Ser Lys Ser Ser Gln Gly Ser Glu Phe
   565 570 575
   Leu Glu Glu Pro Asp Lys Leu Glu Glu Lys Thr Glu Leu Asn Leu Ser
   580 585 590
   Lys Gly Ser Leu Thr Asn Asp Gln Leu Glu Asn Gly Ser Glu Trp Lys
   595 600 605
   Pro Thr Ser Phe Phe Leu Leu Ser Pro Ser Asp Gln Glu Met Asn Glu
   610 615 620
   Asp Phe Ser Leu His Ser Ser Ser Cys Pro Val Thr Asn Glu Ile Lys
   625 630 635 640
   Pro Pro Ser Cys Leu Phe Gln Thr Glu Phe Ser Gln Gly Ile Leu Leu
                   645                 650                 655
   Ser Ser Ser His Arg Leu Phe Glu Asp Gln Arg Phe Gly Ser Ser Leu
               660                 665                 670
   Phe Lys Met Ser Ser Glu Met His Ser Leu His Asn His Leu Gln Ser
           675                 680                 685
   Pro Trp Ser Thr Ser Phe Val Pro Glu Lys Arg Asn Lys Asn Val Asn
       690                 695                 700
   Gln Ser Thr Lys Arg Lys Ile Gln Ser Ser Leu Ser Asn Ala Ser Pro
   705                 710                 715                 720
   Ser Lys Ala Thr Lys Ser
                   725
<210> 4
   <211> 726
   <212> PRT
   <213> Gorilla gorilla
<400> 4
   Met Ala Gln Thr Val Gln Asn Val Thr Leu Ser Leu Thr Leu Pro Ile
   1               5                   10                  15
   Thr Cys His Ile Cys Leu Gly Lys Val Arg Gln Pro Val Ile Cys Ile
               20                  25                  30
   Asn Asn His Val Phe Cys Ser Ile Cys Ile Asp Leu Trp Leu Lys Asn
           35                  40                  45
   Asn Ser Gln Cys Pro Ala Cys Arg Val Pro Ile Thr Pro Glu Asn Pro
       50                  55                  60
   Cys Lys Glu Ile Ile Gly Gly Thr Ser Glu Ser Glu Pro Met Leu Ser
   65                  70                  75                  80
   His Thr Val Arg Lys His Leu Arg Lys Thr Arg Leu Glu Leu Leu His
                   85                  90                  95
   Lys Glu Tyr Glu Asp Glu Ile Asp Cys Leu Gln Lys Glu Val Glu Glu
               100                 105                 110
   Leu Lys Ser Lys Asn Leu Gly Leu Glu Ser Gln Ile Lys Thr Ile Leu
           115                 120                 125
   Asp Pro Leu Thr Leu Val Gln Gly Asn Gln Asn Glu Asp Lys His Leu
       130                 135                 140
   Val Thr Asp Asn Pro Ser Lys Ile Asn Pro Glu Thr Val Ala Glu Trp
   145                 150                 155                 160
   Lys Lys Lys Leu Arg Thr Ala Asn Glu Ile Tyr Glu Lys Val Lys Asp
                   165                 170                 175
   Asp Val Asp Lys Leu Lys Glu Ala Asn Lys Lys Leu Lys Leu Glu Asn
               180                 185                 190
   Gly Gly Leu Val Arg Glu Asn Leu Arg Leu Lys Ala Glu Val Asp Asn
           195                 200                 205
   Arg Ser Pro Gln Lys Phe Gly Arg Phe Ala Val Ala Ala Leu Gln Ser
       210                 215                 220
   Lys Val Glu Gln Tyr Glu Arg Glu Thr Asn Arg Leu Lys Lys Ala Leu
   225                 230                 235                 240
   Glu Arg Ser Asp Lys Tyr Ile Glu Glu Leu Glu Ser Gln Val Ala Gln
                   245                 250                 255
   Leu Lys Asn Ser Ser Glu Glu Lys Glu Ala Met Asn Ser Ile Cys Gln
               260                 265                 270
   Thr Ala Leu Ser Ala Asp Gly Lys Gly Ser Lys Gly Ser Glu Glu Asp
           275                 280                 285
   Val Ala Ser Lys Asn Gln Gly Asp Ser Ala Arg Lys Gln Pro Gly Ser
       290                 295                 300
   Ser Thr Ser Ser Ser Ser His Leu Ala Lys Pro Ser Ser Ser Arg Leu
   305                 310                 315                 320
   Cys Asp Thr Ser Ser Ala Arg Gln Glu Ser Thr Ser Lys Ala Glu Leu
                   325                 330                 335
   Asn Cys Ser Lys Asn Lys Asp Leu Tyr Gln Glu Gln Val Glu Val Met
               340                 345                 350
   Leu Asp Val Thr Asp Thr Ser Met Asp Thr Tyr Leu Glu Arg Glu Trp
           355                 360                 365
   Gly Asn Lys Pro Ser Asp Cys Val Pro Tyr Lys Asp Glu Glu Leu Tyr
       370                 375                 380
   Asp Leu Pro Ala Pro Cys Thr Pro Leu Ser Leu Ser Cys Leu Gln Leu
   385 390 395 400
   Ser Thr Pro Glu Asn Arg Glu Ser Ser Val Val Gln Ala Gly Gly Ser
   405 410 415
   Lys Lys His Ser Asn His Phe Arg Lys Leu Val Phe Asp Asp Phe Cys
   420 425 430
   Asp Ser Ser Asn Val Ser Asn Lys Asp Ser Ser Glu Asp Asp Ile Ser
   435 440 445
   Arg Ser Glu Asn Glu Lys Lys Ser Glu Cys Phe Ser Ser Pro Lys Thr
   450 455 460
   Gly Phe Trp Asp Cys Cys Ser Thr Ser Tyr Ala Gln Asn Leu Asp Phe
   465 470 475 480
   Glu Ser Ser Glu Gly Asn Thr Ile Ala Asn Ser Val Gly Glu Ile Ser
   485 490 495
   Ser Lys Leu Ser Glu Lys Ser Gly Ser Cys Leu Ser Lys Arg Leu Asn
   500 505 510
   Ser Ile Arg Ser Phe Glu Met Asn Arg Thr Arg Thr Ser Ser Glu Ala
   515 520 525
   Ser Met Asp Ala Ala Tyr Leu Asp Lys Ile Ser Glu Leu Asp Ser Met
   530 535 540
   Met Ser Glu Ser Asp Asn Ser Lys Ser Pro Cys Asn Asn Gly Phe Lys
   545 550 555 560
   Ser Leu Asp Leu Asp Gly Leu Ser Lys Ser Ser Gln Gly Ser Glu Phe
   565 570 575
   Leu Glu Glu Pro Asp Lys Leu Glu Glu Lys Thr Glu Leu Asn Leu Ser
   580 585 590
   Lys Gly Ser Leu Thr Asn Asp Gln Leu Glu Asn Gly Ser Glu Trp Lys
   595 600 605
   Pro Thr Ser Phe Phe Leu Leu Ser Pro Ser Asp Gln Glu Met Asn Glu
   610 615 620
   Asp Phe Ser Leu His Ser Ser Ser Cys Pro Val Thr Asn Glu Ile Lys
   625 630 635 640
   Pro Pro Ser Cys Leu Phe Gln Ala Glu Phe Ser Gln Gly Ile Leu Leu
                   645                 650                 655
   Ser Ser Ser His Arg Leu Phe Glu Asp Gln Arg Phe Gly Ser Ser Leu
               660                 665                 670
   Phe Lys Met Ser Ser Glu Met Tyr Ser Leu His Asn His Leu Gln Ser
           675                 680                 685
   Pro Trp Ser Ala Ser Phe Val Pro Glu Lys Arg Asn Lys Asn Val Asn
       690                 695                 700
   Gln Ser Thr Lys Arg Lys Ile Gln Ser Ser Leu Ser Asn Ala Ser Pro
   705                 710                 715                 720
   Ser Lys Ala Thr Lys Ser
                   725
<210> 5
   <211> 726
   <212> **PRT**
   <213> Pongo abelii
<400> 5
   Met Ala Gln Thr Val Gln Asn Val Thr Leu Ser Leu Thr Leu Pro Ile
   1               5                   10                  15
   Thr Cys His Ile Cys Leu Gly Lys Val Arg Gln Pro Val Ile Cys Ile
               20                  25                  30
   Asn Asn His Val Phe Cys Ser Ile Cys Ile Asp Leu Trp Leu Lys Asn
           35                  40                  45
   Asn Ser Gln Cys Pro Ala Cys Arg Val Pro Ile Thr Pro Glu Asn Pro
       50                  55                  60
   Cys Lys Glu Ile Ile Gly Gly Thr Ser Glu Ser Glu Pro Met Leu Ser
   65                  70                  75                  80
   His Thr Val Arg Lys His Leu Arg Lys Thr Arg Leu Glu Leu Leu His
                   85                  90                  95
   Lys Glu Tyr Glu Asp Glu Ile Asp Cys Leu Gln Lys Glu Val Glu Glu
               100                 105                 110
   Leu Lys Ser Lys Asn Leu Ser Leu Glu Ser Gln Ile Lys Thr Ile Leu
           115                 120                 125
   Asp Pro Leu Thr Leu Val Gln Gly Asn Gln Asn Glu Asp Lys His Leu
       130                 135                 140
   Val Thr Asp Asn Pro Ser Lys Ile Asn Pro Glu Thr Val Ala Glu Trp
   145                 150                 155                 160
   Lys Lys Lys Leu Arg Thr Ala Asn Glu Ile Tyr Glu Lys Val Lys Asp
                   165                 170                 175
   Asp Val Asp Lys Leu Lys Glu Ala Asn Lys Lys Leu Lys Leu Glu Asn
               180                 185                 190
   Gly Gly Leu Val Arg Glu Asn Leu Arg Leu Lys Ala Glu Val Asp Asn
           195                 200                 205
   Arg Ser Pro Gln Lys Phe Gly Arg Phe Ala Val Ala Ala Leu Gln Ser
       210                 215                 220
   Lys Val Glu Gln Tyr Glu Arg Glu Thr Ser Arg Leu Lys Lys Ala Leu
   225                 230                 235                 240
   Glu Arg Ser Asp Lys Tyr Ile Glu Glu Leu Glu Ser Gln Val Ala Gln
                   245                 250                 255
   Leu Lys Asn Ser Ser Glu Glu Lys Glu Ala Met Asn Ser Ile Cys Gln
               260                 265                 270
   Thr Ala Leu Pro Ala Asp Gly Lys Gly Ser Lys Gly Ser Glu Glu Asp
           275                 280                 285
   Val Ala Ser Lys Asn Gln Gly Asp Ser Ala Arg Lys Gln Pro Ser Ser
       290                 295                 300
   Ser Thr Ser Ser Ser Ser His Leu Ala Lys Pro Ser Ser Ser Arg Leu
   305                 310                 315                 320
   Cys Asp Thr Ser Ser Ala Arg Gln Glu Ser Thr Ser Lys Ala Glu Leu
                   325                 330                 335
   Asn Cys Ser Lys Asn Lys Asp Leu Tyr Gln Glu Gln Val Glu Val Met
               340                 345                 350
   Leu Asp Val Thr Asp Thr Ser Met Asp Thr Tyr Leu Glu Arg Glu Trp
           355                 360                 365
   Gly Asn Lys Pro Ser Asp Cys Val Pro Tyr Lys Asp Glu Glu Leu Tyr
   370 375 380
   Asp Leu Pro Ala Pro Cys Thr Pro Leu Ser Leu Ser Cys Leu Gln Leu
   385 390 395 400
   Ser Thr Pro Glu Asn Arg Glu Ser Pro Val Val Gln Ala Gly Gly Ser
   405 410 415
   Lys Lys His Ser Asn His Leu Arg Lys Leu Val Phe Asp Asp Phe Cys
   420 425 430
   Asp Ser Ser Asn Val Ser Asn Lys Asp Ser Ser Glu Asp Asp Ile Ser
   435 440 445
   Arg Ser Glu Asn Glu Lys Lys Ser Glu Cys Phe Ser Ser Pro Lys Thr
   450 455 460
   Ala Phe Trp Asp Cys Cys Ser Thr Ser Tyr Ala Gln Asn Leu Asp Phe
   465 470 475 480
   Glu Ser Ser Glu Gly Asn Thr Ile Ala Asn Ser Val Gly Glu Ile Ser
   485 490 495
   Ser Lys Leu Ser Glu Lys Ser Gly Ser Cys Val Ser Lys Arg Leu Asn
   500 505 510
   Ser Ile Arg Ser Phe Glu Met Asn Arg Thr Arg Thr Ser Ser Glu Ala
   515 520 525
   Ser Met Asp Ala Ala Tyr Leu Asp Lys Ile Ser Glu Leu Asp Ser Met
   530 535 540
   Met Ser Glu Ser Asp Asn Ser Lys Ser Pro Cys Asn Asn Gly Phe Lys
   545 550 555 560
   Ser Leu Asp Leu Asp Gly Leu Ser Lys Ser Ser Gln Gly Ser Glu Phe
   565 570 575
   Leu Glu Glu Pro Asp Lys Leu Glu Glu Lys Thr Glu Leu Asn Leu Ser
   580 585 590
   Lys Gly Ser Leu Thr Asn Asp Gln Leu Glu Asn Gly Ser Glu Trp Lys
   595 600 605
   Pro Thr Ser Phe Phe Leu Leu Ser Pro Ser Asp Gln Glu Met Asn Glu
   610 615 620
   Asp Phe Ser Leu His Ser Ser Ser Cys Pro Val Thr Asn Glu Ile Lys
   625                 630                 635                 640
   Pro Pro Ser Cys Leu Phe Gln Thr Glu Phe Ser Gln Gly Ile Leu Leu
                   645                 650                 655
   Ser Ser Ser His Arg Leu Phe Glu Asp Gln Arg Phe Gly Ser Ser Leu
               660                 665                 670
   Phe Lys Met Ser Ser Glu Met His Ser Leu His Asn His Leu Gln Ser
           675                 680                 685
   Pro Trp Ser Thr Ser Phe Val Pro Glu Lys Arg Asn Lys Asn Val Asn
       690                 695                 700
   Gln Ser Thr Lys Arg Lys Ile Gln Ser Ser Leu Ser Asn Ala Ser Pro
   705                 710                 715                 720
   Ser Lys Ala Thr Lys Ser
                   725
<210> 6
   <211> 726
   <212> PRT
   <213> Macaca fascicularis
<400> 6
   Met Ala Gln Thr Val Gln Asn Val Thr Leu Ser Leu Thr Leu Pro Ile
   1               5                   10                  15
   Thr Cys His Ile Cys Leu Gly Lys Val Arg Gln Pro Val Ile Cys Ile
               20                  25                  30
   Asn Asn His Val Phe Cys Ser Ile Cys Ile Asp Leu Trp Leu Lys Asn
           35                  40                  45
   Asn Ser Gln Cys Pro Ala Cys Arg Val Pro Ile Thr Pro Glu Asn Pro
       50                  55                  60
   Cys Lys Glu Ile Ile Gly Gly Thr Ser Glu Ser Glu Pro Thr Leu Ser
   65                  70                  75                  80
   His Thr Val Arg Lys His Leu Arg Lys Thr Arg Leu Glu Leu Leu His
                   85                  90                  95
   Lys Glu Tyr Glu Asp Glu Ile Asp Cys Leu Gln Lys Glu Val Glu Glu
               100                 105                 110
   Leu Lys Ser Lys Asn Leu Ser Leu Glu Ser Gln Ile Lys Thr Ile Leu
   115 120 125
   Asp Pro Leu Thr Leu Val Gln Gly Asn Gln Asn Glu Asp Lys His Leu
   130 135 140
   Val Thr Asp Asn Pro Thr Lys Ile Asn Pro Glu Thr Val Ala Glu Trp
   145 150 155 160
   Lys Lys Lys Leu Arg Thr Ala Asn Glu Ile Tyr Glu Lys Val Lys Asp
   165 170 175
   Asp Val Asp Lys Leu Lys Glu Ala Asn Lys Lys Leu Lys Leu Glu Asn
   180 185 190
   Gly Gly Leu Val Arg Glu Asn Leu Arg Leu Lys Ala Glu Val Asp Asn
   195 200 205
   Arg Ser Pro Gln Lys Phe Gly Arg Phe Ala Val Ala Ala Leu Gln Ser
   210 215 220
   Lys Val Glu Gln Tyr Glu Arg Glu Thr Asn Arg Leu Lys Lys Ala Leu
   225 230 235 240
   Glu Arg Ser Asp Lys Tyr Ile Glu Glu Leu Glu Ser Gln Val Ala Gln
   245 250 255
   Leu Lys Asn Ser Ser Glu Glu Lys Glu Ala Met Asn Ser Ile Cys Arg
   260 265 270
   Arg Ala Pro Ser Ala Asp Gly Lys Gly Ser Lys Gly Ser Glu Glu Asp
   275 280 285
   Val Ala Ser Lys Asn Gln Gly Asp Ser Ala Arg Lys Gln Pro Gly Ser
   290 295 300
   Ser Thr Thr Ser Ser Ser His Leu Ala Lys Pro Ser Ser Ser Arg Leu
   305 310 315 320
   Cys Asp Thr Ser Ser Ala Arg Gln Glu Ser Thr Ser Lys Ala Glu Ile
   325 330 335
   Asn Cys Ser Lys Asn Lys Asp Leu Tyr Gln Lys Gln Val Glu Ile Met
   340 345 350
   Leu Asp Val Thr Asp Thr Ser Met Asp Thr Tyr Leu Glu Arg Glu Trp
   355 360 365
   Gly Asn Lys Pro Ser Asp Cys Val Pro Tyr Lys Asp Glu Glu Leu Tyr
   370 375 380
   Asp Leu Pro Ala Pro Cys Thr Pro Leu Ser Leu Ser Cys Leu Gln Leu
   385 390 395 400
   Ser Thr Pro Glu Asn Arg Glu Ser Ser Val Val Gln Ala Gly Gly Ser
   405 410 415
   Lys Lys His Ser Asn His Leu Arg Lys Leu Val Phe Asp Asp Phe Cys
   420 425 430
   Asp Ser Ser Asn Val Ser Asn Lys Asp Ser Ser Glu Asp Asp Ile Ser
   435 440 445
   Arg Ser Glu Asn Glu Lys Lys Ser Glu Cys Phe Ser Ser Pro Lys Thr
   450 455 460
   Gly Phe Trp Glu Cys Cys Ser Thr Ser Tyr Ala Gln Asn Leu Asp Phe
   465 470 475 480
   Glu Ser Ser Glu Gly Thr Thr Ile Ala Asn Ser Val Gly Glu Ile Ala
   485 490 495
   Ser Lys Leu Ser Glu Lys Ser Gly Ser Cys Val Ser Lys Arg Leu Asn
   500 505 510
   Ser Ile Arg Ser Phe Glu Met Asn Arg Thr Arg Thr Ser Ser Glu Ala
   515 520 525
   Ser Met Asp Ala Ala Tyr Leu Asp Lys Ile Ser Glu Leu Asp Ser Met
   530 535 540
   Met Ser Glu Ser Asp Asn Ser Lys Ser Pro Cys Asn Asn Gly Phe Lys
   545 550 555 560
   Ser Leu Asp Leu Asp Gly Leu Ser Lys Ser Ser Gln Gly Ser Glu Phe
   565 570 575
   Leu Glu Glu Pro Asp Lys Leu Glu Glu Lys Thr Lys Leu Asn Leu Ser
   580 585 590
   Lys Gly Ser Leu Thr Asn Asp Gln Leu Glu Asn Gly Asn Glu Trp Lys
   595 600 605
   Pro Thr Ser Phe Phe Leu Leu Ser Pro Ser Asp Gln Glu Met Asn Glu
   610 615 620
   Asp Phe Ser Leu His Ser Ser Ser Cys Pro Val Thr Asp Glu Ile Lys
   625 630 635 640
   Pro Pro Ser Cys Leu Phe Gln Thr Glu Phe Ser Gln Gly Ile Leu Leu
   645 650 655
   Ser Ser Ser His Arg Leu Phe Glu Asp Gln Arg Phe Gly Ser Ser Leu
   660 665 670
   Phe Lys Met Ser Ser Glu Met His Ser Leu His Asn His Leu Gln Ser
   675 680 685
   Pro Trp Ser Thr Ser Phe Val Pro Glu Lys Arg Asn Lys Asn Val Asn
   690 695 700
   Gln Ser Thr Lys Arg Lys Ile Gln Ser Ser Leu Ser Asn Ala Ser Pro
   705 710 715 720
   Ser Lys Ala Thr Lys Ser
   725
<210> 7
   <211> 726
   <212> PRT
   <213> Macaca mulatta
<400> 7
   Met Ala Gln Thr Val Gln Asn Val Thr Leu Ser Leu Thr Leu Pro Ile
   1 5 10 15
   Thr Cys His Ile Cys Leu Gly Lys Val Arg Gln Pro Val Ile Cys Ile
   20 25 30
   Asn Asn His Val Phe Cys Ser Ile Cys Ile Asp Leu Trp Leu Lys Asn
   35 40 45
   Asn Ser Gln Cys Pro Ala Cys Arg Val Pro Ile Thr Pro Glu Asn Pro
   50 55 60
   Cys Lys Glu Ile Ile Gly Gly Thr Ser Glu Ser Glu Pro Thr Leu Ser
   65 70 75 80
   His Thr Val Arg Lys His Leu Arg Lys Thr Arg Leu Glu Leu Leu His
   85 90 95
   Lys Glu Tyr Glu Asp Glu Ile Asp Cys Leu Gln Lys Glu Val Glu Glu
   100 105 110
   Leu Lys Ser Lys Asn Leu Ser Leu Glu Ser Gln Ile Lys Thr Ile Leu
           115                 120                 125
   Asp Pro Leu Thr Leu Val Gln Gly Asn Gln Asn Glu Asp Lys His Leu
       130                 135                 140
   Val Thr Asp Asn Pro Thr Lys Ile Asn Pro Glu Thr Val Ala Glu Trp
   145                 150                 155                 160
   Lys Lys Lys Leu Arg Thr Ala Asn Glu Ile Tyr Glu Lys Val Lys Asp
                   165                 170                 175
   Asp Val Asp Lys Leu Lys Glu Ala Asn Lys Lys Leu Lys Leu Glu Asn
               180                 185                 190
   Gly Gly Leu Val Arg Glu Asn Leu Arg Leu Lys Ala Glu Val Asp Asn
           195                 200                 205
   Arg Ser Pro Gln Lys Phe Gly Arg Phe Ala Val Ala Ala Leu Gln Ser
       210                 215                 220
   Lys Val Glu Gln Tyr Glu Arg Glu Thr Asn Arg Leu Lys Lys Ala Leu
   225                 230                 235                 240
   Glu Arg Ser Asp Lys Tyr Ile Glu Glu Leu Glu Ser Gln Val Ala Gln
                   245                 250                 255
   Leu Lys Asn Ser Ser Glu Glu Lys Glu Ala Met Asn Ser Ile Cys Arg
               260                 265                 270
   Arg Ala Pro Ser Ala Asp Gly Lys Gly Ser Lys Gly Ser Glu Glu Asp
           275                 280                 285
   Val Ala Ser Lys Asn Gln Gly Asp Ser Ala Arg Lys Gln Pro Gly Ser
       290                 295                 300
   Ser Thr Thr Ser Ser Ser His Leu Ala Lys Pro Ser Ser Ser Arg Leu
   305                 310                 315                 320
   Cys Asp Thr Ser Ser Ala Arg Gln Glu Ser Thr Ser Lys Ala Glu Ile
                   325                 330                 335
   Asn Cys Ser Lys Asn Lys Asp Leu Tyr Gln Lys Gln Val Glu Ile Met
               340                 345                 350
   Leu Asp Val Thr Asp Thr Ser Met Asp Thr Tyr Leu Glu Arg Glu Trp
           355                 360                 365
   Gly Asn Lys Pro Ser Asp Cys Val Pro Tyr Lys Asp Glu Glu Leu Tyr
       370                 375                 380
   Asp Leu Pro Ala Pro Cys Thr Pro Leu Ser Leu Ser Cys Leu Gln Leu
   385                 390                 395                 400
   Ser Thr Pro Glu Asn Arg Glu Ser Ser Val Val Gln Ala Gly Gly Ser
                   405                 410                 415
   Lys Lys His Ser Asn His Leu Arg Lys Leu Val Phe Asp Asp Phe Cys
               420                 425                 430
   Asp Ser Ser Asn Val Ser Asn Lys Asp Ser Ser Glu Asp Asp Ile Ser
           435                 440                 445
   Arg Ser Glu Asn Glu Lys Lys Ser Glu Cys Phe Ser Ser Pro Lys Thr
       450                 455                 460
   Gly Phe Trp Glu Cys Cys Ser Thr Ser Tyr Ala Gln Asn Leu Asp Phe
   465                 470                 475                 480
   Glu Ser Ser Glu Gly Thr Thr Ile Ala Asn Ser Val Gly Glu Ile Ala
                   485                 490                 495
   Ser Lys Leu Ser Glu Lys Ser Gly Ser Cys Val Ser Lys Arg Leu Asn
               500                 505                 510
   Ser Ile Arg Ser Phe Glu Met Asn Arg Thr Arg Thr Ser Ser Glu Ala
           515                 520                 525
   Ser Met Asp Ala Ala Tyr Leu Asp Lys Ile Ser Glu Leu Asp Ser Met
       530                 535                 540
   Met Ser Glu Ser Asp Asn Ser Lys Ser Pro Cys Asn Asn Gly Phe Lys
   545                 550                 555                 560
   Ser Leu Asp Leu Asp Gly Leu Ser Lys Ser Ser Gln Gly Ser Glu Phe
                   565                 570                 575
   Leu Glu Glu Pro Asp Lys Leu Glu Glu Lys Thr Lys Leu Asn Leu Ser
               580                 585                 590
   Lys Gly Ser Leu Thr Asn Asp Gln Leu Glu Asn Gly Asn Glu Trp Lys
           595                 600                 605
   Pro Thr Ser Phe Phe Leu Leu Ser Pro Ser Asp Gln Glu Met Asn Glu
       610                 615                 620
   Asp Phe Ser Leu His Ser Ser Ser Cys Pro Val Thr Asp Glu Ile Lys
   625                 630                 635                 640
   Pro Pro Ser Cys Leu Phe Gln Thr Glu Phe Ser Gln Gly Ile Leu Leu
                   645                 650                 655
   Ser Ser Ser His Arg Leu Phe Glu Asp Gln Arg Phe Gly Ser Ser Leu
               660                 665                 670
   Phe Lys Met Ser Ser Glu Met His Ser Leu His Asn His Leu Gln Ser
           675                 680                 685
   Pro Trp Ser Thr Ser Phe Val Pro Glu Lys Arg Asn Lys Asn Val Asn
       690                 695                 700
   Gln Ser Thr Lys Arg Lys Ile Gln Ser Ser Leu Ser Asn Ala Ser Pro
   705                 710                 715                 720
   Ser Lys Ala Thr Lys Ser
                   725
<210> 8
   <211> 724
   <212> PRT
   <213> Callithrix jacchus
<400> 8
   Met Ala Gln Thr Val Gln Asn Val Thr Leu Ser Leu Thr Leu Pro Ile
   1               5                   10                  15
   Thr Cys His Ile Cys Leu Gly Lys Val Arg Gln Pro Val Ile Cys Ile
               20                  25                  30
   Asn Asn His Val Phe Cys Ser Ile Cys Ile Asp Leu Trp Leu Lys Asn
           35                  40                  45
   Asn Ser Gln Cys Pro Ala Cys Arg Val Pro Ile Thr Pro Glu Asn Pro
       50                  55                  60
   Cys Lys Glu Ile Ile Gly Gly Thr Ser Glu Ser Glu Pro Met Leu Ser
   65                  70                  75                  80
   His Thr Val Arg Lys His Leu Arg Lys Thr Arg Phe Glu Leu Leu His
                   85                  90                  95
   Lys Glu Tyr Glu Asp Glu Ile Asp Cys Leu Gln Lys Glu Val Glu Glu
               100                 105                 110
   Leu Lys Ser Lys Asn Leu Ser Leu Glu Ser Gln Ile Lys Thr Ile Leu
           115                 120                 125
   Asp Pro Leu Thr Leu Val Gln Gly Asn Gln Asn Glu Asn Lys His Leu
       130                 135                 140
   Val Thr Asp Asn Pro Ser Lys Ser Thr Pro Glu Thr Val Ala Glu Trp
   145                 150                 155                 160
   Lys Lys Lys Leu Arg Thr Ala Asn Glu Ile Tyr Glu Lys Val Lys Asp
                   165                 170                 175
   Asp Val Asp Lys Leu Lys Glu Val Asn Lys Lys Leu Lys Leu Glu Asn
               180                 185                 190
   Gly Gly Leu Val Arg Glu Asn Leu Arg Leu Lys Ala Glu Val Asp Asn
           195                 200                 205
   Arg Ser Pro Gln Lys Phe Gly Arg Phe Ala Val Ala Ala Leu Gln Ser
       210                 215                 220
   Lys Val Glu Gln Tyr Glu Arg Glu Thr Asn Arg Leu Lys Lys Ala Leu
   225                 230                 235                 240
   Glu Arg Ser Asp Lys Tyr Ile Glu Glu Leu Glu Ser Gln Val Ala Arg
                   245                 250                 255
   Leu Lys Asn Ser Ser Glu Glu Lys Glu Ala Met Asn Ser Ile Cys Gln
               260                 265                 270
   Arg Ala Leu Ser Ala Asp Gly Lys Gly Ser Lys Gly Ser Glu Glu Asp
           275                 280                 285
   Val Ala Ser Lys Asn Gln Gly Asp Ser Ala Arg Lys Gln Pro Ser Ser
       290                 295                 300
   Ser Thr Ser Ser Ser His Leu Pro Lys Pro Ser Ser Ser Arg Leu Cys
   305                 310                 315                 320
   Asp Ser Ser Ala Arg Gln Glu Asn Thr Ser Lys Ala Glu Leu Asn Cys
                   325                 330                 335
   Ser Lys Asn Lys Asp Leu Tyr Gln Lys Gln Val Glu Ile Met Leu Asp
               340                 345                 350
   Val Thr Asp Thr Ser Met Asp Thr Tyr Leu Glu Arg Glu Trp Gly Asn
   355 360 365
   Lys Pro Ser Asp Cys Val Pro Tyr Lys Gly Glu Glu Leu Tyr Asp Leu
   370 375 380
   Pro Ala Pro Cys Thr Pro Leu Ser Leu Ser Cys Leu His Leu Ser Thr
   385 390 395 400
   Pro Glu Ser Arg Glu Ser Ser Val Val Gln Val Gly Gly Ser Lys Lys
   405 410 415
   His Ser Asn His Leu Arg Lys Leu Val Phe Asp Asp Phe Cys Asp Ser
   420 425 430
   Ser Ser Val Ser Asn Lys Asp Ser Ser Glu Asp Asp Ile Ser Arg Ser
   435 440 445
   Glu Asn Glu Lys Lys Ser Glu Cys Phe Thr Ser Pro Lys Thr Gly Phe
   450 455 460
   Trp Asp Cys Cys Ser Thr Ser Phe Ala Gln Ser Leu Asp Phe Glu Ser
   465 470 475 480
   Ser Glu Gly Asn Thr Ile Ala Asn Ser Val Gly Glu Ile Ser Ser Lys
   485 490 495
   Leu Ser Glu Lys Ser Gly Ser Cys Ile Ser Lys Arg Leu Asn Ser Ile
   500 505 510
   Arg Ser Phe Glu Met Asn Arg Thr Arg Thr Ser Ser Glu Ala Ser Met
   515 520 525
   Asp Ala Ala Tyr Leu Asp Lys Ile Ser Glu Leu Asp Ser Met Met Ser
   530 535 540
   Glu Ser Asp Asn Ser Lys Ser Pro Cys Asn Asn Gly Phe Lys Ser Leu
   545 550 555 560
   Asp Leu Asp Gly Leu Ser Lys Ser Ser Gln Gly Ser Glu Phe Leu Glu
   565 570 575
   Glu Pro Asp Lys Leu Glu Glu Lys Thr Lys Leu Asn Leu Ser Lys Gly
   580 585 590
   Ser Leu Thr Asn Asp Gln Leu Glu Asn Gly Asn Glu Trp Lys Pro Thr
   595 600 605
   Ser Phe Phe Leu Leu Ser Pro Ser Asp Gln Glu Met Asn Asp Asp Phe
   610 615 620
   Ser Leu His Ser Gly Ser Ser Pro Gly Thr Asn Glu Leu Lys Pro Pro
   625 630 635 640
   Ser Cys Leu Phe Gln Thr Glu Phe Ser Gln Gly Ile Leu Leu Ser Ser
   645 650 655
   Ser His Arg Leu Phe Glu Asp Gln Arg Phe Gly Ser Ser Leu Phe Lys
   660 665 670
   Met Ser Ser Glu Met His Ser Leu His Asn His Leu Gln Ser Pro Trp
   675 680 685
   Ser Thr Ser Phe Val Pro Glu Lys Arg Asn Lys Asn Val Asn Gln Ser
   690 695 700
   Thr Lys Arg Lys Ile Gln Ser Ser Leu Ser Asn Ala Ser Pro Ser Lys
   705 710 715 720
   Ala Thr Lys Ser
<210> 9
   <211> 717
   <212> PRT
   <213> Equus caballus
<400> 9
   Met Ala Gln Thr Val Gln Asn Val Thr Leu Ser Leu Thr Leu Pro Ile
   1 5 10 15
   Thr Cys His Ile Cys Leu Gly Lys Val Arg Gln Pro Val Ile Cys Ile
   20 25 30
   Asn Asn His Val Phe Cys Ser Ile Cys Ile Asp Leu Trp Leu Lys Asn
   35 40 45
   Asn Ser Gln Cys Pro Ala Cys Arg Val Pro Ile Thr Pro Glu Asn Pro
   50 55 60
   Cys Lys Glu Ile Ile Gly Gly Thr Ser Glu Ser Glu Pro Met Leu Ser
   65 70 75 80
   His Thr Val Arg Lys His Leu Arg Lys Thr Arg Leu Glu Leu Leu His
   85 90 95
   Lys Glu Tyr Glu Asp Glu Ile Asp Cys Leu Gln Lys Glu Val Glu Glu
   100 105 110
   Leu Lys Ser Lys Asn Leu Ser Leu Glu Ser Gln Ile Lys Thr Ile Leu
   115 120 125
   Asp Pro Leu Thr Leu Met Gln Gly Asn Gln Asn Glu Asp Lys His Pro
   130 135 140
   Val Ala Asp Asn Pro Ser Lys Ile Asp Pro Glu Thr Val Ala Glu Trp
   145 150 155 160
   Lys Lys Lys Leu Arg Thr Ala Asn Glu Ile Tyr Glu Lys Val Lys Asp
   165 170 175
   Asp Val Asp Lys Leu Lys Glu Ala Asn Lys Asn Leu Lys Leu Glu Asn
   180 185 190
   Gly Gly Leu Val Arg Glu Asn Leu Arg Leu Lys Ala Glu Val Asp Asn
   195 200 205
   Arg Ser Pro Gln Lys Phe Gly Arg Phe Thr Val Ala Ala Leu Gln Ser
   210 215 220
   Lys Val Glu Gln Tyr Glu Arg Glu Thr Asn Arg Leu Lys Lys Ala Leu
   225 230 235 240
   Glu Arg Ser Asp Lys Tyr Ile Glu Glu Leu Glu Ser Gln Ile Ala Gln
   245 250 255
   Leu Lys Asn Ser Ser Glu Glu Lys Glu Ala Met Asn Ser Ile Cys Gln
   260 265 270
   Arg Thr Leu Ser Thr Asp Gly Lys Gly Ser Lys Cys Ser Glu Glu Asp
   275 280 285
   Met Ala Ser Lys Asn Gln Gly Asp Gly Ala Arg Lys Gln Leu Gly Ser
   290 295 300
   Ser Thr Ser Ser Ser His Leu Ala Lys Pro Ser Ser Ser Ser Ala Arg
   305 310 315 320
   Gln Glu Gly Ile Ser Lys Thr Glu Pro Asn Cys Ser Lys Asn Lys Asp
   325 330 335
   Leu Tyr Gln Lys Gln Val Glu Ile Met Leu Asp Val Thr Asp Thr Ser
   340 345 350
   Met Asp Thr Tyr Leu Glu Arg Glu Trp Gly Asn Lys Pro Ser Asp Cys
   355 360 365
   Val Pro Tyr Lys Asp Glu Glu Leu Tyr Asp Leu Pro Ala Pro Cys Thr
   370 375 380
   Pro Leu Ser Leu Ser Cys Leu Gln Leu Ser Thr Pro Glu Asn Arg Glu
   385 390 395 400
   Ser Ser Val Val Lys Ala Gly Gly Ser Lys Lys His Ser Asn His Leu
   405 410 415
   Arg Lys Leu Val Phe Asp Asp Phe Cys Asp Ser Ser Ser Val Cys Asn
   420 425 430
   Lys Asp Ser Ser Glu Asp Asp Arg Ser Glu Asn Glu Lys Lys Ser Asp
   435 440 445
   Cys Phe Thr Ser Pro Lys Thr Gly Phe Trp Asp Cys Cys Ser Thr Ser
   450 455 460
   Tyr Ala Gln Ser Leu Asp Phe Glu Ser Ser Glu Gly Asn Thr Ile Ala
   465 470 475 480
   Asn Ser Val Gly Glu Ile Ser Ser Lys Leu Ser Glu Lys Ser Gly Ser
   485 490 495
   Cys Leu Ser Lys Arg Leu Asn Ser Ile Arg Ser Phe Glu Met Asn Arg
   500 505 510
   Thr Arg Thr Ser Ser Glu Ala Ser Met Asp Ala Ala Tyr Leu Asp Lys
   515 520 525
   Ile Ser Glu Leu Asp Ser Met Met Ser Glu Ser Asp Asn Ser Lys Ser
   530 535 540
   Pro Cys Asn Asn Gly Phe Lys Ser Val Asp Leu Asp Gly Leu Ser Lys
   545 550 555 560
   Ser Ser Gln Gly Ser Glu Phe Leu Glu Glu Ser Asp Lys Leu Glu Glu
   565 570 575
   Arg Thr Lys Pro Asn Leu Ser Lys Gly Ser Leu Thr Thr Asp Gln Leu
   580 585 590
   Glu Asn Gly Asn Glu Trp Lys Pro Thr Ser Phe Phe Leu Leu Ser Pro
   595 600 605
   Ser Asp Gln Glu Met Asn Glu Asp Phe Ser Leu His Pro Ser Ser Asn
   610 615 620
   Pro Gly Thr Asn Glu Ile Lys Pro Pro Ser Cys Leu Phe Gln Thr Glu
   625 630 635 640
   Phe Ser Gln Gly Val Leu Leu Ser Ser Ser His Arg Leu Phe Glu Asp
   645 650 655
   Gln Arg Phe Gly Ser Ser Leu Phe Lys Met Ser Ser Glu Met His Gly
   660 665 670
   Leu His Asn Gln Leu Gln Ser Pro Trp Ser Thr Ser Phe Val Pro Glu
   675 680 685
   Lys Arg Asn Lys Asn Val Asn Gln Ser Thr Lys Arg Lys Ile Gln Ser
   690 695 700
   Ser Leu Ser Asn Ala Ser Pro Ser Lys Ala Thr Lys Ser
   705 710 715
<210> 10
   <211> 726
   <212> PRT
   <213> Spermophilus tridecemlineatus
<400> 10
   Met Ala Gln Thr Val Gln Asn Val Thr Leu Ser Leu Thr Leu Pro Ile
   1 5 10 15
   Thr Cys His Ile Cys Leu Gly Lys Val Arg Gln Pro Val Ile Cys Ile
   20 25 30
   Asn Asn His Val Phe Cys Ser Ile Cys Ile Asp Leu Trp Leu Lys Asn
   35 40 45
   Asn Ser Gln Cys Pro Ala Cys Arg Val Pro Ile Thr Pro Glu Asn Pro
   50 55 60
   Cys Lys Glu Ile Ile Gly Gly Thr Ser Glu Ser Glu Pro Met Leu Thr
   65 70 75 80
   His Thr Val Arg Lys His Leu Arg Lys Thr Arg Leu Glu Leu Leu His
   85 90 95
   Lys Glu Tyr Glu Asp Glu Ile Asp Cys Leu Gln Lys Glu Val Glu Glu
               100                 105                 110
   Leu Lys Ser Lys Asn Leu Ser Leu Glu Ser Gln Ile Lys Thr Ile Leu
           115                 120                 125
   Asp Pro Leu Thr Leu Met Gln Gly Ser Gln Asn Glu Asp Lys His Pro
       130                 135                 140
   Val Ala Asp Asn Pro Ser Lys Ile Asp Ser Glu Thr Val Ala Glu Trp
   145                 150                 155                 160
   Lys Lys Lys Leu Arg Thr Ala Asn Glu Ile Tyr Glu Lys Val Lys Asp
                   165                 170                 175
   Asp Val Asp Lys Leu Lys Glu Ala Asn Lys Lys Leu Lys Leu Glu Asn
               180                 185                 190
   Gly Gly Leu Val Arg Glu Asn Leu Arg Leu Lys Ala Glu Val Asp Asn
           195                 200                 205
   Arg Ser Pro Gln Lys Phe Gly Arg Phe Thr Val Ala Ala Leu Gln Ser
       210                 215                 220
   Lys Val Glu Gln Tyr Glu Arg Glu Thr Asn Arg Leu Lys Lys Ala Leu
   225                 230                 235                 240
   Glu Arg Ser Asp Lys Tyr Ile Glu Glu Leu Glu Ser Gln Val Ala Gln
                   245                 250                 255
   Leu Lys Asn Ser Ser Glu Glu Lys Glu Thr Ile Asp Ser Ile Cys Gln
               260                 265                 270
   Arg Ala Leu Ser Ala Asp Gly Asn Gly Ser Lys Ser Ser Glu Glu Asp
           275                 280                 285
   Val Ala Ser Lys Asn Gln Gly Asp Ser Ala Arg Lys Gln Pro Gly Ser
       290                 295                 300
   Ser Ala Ser Ile Thr Val His Leu Ala Lys Pro Ser Ser Gly Arg Ile
   305                 310                 315                 320
   Ser Asp Thr Ser Ser Ala Arg Gln Glu Asn Thr Ser Lys Thr Glu Ser
                   325                 330                 335
   Asn Cys Pro Lys Asn Lys Asp Met Tyr Gln Lys Gln Val Glu Ile Met
               340                 345                 350
   Leu Asp Val Thr Asp Thr Ser Met Asp Thr Tyr Leu Glu Gly Glu Trp
   355 360 365
   Ser Asn Lys Pro Asn Asp Cys Val Pro Phe Lys Asp Glu Glu Leu Tyr
   370 375 380
   Asp Leu Pro Ala Pro Cys Thr Pro Leu Ser Leu Ser Cys Leu Gln Leu
   385 390 395 400
   Asn Thr Pro Glu Asn Arg Glu Ser Ser Val Val Lys Ala Gly Ser Ser
   405 410 415
   Lys Lys His Ser Asn His Leu Arg Lys Leu Val Phe Asp Asp Phe Cys
   420 425 430
   Asp Ser Gln Asn Val Cys Asn Lys Asp Ser Ser Glu Asp Asp Thr Ser
   435 440 445
   Arg Asn Glu Asn Glu Lys Lys Ser Glu Cys Phe Thr Ser Pro Lys Thr
   450 455 460
   Gly Phe Trp Asp Cys Cys Ser Thr Ser Tyr Ala Gln Ser Leu Asp Phe
   465 470 475 480
   Glu Asn Ser Glu Glu Asn Thr Ile Ala Asn Ser Val Gly Glu Ile Ser
   485 490 495
   Leu Lys Leu Ser Glu Lys Ser Gly Ser Cys Leu Ser Lys Arg Leu Asn
   500 505 510
   Cys Ile Arg Thr Phe Glu Met Asn Arg Thr Arg Thr Ser Ser Glu Ala
   515 520 525
   Ser Met Asp Ala Ala Tyr Leu Asp Lys Ile Ser Glu Leu Asp Ser Met
   530 535 540
   Met Ser Glu Ser Asp Asn Ser Lys Ser Pro Cys Asn Asn Gly Phe Lys
   545 550 555 560
   Ser Val Asp Val Asp Gly Leu Ser Lys Ser Ser Gln Gly Ser Glu Phe
   565 570 575
   Leu Glu Glu Ser Glu Lys Leu Glu Glu Arg Thr Lys Leu Asn Leu Ser
   580 585 590
   Lys Ser Ser Leu Thr Thr Asp Gln Leu Glu Asn Gly Asn Glu Trp Lys
   595 600 605
   Pro Thr Ser Phe Phe Leu Leu Ser Pro Ser Asp Gln Glu Met Asn Glu
       610                 615                 620
   Asp Phe Ser Leu His Ser Thr Ser Asn Pro Gly Thr Asn Asp Ile Lys
   625                 630                 635                 640
   Pro Pro Ser Cys Leu Phe Gln Thr Glu Phe Ser Gln Gly Val Leu Leu
                   645                 650                 655
   Ser Ser Ser His Arg Leu Phe Glu Asp Gln Arg Phe Gly Ser Ser Leu
               660                 665                 670
   Phe Lys Thr Ser Ser Glu Met His Ser Leu His Asn His Leu Gln Ser
           675                 680                 685
   Pro Trp Ser Thr Ser Phe Val Pro Glu Lys Arg Asn Lys Asn Val Asn
       690                 695                 700
   Gln Ser Thr Lys Arg Lys Ile Gln Ser Ser Leu Ser Asn Ala Ser Pro
   705                 710                 715                 720
   Ser Lys Ala Thr Lys Ser
                   725
<210> 11
   <211> 717
   <212> PRT
   <213> Ailuropoda melanoleuca
<400> 11
   Met Ala Gln Thr Val Gln Asn Val Thr Leu Ser Leu Thr Leu Pro Ile
   1               5                   10                  15
   Thr Cys His Ile Cys Leu Gly Lys Val Arg Gln Pro Val Ile Cys Ile
               20                  25                  30
   Asn Asn His Val Phe Cys Ser Ile Cys Ile Asp Leu Trp Leu Lys Asn
           35                  40                  45
   Asn Ser Gln Cys Pro Ala Cys Arg Val Pro Ile Thr Pro Glu Asn Pro
       50                  55                  60
   Cys Lys Glu Ile Ile Gly Gly Thr Ser Glu Ser Glu Pro Met Leu Ser
   65                  70                  75                  80
   His Thr Val Arg Lys His Leu Arg Lys Thr Arg Leu Glu Leu Leu His
                   85                  90                  95
   Lys Glu Tyr Glu Asp Glu Ile Asp Cys Leu Gln Lys Glu Val Glu Asp
               100                 105                 110
   Leu Lys Ser Lys Asn Leu Ser Leu Glu Ser Gln Ile Lys Thr Ile Leu
           115                 120                 125
   Asp Pro Leu Thr Leu Met Gln Gly Asn Gln Asn Glu Glu Lys His Pro
       130                 135                 140
   Val Ala Asp Asn Pro Ser Lys Ile Asp Pro Glu Thr Val Ala Glu Trp
   145                 150                 155                 160
   Lys Lys Lys Leu Arg Thr Ala Asn Glu Ile Tyr Glu Lys Val Lys Asp
                   165                 170                 175
   Asp Val Asp Lys Leu Lys Glu Ala Asn Lys Lys Leu Lys Leu Glu Asn
               180                 185                 190
   Gly Gly Leu Val Arg Glu Asn Leu Arg Leu Lys Ala Glu Val Asp Asn
           195                 200                 205
   Arg Ser Pro Gln Lys Phe Gly Arg Phe Thr Val Ala Ala Leu Gln Ser
       210                 215                 220
   Lys Val Glu Gln His Glu Arg Glu Thr Asn Arg Leu Lys Lys Ala Leu
   225                 230                 235                 240
   Glu Arg Ser Asp Lys Tyr Ile Glu Glu Leu Glu Ser Gln Ile Ala Gln
                   245                 250                 255
   Leu Lys Asn Ser Ser Glu Glu Lys Glu Ala Met Asn Ser Val Cys Gln
               260                 265                 270
   Arg Ala Leu Ser Thr Asp Gly Lys Gly Ser Ile Gly Asn Glu Glu Asp
           275                 280                 285
   Met Ala Ser Lys Asn Gln Gly Asp Gly Ala Arg Lys Gln Leu Gly Ser
       290                 295                 300
   Ser Thr Ser Ser Ser His Leu Ala Lys Pro Ser Ser Ser Ser Ala Arg
   305                 310                 315                 320
   Gln Glu Ser Thr Ser Lys Thr Glu Pro Asn Cys Ser Lys Asn Lys Asp
                   325                 330                 335
   Leu Tyr Gln Lys Gln Val Glu Ile Met Leu Asp Val Thr Asp Thr Ser
   340 345 350
   Met Asp Thr Tyr Leu Glu Arg Glu Trp Gly Asn Lys Arg Ser Asp Cys
   355 360 365
   Ile Pro Tyr Lys Asp Glu Glu Leu Tyr Asp Leu Pro Ala Pro Cys Thr
   370 375 380
   Pro Leu Ser Leu Ser Cys Leu Gln Leu Ser Thr Pro Glu Asn Arg Glu
   385 390 395 400
   Ser Pro Val Val Lys Ala Val Gly Ser Lys Lys His Ser Asn His Leu
   405 410 415
   Arg Lys Leu Val Phe Asp Asp Phe Cys Asp Ser Pro Asn Val Cys Asn
   420 425 430
   Lys Asp Ser Ser Glu Asp Asp Arg Ser Glu Asn Glu Lys Lys Ser Glu
   435 440 445
   Cys Phe Thr Ser Ser Lys Thr Gly Phe Trp Asp Cys Cys Ser Thr Ser
   450 455 460
   Tyr Ala Gln Asn Leu Asp Phe Glu Ser Pro Glu Gly Asn Thr Ile Ala
   465 470 475 480
   Asn Ser Val Gly Glu Ile Thr Leu Lys Leu Ser Glu Lys Ser Gly Ser
   485 490 495
   Cys Leu Ser Lys Arg Leu Asn Ser Ile Arg Ser Phe Glu Met Asn Arg
   500 505 510
   Thr Arg Thr Ser Ser Glu Ala Ser Met Asp Ala Ala Tyr Leu Asp Lys
   515 520 525
   Ile Ser Glu Leu Asp Ser Met Met Ser Glu Ser Asp Asn Ser Lys Ser
   530 535 540
   Pro Cys Asn Asn Gly Phe Lys Ser Val Asp Leu Asp Gly Leu Ser Lys
   545 550 555 560
   Ser Ser Gln Gly Ser Glu Phe Leu Glu Glu Pro Glu Lys Leu Glu Glu
   565 570 575
   Arg Thr Lys Ala Asn Leu Ser Lys Gly Ser Leu Thr Thr Asp Gln Leu
   580 585 590
   Glu Asn Gly Asn Asp Trp Lys Pro Thr Ser Phe Phe Leu Leu Ser Pro
   595 600 605
   Ser Asp Gln Glu Met Asn Glu Asp Phe Ser Leu His Thr Ser Ser Asn
   610 615 620
   Pro Gly Thr Asn Glu Ile Lys Pro Pro Ser Cys Leu Phe Gln Thr Glu
   625 630 635 640
   Phe Ser Gln Gly Val Leu Leu Ser Ser Ser His Arg Leu Phe Glu Asp
   645 650 655
   Gln Arg Phe Gly Ser Ser Leu Phe Lys Met Thr Ser Glu Met His Gly
   660 665 670
   Leu His Asn His Leu Gln Ser Pro Trp Ser Thr Ser Phe Val Pro Glu
   675 680 685
   Lys Arg Asn Lys Asn Met Asn Gln Ser Thr Lys Arg Lys Ile Gln Ser
   690 695 700
   Ser Leu Ser Asn Ala Ser Pro Ser Lys Ala Thr Lys Ser
   705 710 715
<210> 12
   <211> 717
   <212> PRT
   <213> Canis familiaris
<400> 12
   Met Ala Gln Thr Val Gln Asn Val Thr Leu Ser Leu Thr Leu Pro Ile
   1 5 10 15
   Thr Cys His Ile Cys Leu Gly Lys Val Arg Gln Pro Val Ile Cys Ile
   20 25 30
   Asn Asn His Val Phe Cys Ser Ile Cys Ile Asp Leu Trp Leu Lys Asn
   35 40 45
   Asn Ser Gln Cys Pro Ala Cys Arg Val Pro Ile Thr Pro Glu Asn Pro
   50 55 60
   Cys Lys Glu Ile Ile Gly Gly Thr Ser Glu Ser Glu Pro Met Leu Ser
   65 70 75 80
   His Thr Val Arg Lys His Leu Arg Lys Thr Arg Leu Glu Leu Leu His
   85 90 95
   Lys Glu Tyr Glu Asp Glu Ile Asp Cys Leu Gln Lys Glu Val Glu Asp
               100                 105                 110
   Leu Lys Ser Lys Asn Leu Ser Leu Glu Ser Gln Ile Lys Thr Ile Leu
           115                 120                 125
   Asp Pro Leu Ala Val Met Gln Gly Asn Gln Asn Glu Asp Lys His Pro
       130                 135                 140
   Val Ala Asp Asn Pro Ser Lys Ile Gly Pro Glu Thr Glu Ala Glu Trp
   145                 150                 155                 160
   Lys Lys Lys Leu Arg Thr Ala Asn Glu Ile Tyr Glu Lys Val Lys Asp
                   165                 170                 175
   Asp Val Asp Lys Leu Lys Glu Ala Asn Lys Lys Leu Lys Leu Glu Asn
               180                 185                 190
   Gly Gly Leu Val Arg Glu Asn Leu Arg Leu Lys Ala Glu Val Asp Asn
           195                 200                 205
   Arg Ser Pro Gln Lys Phe Gly Arg Phe Thr Val Ala Ala Leu Gln Ser
       210                 215                 220
   Lys Val Glu Gln His Glu Arg Glu Thr Asn Arg Leu Lys Lys Ala Leu
   225                 230                 235                 240
   Glu Arg Ser Asp Lys Tyr Ile Glu Glu Leu Glu Ser Gln Ile Ala Gln
                   245                 250                 255
   Leu Lys Asn Ser Ser Glu Glu Lys Glu Ala Met Asn Ser Ile Cys Gln
               260                 265                 270
   Arg Ala Leu Ser Thr Asp Gly Lys Gly Ser Ile Gly Asn Glu Glu Asp
           275                 280                 285
   Met Ala Ser Lys Ser Gln Gly Asp Gly Ala Arg Lys Gln Val Gly Ser
       290                 295                 300
   Ser Thr Ser Ser Ser His Leu Ala Lys Pro Ser Ser Ser Ser Ala Arg
   305                 310                 315                 320
   Gln Glu Cys Thr Ser Lys Thr Glu Pro Asn Cys Ser Lys Asn Lys Asp
                   325                 330                 335
   Leu Tyr Gln Lys Gln Val Glu Ile Met Leu Asp Val Thr Asp Thr Ser
               340                 345                 350
   Met Asp Thr Tyr Leu Glu Arg Glu Trp Gly Asn Lys Pro Ser Asp Cys
           355                 360                 365
   Ile Pro Tyr Lys Asp Glu Glu Leu Tyr Asp Leu Pro Ala Pro Cys Thr
       370                 375                 380
   Pro Leu Ser Leu Ser Cys Leu Gln Leu Ser Thr Pro Glu Asn Arg Glu
   385                 390                 395                 400
   Ser Ser Val Val Lys Ala Val Gly Ser Lys Lys His Ser Asn His Leu
                   405                 410                 415
   Arg Lys Leu Val Phe Asp Asp Phe Cys Asp Ser Pro Asn Val Cys Asn
               420                 425                 430
   Lys Asp Ser Ser Glu Asp Asp Arg Ser Glu Asn Glu Lys Lys Ser Glu
           435                 440                 445
   Cys Phe Thr Ser Ser Lys Thr Gly Phe Trp Asp Cys Cys Ser Thr Ser
       450                 455                 460
   Tyr Ala Gln Asn Leu Asp Phe Glu Ser Ser Glu Gly Asn Thr Ile Ala
   465                 470                 475                 480
   Asn Ser Val Gly Glu Ile Ser Leu Lys Leu Ser Glu Lys Ser Gly Ser
                   485                 490                 495
   Cys Leu Ser Lys Arg Leu Asn Ser Ile Arg Ser Phe Glu Met Asn Arg
               500                 505                 510
   Thr Arg Thr Ser Ser Glu Ala Ser Met Asp Ala Ala Tyr Leu Asp Lys
           515                 520                 525
   Ile Ser Glu Leu Asp Ser Met Met Ser Glu Ser Asp Asn Ser Lys Ser
       530                 535                 540
   Pro Cys Asn Asn Gly Phe Lys Ser Val Asp Leu Asp Gly Leu Ser Lys
   545                 550                 555                 560
   Ser Ser Gln Gly Ser Glu Phe Leu Glu Glu Pro Asp Lys Leu Glu Glu
                   565                 570                 575
   Arg Thr Lys Pro Asn Leu Ser Lys Gly Ser Leu Thr Thr Asp Gln Leu
               580                 585                 590
   Glu Asn Gly Asn Glu Trp Lys Pro Thr Ser Phe Phe Leu Leu Ser Pro
           595                 600                 605
   Ser Asp Gln Glu Met Asn Glu Asp Phe Ser Leu His Thr Ser Ser Asn
   610 615 620
   Pro Gly Thr Asn Glu Ile Lys Pro Pro Ser Cys Leu Phe Gln Thr Glu
   625 630 635 640
   Phe Ser Gln Gly Val Leu Leu Ser Ser Ser His Arg Leu Phe Glu Asp
   645 650 655
   Gln Arg Phe Gly Ser Ser Leu Phe Lys Met Thr Ser Glu Met His Gly
   660 665 670
   Leu His Asn His Leu Gln Ser Pro Trp Ser Ala Ser Phe Val Pro Glu
   675 680 685
   Lys Arg Asn Lys Asn Met Asn Gln Ser Ala Lys Arg Lys Ile Gln Ser
   690 695 700
   Ser Leu Ser Asn Ala Ser Pro Ser Lys Ala Thr Lys Ser
   705 710 715
<210> 13
   <211> 725
   <212> PRT
   <213> Oryctolagus cuniculus
<400> 13
   Met Ala Gln Thr Val Gln Asn Val Thr Leu Ser Leu Thr Leu Pro Ile
   1 5 10 15
   Thr Cys His Ile Cys Leu Gly Lys Val Arg Gln Pro Val Ile Cys Ile
   20 25 30
   Asn Asn His Val Phe Cys Ser Val Cys Ile Asp Leu Trp Leu Lys Asn
   35 40 45
   Asn Ser Gln Cys Pro Ala Cys Arg Val Pro Ile Thr Pro Glu Asn Pro
   50 55 60
   Cys Lys Glu Ile Ile Gly Gly Thr Ser Glu Ser Glu Pro Met Leu Ser
   65 70 75 80
   His Thr Val Arg Lys His Leu Arg Lys Thr Arg Leu Glu Leu Leu His
   85 90 95
   Lys Glu Tyr Glu Asp Glu Ile Asp Cys Leu Gln Lys Glu Val Glu Glu
   100 105 110
   Leu Lys Ser Lys Asn Leu Ser Leu Glu Ser Gln Ile Lys Thr Ile Leu
   115 120 125
   Asp Pro Leu Thr Leu Met Gln Gly Asn Gln Asn Glu Asp Lys His Pro
   130 135 140
   Val Ala Asp Asn Pro Asn Thr Val Asp Pro Glu Thr Ile Ala Glu Trp
   145 150 155 160
   Lys Lys Lys Leu Arg Thr Ala Asn Glu Ile Tyr Glu Lys Val Lys Asp
   165 170 175
   Asp Val Asp Lys Leu Lys Glu Ala Asn Lys Lys Leu Lys Leu Glu Asn
   180 185 190
   Gly Gly Leu Val Arg Glu Asn Leu Arg Leu Lys Ala Glu Val Asp Asn
   195 200 205
   Arg Ser Pro Gln Lys Phe Gly Arg Phe Thr Val Ala Ala Leu Gln Ser
   210 215 220
   Lys Val Glu Gln Tyr Glu Arg Glu Thr Asn Arg Leu Lys Lys Ala Leu
   225 230 235 240
   Glu Arg Ser Asp Lys Tyr Ile Glu Glu Leu Glu Ser Gln Val Thr Gln
   245 250 255
   Leu Lys His Ser Ser Glu Glu Lys Glu Ala Met Asp Ser Ile Cys Gln
   260 265 270
   Arg Val Leu Ser Ala Asp Gly Lys Arg Ile Lys Asp Ser Glu Glu Asp
   275 280 285
   Met Val Ser Lys Asn Gln Cys Asp Asn Ala Arg Thr Gln Ser Gly Ser
   290 295 300
   Ser Thr Ser Ser Ser His Leu Thr Lys Pro Ser Ser Ser Arg Leu Ser
   305 310 315 320
   Asp Thr Ser Ser Ala Arg Gln Glu Ser Thr Ser Lys Thr Glu Pro Asn
   325 330 335
   Ser Ser Lys Asn Lys Asp Leu Tyr Gln Lys Arg Val Glu Ile Met Leu
   340 345 350
   Asp Val Thr Asp Ala Asn Met Asp Ala Tyr Leu Glu Arg Glu Trp Gly
               355                 360                 365
   Asn Lys Pro Ser Asp Cys Val Pro Tyr Lys Asp Glu Glu Leu Tyr Asp
           370                 375                 380
   Leu Pro Ala Pro Cys Thr Pro Leu Ser Leu Ser Cys Leu Gln Leu Asn
   385                 390                 395                 400
   Thr Pro Glu Asn Arg Glu Ser Ser Val Val Lys Ala Gly Ser Ser Arg
                   405                 410                 415
   Lys His Ser Asn His Leu Arg Lys Leu Val Phe Asp Asp Phe Cys Asp
               420                 425                 430
   Ser Thr Asn Val Cys Asn Lys Asp Ser Ser Glu Asp Asp Ile Ser Arg
           435                 440                 445
   Ser Glu Asn Glu Lys Lys Ser Glu Cys Phe Thr Ser Pro Lys Thr Gly
       450                 455                 460
   Phe Trp Asp Cys Cys Ser Thr Ser Tyr Ala Gln Ser Leu Asp Phe Glu
   465                 470                 475                 480
   Ser Ser Glu Gly Asn Asn Met Ala Asn Ser Val Gly Glu Ile Ser Ser
                   485                 490                 495
   Lys Leu Ser Glu Lys Ser Gly Ser Cys Leu Ser Lys Lys Leu Asn Ser
               500                 505                 510
   Ile Arg Ser Phe Glu Ile Asn Arg Thr Arg Thr Ser Ser Glu Ala Ser
           515                 520                 525
   Met Asp Ala Ala Tyr Leu Asp Lys Ile Ser Glu Leu Asp Ser Met Met
       530                 535                 540
   Ser Glu Ser Asp Asn Ser Lys Ser Pro Cys Asn Asn Gly Phe Lys Ser
   545                 550                 555                 560
   Val Asp Leu Asp Gly Leu Ser Lys Ser Ser Gln Gly Ser Glu Phe Leu
                   565                 570                 575
   Glu Glu Pro Glu Lys Leu Glu Glu Arg Thr Lys Leu Ser Leu Ser Lys
               580                 585                 590
   Gly Ser Leu Thr Thr Asp Gln Leu Ala Asn Gly Asn Glu Trp Lys Pro
           595                 600                 605
   Ala Ser Phe Phe Leu Leu Ser Pro Ser Asp Gln Glu Met Asn Glu Asp
       610                 615                 620
   Phe Ser Leu His Pro Thr Ser Asn Pro Gly Thr Asn Glu Ile Lys Pro
   625                 630                 635                 640
   Pro Ser Cys Leu Phe Gln Thr Glu Phe Ser Gln Asn Val Leu Leu Ser
                   645                 650                 655
   Ser Ser His Arg Leu Phe Glu Asp Gln Arg Phe Gly Ser Ser Leu Phe
               660                 665                 670
   Lys Met Ser Ser Glu Met His Gly Leu Gly Asn His Leu Gln Ser Pro
           675                 680                 685
   Trp Ser Thr Ser Phe Val Pro Glu Lys Arg Asn Lys Asn Met Asn Gln
       690                 695                 700
   Ser Thr Lys Arg Lys Ile Gln Ser Ser Leu Ser Asn Ala Ser Pro Ser
   705                 710                 715                 720
   Lys Ala Thr Lys Ser
                   725
<210> 14
   <211> 727
   <212> PRT
   <213> Otolemur garnettii
<400> 14
   Met Ala Gln Thr Val Gln Asn Val Thr Leu Ser Leu Thr Leu Pro Ile
   1               5                   10                  15
   Thr Cys His Ile Cys Leu Gly Lys Val Arg Gln Pro Val Ile Cys Ile
               20                  25                  30
   Asn Asn His Val Phe Cys Ser Ile Cys Ile Asp Leu Trp Leu Lys Asn
           35                  40                  45
   Asn Ser Gln Cys Pro Ala Cys Arg Val Pro Ile Thr Pro Ala Asn Pro
       50                  55                  60
   Cys Lys Glu Ile Ile Gly Gly Thr Ser Glu Ser Glu Pro Met Leu Ser
   65                  70                  75                  80
   His Thr Val Arg Lys His Leu Arg Lys Thr Arg Leu Glu Leu Leu His
                   85                  90                  95
   Lys Glu Tyr Glu Asp Glu Ile Asp Cys Leu Gln Lys Glu Val Glu Glu
               100                 105                 110
   Leu Lys Ser Lys Asn Leu Ser Leu Glu Ser Gln Ile Lys Thr Ile Leu
           115                 120                 125
   Asp Pro Leu Thr Leu Ile Gln Asp Asn Gln Asn Glu Asp Lys His Leu
       130                 135                 140
   Val Ile Asp Asn Ser Ser Lys Ile Asp Gln Glu Thr Val Ala Glu Trp
   145                 150                 155                 160
   Lys Lys Lys Leu Arg Thr Ala Asn Glu Ile Tyr Glu Lys Val Lys Asp
                   165                 170                 175
   Asp Val Asp Lys Leu Lys Glu Ala Asn Lys Lys Leu Lys Leu Glu Asn
               180                 185                 190
   Gly Gly Leu Val Arg Glu Asn Leu Arg Leu Lys Ala Glu Val Asp Asn
           195                 200                 205
   Arg Ser Pro Gln Lys Phe Gly Arg Phe Thr Val Ala Ala Leu Gln Ser
       210                 215                 220
   Lys Val Glu Gln Tyr Glu Arg Glu Thr Asn Arg Leu Lys Lys Ala Leu
   225                 230                 235                 240
   Glu Arg Ser Asp Lys Tyr Ile Glu Glu Leu Glu Ser Gln Val Ala Gln
                   245                 250                 255
   Leu Lys Lys Ser Gly Lys Glu Lys Glu Ala Met Asn Ser Ile Cys Gln
               260                 265                 270
   Arg Ser Leu Ser Ala Asp Gly Met Gly Gly Lys Gly Ser Glu Glu Asp
           275                 280                 285
   Val Ala Ser Lys Asn Gln Gly Asp Ser Ala Ser Lys Gln Ser Gly Ser
       290                 295                 300
   Ser Thr Ser Ser Ser Ser His Leu Ala Lys Ala Ser Ser Ser Lys Leu
   305                 310                 315                 320
   Ser Asp Thr Gly Ser Ala Arg Gln Glu Ser Thr Ile Asn Thr Asp Leu
                   325                 330                 335
   Asn Cys Ser Lys Asn Lys Asp Leu Tyr Gln Lys Arg Val Glu Ile Met
               340                 345                 350
   Leu Asp Val Thr Asp Thr His Met Asp Thr Tyr Leu Glu Ser Glu Trp
   355 360 365
   Asp Asn Lys Pro Ser Asp Cys Val Pro Tyr Lys Asp Glu Glu Leu Tyr
   370 375 380
   Asp Leu Pro Ala Pro Cys Thr Pro Leu Ser Leu Ser Cys Leu Gln Leu
   385 390 395 400
   Ser Thr Pro Glu Asn Arg Glu Ser Ser Thr Val Lys Ala Gly Gly Ser
   405 410 415
   Ile Lys His Ser Asn His Leu Arg Lys Leu Val Phe Asp Asp Phe Cys
   420 425 430
   Glu Ser Ser Ser Val Ser Ile Lys Asp Ser Ser Glu Asp Asp Thr Asn
   435 440 445
   Arg Asn Glu Asn Asp Asn Lys Ser Glu Cys Phe Thr Thr Pro Lys Thr
   450 455 460
   Gly Phe Trp Asp Cys Cys Ser Thr Ser Tyr Ala Gln Ser Leu Asp Phe
   465 470 475 480
   Glu Ser Ser Glu Gly Asn Thr Ile Ala Asn Ser Val Gly Glu Ile Ser
   485 490 495
   Ser Lys Leu Gly Glu Lys Pro Gly Ser Cys Leu Ser Lys Arg Leu Asn
   500 505 510
   Ser Ile Arg Ser Phe Glu Met Asn Arg Thr Arg Thr Ser Ser Glu Ala
   515 520 525
   Ser Met Asp Ala Ala Tyr Leu Asp Lys Ile Ser Glu Leu Asp Ser Met
   530 535 540
   Met Ser Glu Ser Asp Asn Ser Lys Ser Pro Cys Asn Asn Gly Phe Lys
   545 550 555 560
   Ser Val Asp Leu Asp Gly Leu Ser Lys Ser Ser Gln Gly Ser Glu Phe
   565 570 575
   Leu Gly Glu Ser Asp Asn Leu Glu Glu Arg Thr Lys Leu Asn Leu Ser
   580 585 590
   Lys Ser Ser Leu Thr Thr Ala Asp His Leu Glu Asn Gly Asn Glu Trp
   595 600 605
   Lys Pro Thr Ser Phe Phe Leu Leu Ser Pro Ser Asp Gln Glu Met Asn
   610 615 620
   Glu Asp Phe Ser Leu His Pro Ser Ser Asn Pro Gly Thr Asn Glu Thr
   625 630 635 640
   Lys Pro Pro Ser Cys Leu Phe Gln Thr Glu Phe Ser Gln Gly Ile Leu
   645 650 655
   Leu Ser Ser Ser His Arg Leu Phe Glu Asp Gln Arg Phe Gly Ser Ser
   660 665 670
   Leu Phe Lys Met Ser Ser Glu Met His Gly Leu His Asn His Leu Gln
   675 680 685
   Ser Pro Trp Ser Thr Ser Phe Val Pro Glu Lys Arg Asn Lys Asn Val
   690 695 700
   Ser Gln Ser Thr Lys Arg Lys Ile Gln Ser Ser Leu Ser Asn Ala Ser
   705 710 715 720
   Pro Ser Lys Ala Thr Lys Ser
   725
<210> 15
   <211> 725
   <212> PRT
   <213> Loxodonta africana
<400> 15
   Met Ala Gln Thr Val Gln Asn Val Thr Leu Ser Leu Thr Leu Pro Ile
   1 5 10 15
   Thr Cys His Ile Cys Leu Gly Lys Val Arg Gln Pro Val Ile Cys Ile
   20 25 30
   Asn Asn His Val Phe Cys Ser Ile Cys Ile Asp Leu Trp Leu Lys Asn
   35 40 45
   Asn Ser Gln Cys Pro Ala Cys Arg Val Pro Ile Thr Pro Glu Asn Pro
   50 55 60
   Cys Lys Glu Ile Ile Gly Gly Thr Ser Glu Ser Glu Pro Met Leu Ser
   65 70 75 80
   His Thr Val Arg Lys His Leu Arg Lys Thr Arg Leu Glu Leu Leu His
   85 90 95
   Lys Glu Tyr Glu Asp Glu Ile Asp Cys Leu Gln Lys Glu Val Glu Glu
   100 105 110
   Leu Lys Ser Lys Asn Leu Ser Leu Glu Ser Gln Ile Lys Thr Ile Leu
   115 120 125
   Asp Pro Leu Thr Leu Met Gln Gly Asn Gln Asn Glu Asp Lys His Thr
   130 135 140
   Val Ala Asp Asn Pro Ser Lys Ile Asp Ser Glu Ala Val Ala Glu Trp
   145 150 155 160
   Lys Lys Lys Leu Arg Thr Ala Asn Glu Ile Tyr Glu Lys Val Lys Asp
   165 170 175
   Asp Val Asp Lys Leu Lys Glu Ala Asn Lys Lys Leu Lys Leu Glu Asn
   180 185 190
   Gly Gly Leu Val Arg Glu Asn Leu Arg Leu Lys Ala Glu Val Asp Asn
   195 200 205
   Arg Ser Pro Gln Lys Phe Gly Arg Phe Thr Val Ala Ala Leu Gln Ser
   210 215 220
   Lys Val Glu Gln Tyr Glu Arg Glu Thr Asn Arg Leu Lys Lys Ala Leu
   225 230 235 240
   Glu Arg Ser Asp Lys Tyr Ile Glu Glu Leu Glu Ser Gln Val Ala Gln
   245 250 255
   Leu Lys Asn Ser Ser Asp Glu Lys Glu Ala Val Asn Ser Ile Cys Gln
   260 265 270
   Arg Val Leu Ser Thr Asp Gly Ser Gly Asn Thr Gly Ser Glu Glu Asp
   275 280 285
   Met Ala Ser Lys Ser Gln Gly Asp Gly Ala Arg Lys Gln Pro Gly Ser
   290 295 300
   Ser Thr Ser Ser Ser Ser His Leu Ala Lys Pro Ser Ser Ser Arg Leu
   305 310 315 320
   Ser Asp Thr Ser Ser Val Arg Gln Glu Ser Thr Ser Lys Thr Glu Ser
   325 330 335
   Asn His Ser Asn Lys Asp Leu Tyr Gln Lys Gln Val Glu Ile Met Leu
               340                 345                 350
   Asp Val Thr Asp Thr Ser Met Asp Thr Tyr Leu Glu Ser Glu Trp Gly
           355                 360                 365
   Asn Lys Ala Ser Glu Cys Val Pro Tyr Lys Asp Glu Glu Leu Tyr Asp
       370                 375                 380
   Leu Pro Ala Pro Cys Thr Pro Leu Ser Leu Ser Cys Leu Gln Leu Ser
   385                 390                 395                 400
   Thr Pro Glu Thr Arg Glu Ser Ser Val Val Lys Ala Gly Gly Ser Lys
                   405                 410                 415
   Lys His Ser Asn His Leu Arg Lys Leu Val Phe Asp Asp Phe Cys Asp
               420                 425                 430
   Ser Pro Asn Val Cys Asn Lys Asp Ser Ser Gly Asp Ala Ile Ser Arg
           435                 440                 445
   Ser Glu Asn Glu Lys Lys Ser Asp Cys Phe Thr Ser Pro Lys Thr Arg
       450                 455                 460
   Phe Trp Asp Cys Cys Ser Thr Ser Tyr Ala Gln Ser Leu Gly Phe Glu
   465                 470                 475                 480
   Ser Ser Glu Gly Asn Thr Ile Ala Asn Ser Val Gly Glu Met Ser Ser
                   485                 490                 495
   Lys Ser Ser Glu Lys Ser Gly Ser Cys Leu Ser Lys Arg Leu Asn Ser
               500                 505                 510
   Ile Arg Ser Phe Glu Met Asn Arg Thr Arg Thr Ser Ser Glu Ala Ser
           515                 520                 525
   Met Asp Ala Ala Tyr Leu Asp Lys Ile Ser Glu Leu Asp Ser Met Met
       530                 535                 540
   Ser Glu Ser Asp Asn Ser Lys Ser Pro Cys Asn Asn Gly Phe Lys Ser
   545                 550                 555                 560
   Val Asp Leu Asp Gly Leu Ser Lys Ser Ala Gln Ser Thr Glu Phe Leu
                   565                 570                 575
   Asp Glu Pro Asp Lys Leu Glu Glu Arg Thr Lys Pro Asn Leu Ser Lys
               580                 585                 590
   Gly Ser Leu Thr Thr Asp Gln Leu Glu Asn Glu Asn Glu Trp Lys Ser
   595 600 605
   Thr Ser Phe Phe Leu Leu Pro Pro Ser Asp Gln Glu Met Asn Glu Asp
   610 615 620
   Phe Ser Leu His Pro Ser Ser Asn Pro Gly Thr Asn Glu Ile Lys Pro
   625 630 635 640
   Pro Ser Cys Leu Phe Gln Thr Glu Phe Ser Gln Asn Val Leu Leu Ser
   645 650 655
   Ser Ser His Gln Leu Phe Glu Asp Gln Arg Phe Gly Ser Ser Leu Phe
   660 665 670
   Lys Met Ser Ser Glu Met His Gly Leu His Asn His Leu Gln Ser Pro
   675 680 685
   Trp Ser Thr Ser Phe Val Ala Glu Lys Arg Asn Lys Asn Val Asn His
   690 695 700
   Ser Ala Lys Arg Lys Ile Gln Ser Ser Leu Ser Asn Ala Ser Pro Ser
   705 710 715 720
   Lys Ala Thr Lys Ser
   725
<210> 16
   <211> 716
   <212> PRT
   <213> Myotis lucifugus
<400> 16
   Met Ala Gln Thr Val Gln Asn Val Thr Leu Ser Leu Thr Leu Pro Ile
   1 5 10 15
   Thr Cys His Ile Cys Leu Gly Lys Val Arg Gln Pro Val Ile Cys Ile
   20 25 30
   Asn Asn His Val Phe Cys Ser Ile Cys Ile Asp Leu Trp Leu Lys Asn
   35 40 45
   Asn Ser Gln Cys Pro Ala Cys Arg Val Pro Ile Thr Pro Glu Asn Pro
   50 55 60
   Cys Lys Glu Ile Ile Gly Gly Thr Ser Glu Ser Glu Pro Met Leu Ser
   65 70 75 80
   His Thr Val Arg Lys His Leu Arg Lys Thr Arg Leu Glu Leu Leu His
                   85                  90                  95
   Lys Glu Tyr Glu Asp Glu Ile Asp Cys Leu Gln Lys Glu Val Glu Glu
               100                 105                 110
   Leu Lys Asn Lys Asn Leu Ser Leu Glu Ser Gln Ile Lys Thr Ile Leu
           115                 120                 125
   Asp Pro Leu Thr Leu Met Gln Gly Asn Gln Asn Glu Glu Lys His Pro
       130                 135                 140
   Val Ala Asp Asn Ser Ser Lys Ile Asp Pro Glu Thr Val Ala Glu Trp
   145                 150                 155                 160
   Lys Lys Lys Leu Arg Thr Ala Asn Glu Ile Tyr Glu Lys Val Lys Asp
                   165                 170                 175
   Asp Val Asp Lys Leu Lys Glu Ala Asn Lys Lys Leu Lys Leu Glu Asn
               180                 185                 190
   Gly Gly Leu Met Arg Glu Asn Leu Arg Leu Lys Ala Glu Val Asp Asn
           195                 200                 205
   Arg Ser Pro Gln Lys Phe Gly Arg Phe Thr Val Ala Ala Leu Gln Ser
       210                 215                 220
   Lys Val Glu Gln Tyr Glu Arg Glu Thr Asn Arg Leu Lys Lys Ala Leu
   225                 230                 235                 240
   Glu Arg Ser Asp Lys Tyr Ile Glu Glu Leu Glu Ser Gln Val Ala Gln
                   245                 250                 255
   Leu Lys Asn Ser Ser Glu Glu Lys Glu Ala Met Asn Ser Ile Cys Gln
               260                 265                 270
   Arg Ala Leu Ser Thr Asp Gly Lys Gly Cys Lys Gly Ser Glu Glu Asp
           275                 280                 285
   Met Ala Ser Thr Asn Gln Gly Asp Gly Ala Arg Lys Gln Leu Gly Ser
       290                 295                 300
   Ser Thr Pro Ser Ser His Leu Thr Gln Pro Ser Asn Ser Ser Ala Arg
   305                 310                 315                 320
   Gln Glu Ser Thr Ser Lys Thr Glu Leu Asn Cys Ser Lys Asn Lys Asp
                   325                 330                 335
   Leu Tyr Gln Lys Gln Val Glu Ile Met Leu Gly Val Thr Asp Ala Ser
   340 345 350
   Met Asp Thr Tyr Leu Glu Arg Glu Trp Gly Asn Lys Pro Thr Asp Ser
   355 360 365
   Thr Pro Tyr Lys Asp Glu Leu Tyr Asp Leu Pro Ala Pro Cys Thr Pro
   370 375 380
   Leu Ser Leu Ser Cys Leu Gln Leu Ser Thr Pro Glu Asn Arg Glu Ser
   385 390 395 400
   Ser Met Ala Lys Ala Gly Ser Ser Lys Lys His Ser Asn His Leu Arg
   405 410 415
   Lys Leu Val Phe Asp Asp Phe Cys Asn Ser Thr Asn Val Cys Asn Lys
   420 425 430
   Asp Ser Ser Glu Asp Asp Lys Ser Glu Asn Glu Lys Lys Ser Glu Cys
   435 440 445
   Phe Thr Ser Pro Lys Thr Gly Phe Trp Asp Cys Cys Ser Thr Ser Tyr
   450 455 460
   Ala Gln Ser Leu Asp Phe Glu Ser Ala Glu Gly Asn Thr Ile Ala Asn
   465 470 475 480
   Ser Val Gly Glu Ile Ser Ser Lys Leu Ser Glu Lys Ser Gly Ser Cys
   485 490 495
   Leu Ser Lys Arg Leu Asn Ser Ile Arg Ser Phe Glu Met Asn Arg Thr
   500 505 510
   Arg Thr Ser Ser Glu Ala Ser Met Asp Ala Ala Tyr Leu Asp Lys Ile
   515 520 525
   Ser Glu Leu Asp Ser Met Met Ser Glu Ser Asp Asn Ser Lys Ser Pro
   530 535 540
   Cys Asn Asn Gly Phe Lys Ser Val Asp Leu Asp Gly Leu Ser Lys Ser
   545 550 555 560
   Ser Gln Gly Ser Glu Phe Leu Glu Glu Pro Asp Lys Leu Glu Glu Arg
   565 570 575
   Thr Lys Pro Asn Leu Ser Lys Gly Ser Leu Thr Ser Asp Gln Leu Glu
   580 585 590
   Asn Gly Asn Glu Trp Lys Pro Ser Ser Phe Phe Leu Leu Ser Pro Ser
           595                 600                 605
   Asp Gln Glu Met Ser Glu Glu Phe Ser Leu His Pro Ser Ser Asn Ser
       610                 615                 620
   Gly Thr Asn Glu Leu Lys Pro Pro Ser Cys Leu Phe Gln Thr Glu Phe
   625                 630                 635                 640
   Ser Glu Gly Val Leu Leu Ser Ser Ser His Arg Leu Phe Glu Asp Gln
                   645                 650                 655
   Arg Phe Gly Ser Ser Leu Phe Lys Met Ser Ser Glu Met Gln Gly Leu
               660                 665                 670
   Pro Asn His Leu Gln Ser Pro Trp Ser Thr Ser Phe Val Pro Glu Lys
           675                 680                 685
   Lys Asn Lys Asn Val Asn Gln Ser Thr Lys Arg Lys Ile Gln Ser Ser
       690                 695                 700
   Leu Ser Asn Ala Ser Pro Ser Lys Ala Thr Lys Ser
   705                 710                 715
<210> 17
   <211> 724
   <212> PRT
   <213> Heterocephalus glaber
<400> 17
   Met Ala Gln Thr Val Gln Asn Val Thr Leu Ser Leu Thr Leu Pro Ile
   1               5                   10                  15
   Thr Cys His Ile Cys Leu Gly Lys Val Arg Gln Pro Val Ile Cys Ile
               20                  25                  30
   Asn Asn His Val Phe Cys Ser Ile Cys Ile Asp Leu Trp Leu Lys Asn
           35                  40                  45
   Asn Ser Gln Cys Pro Ala Cys Arg Val Pro Ile Thr Pro Glu Asn Pro
       50                  55                  60
   Cys Lys Glu Ile Ile Gly Gly Thr Ser Glu Ser Glu Pro Met Leu Ser
   65                  70                  75                  80
   His Ala Val Arg Lys His Leu Arg Lys Thr Arg Leu Glu Leu Leu His
                   85                  90                  95
   Lys Glu Tyr Glu Asp Glu Ile Asp Cys Leu Gln Lys Glu Val Glu Glu
               100                 105                 110
   Leu Lys Ser Lys Asn Leu Ser Leu Glu Ser Gln Ile Lys Ala Ile Leu
           115                 120                 125
   Asp Pro Leu Thr Leu Met Gln Gly Asn Gln Asn Glu Asp Lys His Pro
       130                 135                 140
   Val Ala Asp Asn Ser Ser Lys Ile Asp Leu Glu Thr Val Glu Glu Trp
   145                 150                 155                 160
   Lys Lys Lys Leu Arg Thr Ala Asn Glu Ile Tyr Glu Lys Val Lys Asp
                   165                 170                 175
   Asp Val Asp Lys Leu Lys Glu Ala Asn Lys Lys Leu Lys Leu Glu Asn
               180                 185                 190
   Gly Gly Leu Val Arg Glu Asn Leu Arg Leu Lys Ala Glu Val Asp Asn
           195                 200                 205
   Arg Ser Pro Gln Lys Phe Gly Arg Phe Thr Val Ala Ala Leu Gln Ser
       210                 215                 220
   Lys Val Glu Gln Tyr Glu Arg Glu Thr Ser Arg Leu Lys Lys Ala Leu
   225                 230                 235                 240
   Glu Arg Ser Asp Lys Tyr Ile Glu Glu Leu Glu Ser Gln Val Ala Gln
                   245                 250                 255
   Leu Lys Thr Ser Gly Glu Glu Lys Glu Ala Met Asp Ala Ile Cys Gln
               260                 265                 270
   Arg Ala Leu Ser Ala Asp Gly Lys Gly Gly Ser Lys Glu Asp Val Glu
           275                 280                 285
   Ser Asn Ser Gln Gly Asp Ser Ala Arg Lys Gln Pro Arg Leu Ser Thr
       290                 295                 300
   Ser Ser Ser Ser Asn Leu Ala Lys Pro Cys Ile Ser Arg Leu Ser Asp
   305                 310                 315                 320
   Thr Ser Thr Ala Arg Gln Glu Ser Thr Ser Lys Thr Glu Pro Asn His
                   325                 330                 335
   Pro Lys Asn Lys Asp Leu Tyr Gln Lys Pro Val Glu Ile Met Leu Asp
   340 345 350
   Val Thr Asp Thr Ser Met Asp Thr Tyr Leu Glu Gly Glu Trp Ser Asn
   355 360 365
   Lys Pro Ser Asn Cys Val Pro Tyr Lys Asp Glu Glu Leu Tyr Asp Leu
   370 375 380
   Pro Ala Pro Cys Thr Pro Leu Ser Leu Ser Cys Leu Gln Leu Asn Thr
   385 390 395 400
   Pro Glu Asn Arg Glu Ser Ser Val Val Lys Ala Gly Gly Ser Lys Lys
   405 410 415
   His Ser Asn His Leu Arg Lys Leu Val Phe Asp Asp Phe Cys Asp Ser
   420 425 430
   Ser Asn Val Cys Thr Lys Glu Cys Ser Glu Ala Asp Ile Ser Arg Asn
   435 440 445
   Glu Asn Glu Lys Lys Ser Glu Cys Phe Ala Ser Pro Lys Thr Gly Phe
   450 455 460
   Trp Asp Cys Cys Ser Thr Ser Tyr Ala Gln Ser Leu Asp Phe Glu Asn
   465 470 475 480
   Ser Glu Gly Asn Ile Ile Ala Asn Ser Val Gly Glu Ile Cys Pro Lys
   485 490 495
   Leu Ile Glu Lys Ala Ser Ser Cys Leu Ser Lys Arg Leu Asn Ser Ile
   500 505 510
   Arg Ser Phe Glu Met Asn Arg Thr Arg Thr Ser Ser Glu Ala Ser Met
   515 520 525
   Asp Ala Ala Tyr Leu Asp Lys Ile Ser Glu Leu Asp Ser Met Met Ser
   530 535 540
   Glu Ser Asp Asn Ser Lys Ser Pro Cys Asn Asn Gly Phe Lys Ser Val
   545 550 555 560
   Asp Leu Asp Gly Leu Ser Lys Ser Ser Gln Gly Asn Glu Phe Leu Glu
   565 570 575
   Glu Pro Val Lys Leu Glu Glu Arg Thr Glu Leu Lys Leu Arg Lys Gly
   580 585 590
   Ser Leu Thr Ala Asp Gln Leu Glu Asn Gly Asn Glu Trp Lys Pro Thr
   595 600 605
   Ser Phe Phe Leu Leu Ser Pro Ser Asp Gln Glu Met Asn Glu Asp Phe
   610 615 620
   Ala Leu His Pro Thr Ser Asn Pro Glu Thr Asn Glu Ile Lys Pro Pro
   625 630 635 640
   Ser Cys Leu Phe Gln Pro Glu Phe Ser Gln Gly Val Leu Leu Ser Ser
   645 650 655
   Ser His Gly Leu Phe Glu Asp Gln Arg Phe Gly Ser Ser Leu Phe Lys
   660 665 670
   Leu Ser Ser Glu Met His Ser Leu His Asn His Leu Gln Ser Pro Trp
   675 680 685
   Ser Ala Ser Phe Val Pro Glu Lys Arg Asn Lys Asn Val Asn Gln Ser
   690 695 700
   Thr Lys Arg Lys Ile His Ser Ser Leu Ser Asn Ala Ser Pro Ser Lys
   705 710 715 720
   Ala Thr Lys Ser
<210> 18
   <211> 751
   <212> PRT
   <213> Pteropus alecto
<400> 18
   Met Ala Leu Leu Gly Cys Arg Ser Arg Pro Arg Ala Val Pro Val Ala
   1 5 10 15
   Ala Arg Ile Arg Gly Arg Arg Asp Phe Arg Val Gly Gly Ile Leu Asn
   20 25 30
   Ala Ala Met Ala Gln Thr Val Gln Asn Val Thr Leu Ser Leu Thr Leu
   35 40 45
   Pro Ile Thr Cys His Ile Cys Leu Gly Lys Val Arg Gln Pro Val Ile
   50 55 60
   Cys Ile Asn Asn His Val Phe Cys Ser Val Cys Ile Asp Leu Trp Leu
   65 70 75 80
   Lys Asn Asn Ser Gln Cys Pro Ala Cys Arg Val Pro Ile Thr Pro Glu
                   85                  90                  95
   Asn Pro Cys Lys Glu Ile Ile Gly Gly Thr Ser Glu Ser Glu Pro Met
               100                 105                 110
   Leu Ser His Thr Val Arg Lys His Leu Arg Lys Thr Arg Leu Glu Leu
           115                 120                 125
   Leu His Lys Glu Tyr Glu Asp Glu Ile Asp Cys Leu Gln Lys Glu Met
       130                 135                 140
   Glu Glu Leu Lys Ser Lys Asn Leu Ser Leu Glu Ser Gln Ile Lys Thr
   145                 150                 155                 160
   Ile Leu Asp Pro Leu Ile Phe Thr Gln Gly Asn Gln Ser Glu Asp Lys
                   165                 170                 175
   His Pro Val Val Asp Asn Pro Ser Lys Ile Asp Pro Glu Thr Val Ala
               180                 185                 190
   Glu Trp Lys Lys Lys Leu Arg Thr Ala Asn Glu Ile Tyr Glu Lys Val
           195                 200                 205
   Lys Asp Asp Val Asp Lys Leu Lys Glu Ala Asn Lys Lys Leu Lys Leu
       210                 215                 220
   Glu Asn Gly Gly Leu Met Arg Glu Asn Leu Arg Leu Lys Ala Glu Val
   225                 230                 235                 240
   Asp Asn Arg Ser Pro Gln Lys Phe Gly Arg Phe Thr Ile Ala Ala Leu
                   245                 250                 255
   Gln Ser Lys Val Glu Gln Tyr Glu Arg Glu Thr Asn Arg Leu Lys Lys
               260                 265                 270
   Ala Leu Glu Arg Ser Asp Lys Tyr Ile Glu Glu Leu Glu Ser Gln Ile
           275                 280                 285
   Ala Gln Leu Lys Asn Ser Ser Glu Glu Lys Glu Ala Met Asn Ser Ile
       290                 295                 300
   Cys Gln Asn Thr Leu Ser Thr Asp Asp Lys Gly Ser Lys Gly Ser Lys
   305                 310                 315                 320
   Glu Asp Met Pro Ser Lys Asn Gln Gly Asp Gly Ala Arg Lys Gln Leu
                   325                 330                 335
   Cys Ser Ala Thr Ser Ser Ser His Leu Ala Gln Pro Ser Asn Ser Ser
               340                 345                 350
   Ala Arg Gln Glu Ser Thr Ser Lys Thr Glu Pro Asn Cys Ser Lys Asn
           355                 360                 365
   Lys Asp Leu Tyr Gln Lys Gln Val Glu Ile Met Leu Asp Val Thr Asp
       370                 375                 380
   Thr Ser Met Asp Thr Tyr Leu Glu Arg Glu Trp Gly Asn Lys Pro Ser
   385                 390                 395                 400
   Asp Cys Ala Pro Tyr Lys Ala Glu Glu Leu Tyr Asp Leu Pro Ala Pro
                   405                 410                 415
   Cys Thr Pro Leu Ser Leu Ser Cys Leu Gln Leu Ser Thr Pro Glu Asn
               420                 425                 430
   Arg Glu Asn Ser Val Gly Lys Ala Gly Cys Ser Lys Asn His Ser Asn
           435                 440                 445
   His Leu Arg Lys Leu Val Phe Asp Asp Phe Cys Asp Ser Pro Asn Val
       450                 455                 460
   Cys Asn Lys Glu Ser Ser Glu Asp Gly Arg Ser Glu Asn Glu Lys Lys
   465                 470                 475                 480
   Ser Glu Cys Phe Pro Ser Pro Lys Thr Gly Phe Trp Asp Cys Cys Ser
                   485                 490                 495
   Thr Ser Tyr Ala Gln Ser Leu Asp Phe Glu Asn Ser Glu Gly Asn Leu
               500                 505                 510
   Ile Ala Asn Ser Val Gly Glu Ile Ser Ser Lys Phe Ser Glu Lys Ser
           515                 520                 525
   Gly Ser Cys Leu Ser Lys Arg Leu Asn Ser Ile Arg Ser Phe Glu Met
       530                 535                 540
   Asn Arg Thr Arg Thr Ser Ser Glu Ala Ser Met Asp Ala Ala Tyr Leu
   545                 550                 555                 560
   Asp Lys Ile Ser Glu Leu Asp Ser Met Met Ser Glu Ser Asp Asn Ser
                   565                 570                 575
   Lys Ser Pro Cys Asn Asn Gly Phe Lys Ser Val Asp Leu Asp Gly Leu
               580                 585                 590
   Ser Lys Ser Ser Gln Gly Ser Glu Phe Leu Glu Glu Pro Asp Glu Leu
           595                 600                 605
   Glu Glu Arg Thr Lys Pro Asp Leu Ser Lys Gly Ser Leu Thr Thr Gly
       610                 615                 620
   Gln Leu Glu Asn Gly Asn Glu Trp Lys Pro Thr Ser Tyr Phe Phe Leu
   625                 630                 635                 640
   Ser Pro Ser Asp Gln Glu Met Asn Glu Asp Phe Ser Leu His Pro Ser
                   645                 650                 655
   Ser Asp Ser Gly Thr Thr Glu Ile Lys Pro Pro Ser Cys Leu Phe Gln
               660                 665                 670
   Thr Glu Phe Ser Gln Ser Val Leu Leu Ser Ser Ser His Arg Leu Phe
           675                 680                 685
   Glu Asp Gln Arg Phe Glu Ser Ser Leu Phe Lys Met Ser Ser Glu Met
       690                 695                 700
   His Gly Leu His Asn His Leu Gln Ser Pro Trp Ser Thr Ser Phe Val
   705                 710                 715                 720
   Pro Glu Lys Arg Asn Lys Asn Val Asn Gln Ser Thr Lys Arg Lys Ile
                   725                 730                 735
   Gln Ser Ser Leu Ser Asn Ala Ser Pro Ser Lys Ala Thr Lys Ser
               740                 745                 750
<210> 19
   <211> 717
   <212> PRT
   <213> Bos grunniens
<400> 19
   Met Ala Gln Thr Val Gln Asn Val Thr Leu Ser Leu Thr Leu Pro Ile
   1               5                   10                  15
   Thr Cys His Ile Cys Leu Gly Lys Val Arg Gln Pro Val Ile Cys Ile
               20                  25                  30
   Asn Asn His Val Phe Cys Ser Val Cys Ile Asp Leu Trp Leu Lys Asn
           35                  40                  45
   Asn Ser Gln Cys Pro Ala Cys Arg Val Pro Ile Thr Pro Glu Asn Pro
       50                  55                  60
   Cys Lys Glu Ile Ile Gly Gly Thr Ser Glu Ser Glu Pro Met Leu Ser
   65 70 75 80
   His Thr Val Arg Lys His Leu Arg Lys Thr Arg Leu Glu Leu Leu His
   85 90 95
   Lys Glu Tyr Glu Asp Glu Ile Asp Cys Leu Gln Lys Glu Val Asp Glu
   100 105 110
   Leu Lys Ser Lys Asn Leu Ser Leu Glu Ser Gln Ile Lys Thr Ile Leu
   115 120 125
   Asp Pro Leu Thr Leu Met Gln Gly Asn Gln Asn Glu Asp Lys Leu Pro
   130 135 140
   Val Ala Asp Asn Pro Ser Lys Ile Asp Pro Glu Thr Val Ala Glu Trp
   145 150 155 160
   Lys Lys Arg Leu Arg Thr Ala Asn Glu Ile Tyr Glu Lys Val Lys Asp
   165 170 175
   Asp Val Asp Lys Leu Lys Glu Ala Asn Lys Lys Leu Lys Leu Glu Asn
   180 185 190
   Gly Gly Leu Val Arg Glu Asn Leu Arg Leu Lys Ala Glu Val Asp Asn
   195 200 205
   Arg Ser Pro Gln Lys Phe Gly Arg Phe Thr Val Ala Ala Leu Gln Ser
   210 215 220
   Lys Val Glu Gln Tyr Glu Arg Glu Thr Asn Arg Leu Lys Lys Ala Leu
   225 230 235 240
   Glu Arg Ser Asp Lys Tyr Ile Glu Glu Leu Glu Ser Gln Ile Thr Gln
   245 250 255
   Leu Lys Asn Ser Ser Asp Glu Lys Glu Ala Met Asn Ala Ile Cys Gln
   260 265 270
   Arg Ala Leu Ser Thr Asp Gly Lys Gly Ser Lys Gly Thr Glu Glu Asp
   275 280 285
   Met Ala Ser Lys Asn Gln Gly Asp Gly Ala Arg Lys Gln Leu Gly Ser
   290 295 300
   Ala Thr Ser Ser Ser His Leu Ala Lys Pro Ser Ser Ser Ser Val Arg
   305 310 315 320
   Gln Glu Gly Thr Ser Lys Thr Glu Pro Asn Cys Ser Lys Asn Gln Asp
   325 330 335
   Leu Tyr Gln Lys Arg Val Glu Thr Met Leu Thr Val Thr Asp Thr Ser
   340 345 350
   Met Asp Thr Tyr Leu Glu Arg Glu Trp Gly Asn Lys Pro Ser Asp Cys
   355 360 365
   Met Pro Tyr Lys Asp Glu Glu Leu Tyr Asp Leu Pro Ala Ser Cys Ala
   370 375 380
   Pro Leu Ser Leu Ser Cys Leu Gln Leu Asn Thr Pro Asp Asn Arg Glu
   385 390 395 400
   Ser Ser Val Val Lys Ala Gly Gly Ser Lys Lys Gln Ser Asn His Leu
   405 410 415
   Arg Lys Leu Met Phe Asp Glu Phe Cys Asp Ser Pro Asn Val Cys Asn
   420 425 430
   Lys Asp Ser Ser Glu Asp Asp Arg Ser Glu Ser Glu Lys Lys Ser Glu
   435 440 445
   Cys Phe Thr Ser Pro Lys Thr Gly Phe Trp Asp Cys Cys Ser Thr Ser
   450 455 460
   Tyr Ala Pro Ser Leu Asp Phe Glu Asn Ser Glu Glu Asn Thr Ile Ala
   465 470 475 480
   Asn Cys Val Gly Glu Ile Ser Ser Lys Leu Ser Glu Lys Pro Gly Ser
   485 490 495
   Cys Leu Ser Lys Arg Leu Asn Ser Leu Arg Ser Phe Glu Met Asn Arg
   500 505 510
   Thr Arg Thr Ser Ser Glu Ala Ser Met Asp Ala Ala Tyr Leu Asp Lys
   515 520 525
   Ile Ser Glu Leu Asp Ser Met Met Ser Glu Ser Asp Asn Ser Lys Ser
   530 535 540
   Pro Cys Asn Asn Gly Phe Lys Ser Leu Asp Leu Asp Gly Leu Ser Lys
   545 550 555 560
   Ser Ser Gln Cys Ser Glu Phe Leu Glu Glu Thr Asp Lys Leu Glu Glu
   565 570 575
   Arg Thr Lys Pro Asn Leu Ser Lys Gly Ser Leu Thr Thr Asp Gln Leu
   580 585 590
   Glu Asn Gly Asn Glu Trp Lys Pro Asn Ser Phe Phe Leu Leu Ser Pro
   595 600 605
   Ser Asp Gln Glu Met Asn Glu Asp Phe Ser Leu His Ser Ser Ser Asn
   610 615 620
   Pro Gly Thr Asn Glu Ile Lys Pro Pro Ser Cys Leu Phe Gln Thr Glu
   625 630 635 640
   Phe Ser Gln Gly Val Leu Leu Ser Ser Thr Asn Arg Leu Phe Glu Asp
   645 650 655
   Gln Arg Phe Gly Ser Ser Leu Phe Lys Met Ser Ser Glu Met Pro Gly
   660 665 670
   Leu His Thr His Leu Gln Ser Pro Trp Ser Thr Ser Phe Val Pro Glu
   675 680 685
   Lys Arg Asn Lys Asn Val Asn Gln Ser Ala Lys Arg Lys Ile Gln Ser
   690 695 700
   Ser Leu Ser Asn Ala Ser Pro Ser Lys Ala Thr Lys Ser
   705 710 715
<210> 20
   <211> 717
   <212> PRT
   <213> Bos taurus
<400> 20
   Met Ala Gln Thr Val Gln Asn Val Thr Leu Ser Leu Thr Leu Pro Ile
   1 5 10 15
   Thr Cys His Ile Cys Leu Gly Lys Val Arg Gln Pro Val Ile Cys Ile
   20 25 30
   Asn Asn His Val Phe Cys Ser Val Cys Ile Asp Leu Trp Leu Lys Asn
   35 40 45
   Asn Ser Gln Cys Pro Ala Cys Arg Val Pro Ile Thr Pro Glu Asn Pro
   50 55 60
   Cys Lys Glu Ile Ile Gly Gly Thr Ser Glu Ser Glu Pro Met Leu Ser
   65 70 75 80
   His Thr Val Arg Lys His Leu Arg Lys Thr Arg Leu Glu Leu Leu His
   85 90 95
   Lys Glu Tyr Glu Asp Glu Ile Asp Cys Leu Gln Lys Glu Val Asp Glu
   100 105 110
   Leu Lys Ser Lys Asn Leu Ser Leu Glu Ser Gln Ile Lys Thr Ile Leu
   115 120 125
   Asp Pro Leu Thr Leu Met Gln Gly Asn Gln Asn Glu Asp Lys Leu Pro
   130 135 140
   Val Ala Asp Asn Pro Ser Lys Ile Asp Pro Glu Thr Val Ala Glu Trp
   145 150 155 160
   Lys Lys Arg Leu Arg Thr Ala Asn Glu Ile Tyr Glu Lys Val Lys Asp
   165 170 175
   Asp Val Asp Lys Leu Lys Glu Ala Asn Lys Lys Leu Lys Leu Glu Asn
   180 185 190
   Gly Gly Leu Val Arg Glu Asn Leu Arg Leu Lys Ala Glu Val Asp Asn
   195 200 205
   Arg Ser Pro Gln Lys Phe Gly Arg Phe Thr Val Ala Ala Leu Gln Ser
   210 215 220
   Lys Val Glu Gln Tyr Glu Arg Glu Thr Asn Arg Leu Lys Lys Ala Leu
   225 230 235 240
   Glu Arg Ser Asp Lys Tyr Ile Glu Glu Leu Glu Ser Gln Ile Thr Gln
   245 250 255
   Leu Lys Asn Ser Ser Asp Glu Lys Glu Ala Met Asn Ala Ile Cys Gln
   260 265 270
   Arg Ala Leu Ser Thr Asp Gly Lys Gly Ser Lys Gly Thr Glu Glu Asp
   275 280 285
   Met Ala Ser Lys Asn Gln Gly Asp Gly Ala Arg Lys Gln Leu Gly Ser
   290 295 300
   Ala Thr Ser Ser Ser His Leu Ala Lys Pro Ser Ser Ser Ser Val Arg
   305 310 315 320
   Gln Glu Gly Thr Ser Lys Thr Glu Pro Asn Cys Ser Lys Asn Gln Asp
                   325                 330                 335
   Leu Tyr Gln Lys Arg Val Glu Thr Met Leu Thr Val Thr Asp Thr Ser
               340                 345                 350
   Met Asp Thr Tyr Leu Glu Arg Glu Trp Gly Asn Lys Pro Ser Asp Cys
           355                 360                 365
   Met Pro Tyr Lys Asp Glu Glu Leu Tyr Asp Leu Pro Ala Ser Cys Ala
       370                 375                 380
   Pro Leu Ser Leu Ser Cys Leu Gln Leu Asn Thr Pro Asp Asn Arg Glu
   385                 390                 395                 400
   Ser Ser Val Val Lys Ala Gly Gly Ser Lys Lys Gln Ser Asn His Leu
                   405                 410                 415
   Arg Lys Leu Met Phe Asp Glu Phe Cys Asp Ser Pro Asn Val Cys Asn
               420                 425                 430
   Lys Asp Ser Ser Glu Asp Asp Arg Ser Glu Ser Glu Lys Lys Ser Glu
           435                 440                 445
   Cys Phe Thr Ser Pro Lys Thr Gly Phe Trp Asp Cys Cys Ser Thr Ser
       450                 455                 460
   Tyr Ala Pro Ser Leu Asp Phe Glu Asn Ser Glu Glu Asn Thr Ile Ala
   465                 470                 475                 480
   Asn Cys Val Gly Glu Ile Ser Ser Lys Leu Ser Glu Lys Pro Gly Ser
                   485                 490                 495
   Cys Leu Ser Lys Arg Leu Asn Ser Leu Arg Ser Phe Glu Met Asn Arg
               500                 505                 510
   Thr Arg Thr Ser Ser Glu Ala Ser Met Asp Ala Ala Tyr Leu Asp Lys
           515                 520                 525
   Ile Ser Glu Leu Asp Ser Met Met Ser Glu Ser Asp Asn Ser Lys Ser
       530                 535                 540
   Pro Cys Asn Asn Gly Phe Lys Ser Leu Asp Leu Asp Gly Leu Ser Lys
   545                 550                 555                 560
   Ser Ser Gln Cys Ser Glu Phe Leu Glu Glu Thr Asp Lys Leu Glu Glu
                   565                 570                 575
   Arg Thr Lys Pro Asn Leu Ser Lys Gly Ser Leu Thr Thr Asp Gln Leu
               580                 585                 590
   Glu Asn Gly Asn Glu Trp Lys Pro Asn Ser Phe Phe Leu Leu Ser Pro
           595                 600                 605
   Ser Asp Gln Glu Met Asn Glu Asp Phe Ser Leu His Ser Ser Ser Asn
       610                 615                 620
   Pro Gly Thr Asn Glu Ile Lys Pro Pro Ser Cys Leu Phe Gln Thr Glu
   625                 630                 635                 640
   Phe Ser Gln Gly Val Leu Leu Ser Ser Thr Asn Arg Leu Phe Glu Asp
                   645                 650                 655
   Gln Arg Phe Gly Ser Ser Leu Phe Lys Met Ser Ser Glu Met Pro Gly
               660                 665                 670
   Leu His Thr His Leu Gln Ser Pro Trp Ser Thr Ser Phe Val Pro Glu
           675                 680                 685
   Lys Arg Asn Lys Asn Val Asn Gln Ser Ala Lys Arg Lys Ile Gln Ser
       690                 695                 700
   Ser Leu Ser Asn Ala Ser Pro Ser Lys Ala Thr Lys Ser
   705                 710                 715
<210> 21
   <211> 723
   <212> PRT
   <213> Cavia porcellus
<400> 21
   Met Ala Gln Ser Val Gln Asn Val Thr Leu Ser Leu Thr Leu Pro Ile
   1               5                   10                  15
   Thr Cys His Ile Cys Leu Gly Lys Val Arg Gln Pro Val Ile Cys Ile
               20                  25                  30
   Asn Asn His Val Phe Cys Ser Ile Cys Ile Asp Leu Trp Leu Lys Asn
           35                  40                  45
   Asn Ser Gln Cys Pro Ala Cys Arg Val Pro Ile Thr Pro Glu Asn Pro
       50                  55                  60
   Cys Lys Glu Ile Ile Gly Gly Ala Ser Glu Ser Glu Pro Met Leu Ser
   65                  70                  75                  80
   His Thr Val Arg Lys His Leu Arg Lys Thr Arg Leu Glu Leu Leu His
   85 90 95
   Lys Glu Tyr Glu Asp Glu Ile Asp Cys Leu Gln Lys Glu Val Glu Glu
   100 105 110
   Leu Lys Asn Lys Asn Leu Ser Leu Glu Ser Gln Ile Lys Thr Ile Leu
   115 120 125
   Asp Pro Leu Thr Leu Met Gln Gly Asn Gln Asn Glu Asp Lys Leu Pro
   130 135 140
   Val Ala Asp Asn Pro Ser Lys Ile Asp Pro Gln Thr Val Ser Glu Trp
   145 150 155 160
   Lys Lys Lys Leu Arg Thr Ala Asn Glu Ile Tyr Glu Lys Val Lys Asp
   165 170 175
   Asp Val Asp Lys Leu Lys Glu Val Asn Lys Lys Leu Lys Leu Glu Asn
   180 185 190
   Gly Gly Leu Val Arg Glu Asn Leu Arg Leu Lys Ala Glu Val Asp Asn
   195 200 205
   Arg Ser Pro Gln Lys Phe Gly Arg Phe Thr Val Ala Ala Leu Gln Ser
   210 215 220
   Lys Val Glu Gln Tyr Glu Arg Glu Thr Asn Arg Leu Lys Lys Ala Leu
   225 230 235 240
   Glu Arg Ser Asp Lys Tyr Ile Glu Glu Leu Glu Ser Gln Ile Ala Gln
   245 250 255
   Leu Lys Asn Ser Ser Glu Glu Arg Glu Thr Met Asp Ala Ile Cys Gln
   260 265 270
   Arg Ala Leu Ser Ala Asp Gly Lys Gly Gly Ser Glu Glu Asp Val Ala
   275 280 285
   Ser Asn Asn Gln Gly Asp Ser Ala Arg Lys Pro Ser Ser Ser Thr Leu
   290 295 300
   Ser Ser Ser His Leu Gly Lys Leu Ser Ser Ser Arg Leu Ser Asp Thr
   305 310 315 320
   Ser Ala Ala Arg Gln Glu Ile Thr Cys Lys Thr Glu Pro Asn Gln His
   325 330 335
   Lys Asn Lys Asp Leu Tyr Gln Lys Gln Val Glu Ile Met Leu Asp Val
   340 345 350
   Ser Asp Thr Ser Met Asn Thr Tyr Leu Glu Gly Glu Trp Gly Gly Lys
   355 360 365
   Pro Ser Asn Cys Val Ala Tyr Lys Asp Glu Glu Leu Tyr Asp Leu Pro
   370 375 380
   Ala Pro Cys Thr Pro Leu Ser Leu Ser Cys Leu Gln Leu Asn Thr Pro
   385 390 395 400
   Glu Asn Arg Glu Ser Ser Ala Val Lys Ala Gly Gly Ser Lys Lys His
   405 410 415
   Ser Asn His Leu Arg Lys Leu Val Phe Asp Asp Phe Cys Glu Ser Pro
   420 425 430
   Asn Val Cys Asn Lys Asp Cys Ser Glu Asp Asp Val Ser Arg Asn Glu
   435 440 445
   Asn Glu Lys Lys Ser Glu Cys Phe Ala Pro Pro Lys Thr Ala Phe Trp
   450 455 460
   Asp Cys Cys Ser Thr Ser Tyr Ala Gln Ser Leu Asp Phe Glu Asn Ser
   465 470 475 480
   Glu Gly Asn Ile Ile Ala Asn Ser Val Gly Glu Ile Pro Ser Lys Leu
   485 490 495
   Ser Glu Lys Ser Thr Ser Cys Leu Ser Lys Arg Leu Asn Ser Ile Arg
   500 505 510
   Ser Phe Glu Met Asn Arg Thr Arg Thr Ser Ser Glu Ala Ser Met Asp
   515 520 525
   Ala Ala Tyr Leu Asp Lys Ile Ser Glu Leu Asp Ser Met Met Ser Glu
   530 535 540
   Ser Asp Asn Ser Lys Ser Pro Cys Asn Asn Gly Phe Lys Ser Val Asp
   545 550 555 560
   Leu Asp Gly Leu Ser Lys Ser Ser Gln Gly Asn Asp Phe Leu Glu Glu
   565 570 575
   Pro Ser Lys Leu Asp Glu Arg Thr Glu Leu Asn Leu Pro Lys Ser Ser
               580                 585                 590
   Leu Thr Ala Asp Gln Leu Lys Asn Gly Asn Glu Trp Lys Pro Thr Ser
           595                 600                 605
   Phe Phe Leu Leu Ser Pro Ser Asp Gln Glu Met Asn Glu Asp Phe Ser
       610                 615                 620
   Leu His Ser Thr Ser Asn Pro Glu Thr Asn Glu Ile Lys Pro Ser Ser
   625                 630                 635                 640
   Cys Leu Phe Gln Thr Glu Phe Ser Gln Gly Val Leu Leu Ser Ser Ser
                   645                 650                 655
   His Arg Leu Phe Glu Asp Glu Arg Phe Gly Ser Ser Leu Phe Lys Met
               660                 665                 670
   Ser Ser Glu Met His Ser Leu His Asn His Leu Gln Ser Pro Trp Ser
           675                 680                 685
   Ala Ser Phe Val Pro Glu Lys Arg Asn Lys Asn Val Asn Gln Ser Thr
       690                 695                 700
   Lys Arg Lys Ile His Ser Thr Leu Ser Asn Ala Ser Pro Ser Lys Ala
   705                 710                 715                 720
   Thr Lys Ser
<210> 22
   <211> 717
   <212> PRT
   <213> Sus scrofa
<400> 22
   Met Ala Gln Thr Val Gln Asn Val Thr Leu Ser Leu Thr Leu Pro Ile
   1               5                   10                  15
   Thr Cys His Ile Cys Leu Gly Lys Val Arg Gln Pro Val Ile Cys Ile
               20                  25                  30
   Asn Asn His Val Phe Cys Ser Ile Cys Ile Asp Leu Trp Leu Lys Asn
           35                  40                  45
   Asn Ser Gln Cys Pro Ala Cys Arg Val Pro Ile Thr Pro Glu Asn Pro
       50                  55                  60
   Cys Lys Glu Ile Ile Gly Gly Thr Ser Glu Ser Glu Pro Met Leu Ser
   65 70 75 80
   His Thr Val Arg Lys His Leu Arg Lys Thr Arg Leu Glu Leu Leu His
   85 90 95
   Lys Glu Tyr Glu Asp Glu Ile Asp Cys Leu Gln Lys Glu Ile Glu Glu
   100 105 110
   Leu Lys Ser Lys Asn Leu Ser Leu Glu Ser Gln Ile Lys Thr Ile Leu
   115 120 125
   Asp Pro Leu Thr Leu Met Gln Gly Asn Gln Asn Glu Asp Lys His Pro
   130 135 140
   Val Ala Asp Asn Leu Ser Lys Val Asp Pro Glu Thr Val Ala Glu Trp
   145 150 155 160
   Lys Lys Lys Leu Arg Thr Ala Asn Glu Ile Tyr Glu Lys Val Lys Asp
   165 170 175
   Asp Val Asp Lys Leu Lys Glu Ala Asn Lys Lys Leu Lys Leu Glu Asn
   180 185 190
   Gly Gly Leu Val Arg Glu Asn Leu Arg Leu Lys Ala Glu Val Asp Asn
   195 200 205
   Arg Ser Pro Gln Lys Phe Gly Arg Phe Thr Val Ala Ala Leu Gln Ser
   210 215 220
   Lys Val Glu Gln Tyr Glu Arg Glu Thr Asn Arg Leu Lys Lys Ala Leu
   225 230 235 240
   Glu Arg Ser Asp Lys Tyr Ile Glu Glu Leu Glu Ser Gln Ile Thr Gln
   245 250 255
   Leu Lys Asn Ser Ser Asp Glu Lys Glu Ala Arg Asn Ser Ile Cys Gln
   260 265 270
   Arg Ala Leu Ser Thr Asp Gly Lys Gly Asn Thr Gly Ser Glu Glu Asp
   275 280 285
   Met Ala Ser Lys Asn Gln Gly Asp Gly Ala Arg Lys Gln Leu Gly Ser
   290 295 300
   Ser Thr Ser Ser Ser His Leu Ala Lys Pro Ser Ser Ser Ser Ala Arg
   305 310 315 320
   Gln Glu Gly Thr Asn Lys Thr Glu Pro Ser Cys Cys Lys Asn Lys Asp
                   325                 330                 335
   Leu Tyr Gln Lys Arg Ala Glu Ile Met Leu Asp Val Thr Asp Thr Ser
               340                 345                 350
   Met Asp Thr Tyr Leu Glu Arg Glu Trp Gly Gly Lys Pro Ser Asp Cys
           355                 360                 365
   Val Ala Tyr Lys Asp Glu Glu Leu Tyr Asp Leu Pro Ala Pro Cys Thr
       370                 375                 380
   Pro Leu Ser Leu Ser Cys Leu Gln Leu Ser Thr Pro Glu Asn Arg Glu
   385                 390                 395                 400
   Ser Ser Val Val Lys Ala Gly Val Ser Lys Lys His Pro Asn His Leu
                   405                 410                 415
   Arg Lys Leu Val Phe Asp Asp Phe Cys Asp Ser Pro Gly Val Ser Asn
               420                 425                 430
   Lys Asp Ser Ser Glu Asp Asp Arg Ser Glu Ser Glu Lys Lys Ser Glu
           435                 440                 445
   Cys Phe Thr Ser Pro Lys Thr Gly Phe Trp Asp Cys Cys Ser Pro Ser
       450                 455                 460
   Tyr Thr Pro Ser Leu Asp Phe Glu Ser Ser Glu Gly Thr Ile Ala Asn
   465                 470                 475                 480
   Ser Val Gly Glu Phe Leu Lys Leu Ser Glu Lys Ser Gly Ser Cys Leu
                   485                 490                 495
   Ser Lys Arg Leu Asn Ser Ile Arg Thr Phe Glu Met Asn Arg Thr Arg
               500                 505                 510
   Thr Ser Ser Glu Ala Ser Met Asp Ala Ala Tyr Leu Asp Lys Ile Ser
           515                 520                 525
   Glu Leu Asp Ser Met Met Ser Glu Ser Asp Asn Ser Lys Ser Pro Cys
       530                 535                 540
   Asn Asn Gly Phe Lys Ser Leu Asp Leu Asp Gly Leu Ser Lys Ser Ser
   545                 550                 555                 560
   Gln Gly Ser Glu Phe Leu Glu Glu Pro Asp Lys Leu Glu Glu Arg Thr
                   565                 570                 575
   Lys Pro His Leu Ser Lys Gly Ser Leu Thr Thr Asp Gln Leu Glu Asn
   580 585 590
   Gly Asn Glu Trp Lys Ser Asn Ser Phe Phe Leu Leu Ser Pro Ser Asp
   595 600 605
   Gln Glu Met Asn Glu Asp Phe Ser Leu His Pro Ser Ser Asn Pro Gly
   610 615 620
   Thr Asn Glu Ile Lys Pro Pro Ser Cys Leu Phe Gln Thr Glu Phe Ser
   625 630 635 640
   Gln Gly Val Leu Leu Ser Ser Ser His Arg Leu Phe Glu Asp Gln Arg
   645 650 655
   Phe Gly Ser Ser Leu Phe Lys Met Ser Ser Glu Met His Gly Leu Gln
   660 665 670
   Asn His Leu Gln Ser Pro Trp Ser Thr Ser Thr Ser Phe Val Pro Glu
   675 680 685
   Lys Arg Asn Lys Asn Met Asn Gln Ser Thr Lys Arg Lys Ile Gln Ser
   690 695 700
   Ser Leu Ser Asn Ala Ser Pro Ser Lys Ala Thr Lys Ser
   705 710 715
<210> 23
   <211> 722
   <212> PRT
   <213> Mus musculus
<400> 23
   Met Ala Gln Thr Val Gln Asn Val Thr Leu Ser Leu Thr Leu Pro Ile
   1 5 10 15
   Thr Cys His Ile Cys Leu Gly Lys Val Arg Gln Pro Val Val Cys Thr
   20 25 30
   Asn Asn His Val Phe Cys Ser Ile Cys Ile Asp Leu Trp Leu Lys Asn
   35 40 45
   Asn Ser Gln Cys Pro Ala Cys Arg Val Pro Ile Thr Pro Glu Asn Pro
   50 55 60
   Cys Lys Glu Ile Ile Gly Gly Thr Ser Glu Ser Glu Pro Met Leu Ser
   65 70 75 80
   His Thr Val Arg Lys His Leu Arg Lys Thr Arg Leu Glu Leu Leu His
                   85                  90                  95
   Arg Glu Tyr Glu Asp Glu Ile Asp Cys Leu Gln Lys Glu Val Glu Glu
               100                 105                 110
   Leu Lys Ser Lys Asn Leu Ser Leu Glu Ser Gln Ile Lys Thr Ile Leu
           115                 120                 125
   Asp Pro Leu Ala Leu Met Gln Gly Ser Gln Asn Glu Asp Lys His Pro
       130                 135                 140
   Leu Ala Asp Asn Pro Ser Lys Met Asp Pro Asp Ser Val Val Glu Trp
   145                 150                 155                 160
   Lys Lys Lys Leu Arg Thr Ala Asn Glu Ile Tyr Glu Lys Val Lys Asp
                   165                 170                 175
   Asp Val Asp Lys Leu Lys Glu Ala Asn Lys Lys Leu Lys Leu Glu Asn
               180                 185                 190
   Gly Gly Leu Leu Arg Glu Asn Leu Arg Leu Lys Ala Glu Val Asp Asn
           195                 200                 205
   Arg Ser Pro Gln Lys Phe Gly Arg Phe Thr Val Ala Ala Leu Gln Ser
       210                 215                 220
   Lys Val Glu Gln Tyr Glu Arg Glu Thr Asn Arg Leu Lys Lys Ala Leu
   225                 230                 235                 240
   Glu Arg Ser Asp Lys Tyr Ile Glu Glu Leu Glu Ser Gln Val Ala His
                   245                 250                 255
   Leu Lys His Ser Glu Glu Ala Lys Glu Asp Val Asp Ala Leu Cys Gln
               260                 265                 270
   Arg Ala Pro Ser Ala Asp Ser Lys Gly Pro Asn Gly Ser Asp Glu Leu
           275                 280                 285
   Gly Pro Pro Lys Asn Gln Ser Asp Ser Ala Arg Lys Gln Ala Gly Ser
       290                 295                 300
   Ala Ser Ala Ser His Leu Ala Ser Pro Ser Ser Ser Arg Leu Ala Asp
   305                 310                 315                 320
   Ser Gly Ser Val Arg Gln Glu Ser Thr Ser Arg Thr Glu Pro Asn Cys
   325 330 335
   Pro Gln Asn Lys Asp Arg Tyr Pro Lys Pro Thr Glu Pro Arg Leu Gly
   340 345 350
   Ala Arg Glu Thr Pro Met Asp Thr Tyr Leu Glu Arg Glu Trp Gly Ser
   355 360 365
   Lys Pro Ser Asp Cys Ala Pro Tyr Lys Glu Asp Glu Leu Tyr Gly Ile
   370 375 380
   Pro Ala Ser Cys Thr Pro Leu Ser Leu Ser Cys Leu Gln Leu Asn Thr
   385 390 395 400
   Pro Glu Asn Arg Glu Asn Pro Val Ile Lys Ala Gly Ser Ser Lys Lys
   405 410 415
   His Ala Asn His Leu Arg Lys Leu Val Phe Asp Asp Phe Cys Asp Ser
   420 425 430
   Pro Asn Ala Cys Asn Asn Asn Ser Ser Glu Asp Asp Arg Arg Glu Asn
   435 440 445
   Glu Lys Lys Ser Asp Cys Phe Ala Ser Ser Lys Thr Gly Phe Trp Asp
   450 455 460
   Cys Cys Ser Thr Ser Tyr Ala Gln Ser Leu Glu Phe Asp Gly Ser Glu
   465 470 475 480
   Gly Asn Ala Ile Ala Asn Ser Val Gly Glu Ile Pro Ser Lys Leu Ser
   485 490 495
   Glu Lys Ser Gly Ser Cys Leu Ser Lys Arg Leu Ser Cys Ile Arg Ser
   500 505 510
   Leu Glu Met Asn Arg Thr Arg Thr Ser Ser Glu Ala Ser Met Asp Ala
   515 520 525
   Ala Tyr Leu Asp Lys Ile Ser Glu Leu Asp Ser Met Met Ser Glu Ser
   530 535 540
   Asp Asn Ser Lys Ser Pro Cys Asn Asn Gly Phe Lys Ser Val Glu Val
   545 550 555 560
   Glu Gly Pro Ser Lys Ser Pro Gln Gly Arg Glu Phe Leu Glu Glu Pro
   565 570 575
   Asp Lys Leu Gln Glu Gly Ser Lys Leu Asn Leu Ser Lys Pro Ala Leu
               580                 585                 590
   Thr Ala Asp Gly Leu Glu Ser Gly Gly Glu Trp Lys Pro Ser Ser Phe
           595                 600                 605
   Phe Leu Leu Ser Pro Ala Asp His Glu Met Ser Glu Asp Phe Ser Leu
       610                 615                 620
   His Ser Thr Ser His Ser Gly Thr Ser Glu Val Lys Pro Pro Asn Cys
   625                 630                 635                 640
   Leu Phe Gln Thr Glu Phe Ser Gln Gly Ala Leu Leu Ser Ser Ser Gln
                   645                 650                 655
   Gly Leu Phe Glu Asp Gln Arg Phe Gly Ser Ser Leu Phe Lys Met Ser
               660                 665                 670
   Ser Glu Met Gln Ser Leu His Ser Pro Leu Gln Ser Pro Trp Ser Ala
           675                 680                 685
   Ala Phe Val Pro Glu Lys Arg Ser Lys Asn Gly Asn Gln Ser Thr Lys
       690                 695                 700
   Arg Lys Ile Gln Ser Ser Leu Ala Asn Ala Ser Pro Ser Lys Ala Thr
   705                 710                 715                 720
   Lys Ser
<210> 24
   <211> 659
   <212> PRT
   <213> Xenopus laevis
<400> 24
   Met Ala Gln Thr Val Gln Asn Val Thr Leu Ser Leu Thr Leu Pro Ile
   1               5                   10                  15
   Thr Cys His Ile Cys Leu Gly Lys Val Arg Gln Pro Val Ile Cys Val
               20                  25                  30
   Asn His His Val Phe Cys Thr Val Cys Ile Glu Leu Trp Leu Lys Asn
           35                  40                  45
   Asn Asn Gln Cys Pro Val Cys Arg Val Pro Ile Thr Ala Glu Asn Pro
       50                  55                  60
   Cys Lys Asp Ile Ile Gly Gly Thr Gly Glu Asn Glu Cys Ala Leu Asp
   65                  70                  75                  80
   His Ser Val Arg Lys His Leu Arg Lys Thr Arg Leu Glu Leu Leu His
                   85                  90                  95
   Asn Glu Tyr Glu Glu Glu Ile Glu Ser Leu Leu Lys Glu Arg Glu Glu
               100                 105                 110
   Leu Lys Gln Lys Asn Val Thr Leu Glu Lys Glu Arg Ser Pro Thr Thr
           115                 120                 125
   Thr Val Ser Leu Gly His Asp Cys Asn Cys Gly Thr Lys Gln Gly Thr
       130                 135                 140
   Glu Asn Thr Ser Ser Glu Gln Ser Thr Ser Glu Lys Trp Lys Lys Lys
   145                 150                 155                 160
   Leu Glu Glu Thr Asn Ala Ala Ser Arg Lys Leu Val Ala Asp Val Glu
                   165                 170                 175
   Lys Leu Lys Glu Glu Asn Thr Lys Leu Ile Asn Glu Asn Ile Asp Tyr
               180                 185                 190
   Val Arg Glu Asn Phe Arg Leu Lys Thr Glu Val Asp Ser Arg Ser Pro
           195                 200                 205
   Gln Lys Phe Gly Arg Phe Thr Val Ala Ala Leu His Ala Lys Ile Asp
       210                 215                 220
   Gln Tyr Glu Lys Glu Met Asn Arg Leu Lys Lys Ala Leu Glu Arg Ser
   225                 230                 235                 240
   Asp Lys Tyr Ile Glu Glu Leu Glu Val Gln Val Asp Gln Leu Lys Arg
                   245                 250                 255
   Pro Ala Asp Trp Lys Pro Asn Glu Arg Ser His Asn Glu Asn Pro Val
               260                 265                 270
   Arg Asp Lys Glu Thr Val Asn Ser Glu Ile Gly Asp Ser Ser Leu Asn
           275                 280                 285
   Tyr Glu Gly Val Lys Leu Glu Gln Met Asp Lys Ile Pro Glu Lys Ala
       290                 295                 300
   Glu Lys Asn Gln Glu Asp Ser Asn Ser Thr Cys Pro Thr Ala Asn Asn
   305                 310                 315                 320
   His Lys Phe Ile Val Asn Lys Ser Glu Tyr Leu Ser Arg Thr Gly Cys
   325 330 335
   Ser Met Asp Phe Gln Lys Asp Arg Lys Ser Phe Pro Ser Ser Ser Ser
   340 345 350
   Ala Thr Trp Lys Glu Ser Leu Lys Ser Pro His Gly Lys Asn Ser Ser
   355 360 365
   Arg Asn Lys Pro Arg Glu Ser Asn Thr Pro Cys Lys Glu Lys Glu Ile
   370 375 380
   His Glu Phe Ser Ser Pro Gly Thr Ser Leu Ser Phe Ser Ser Leu Gln
   385 390 395 400
   Leu Asn Ser Pro Asp Cys Lys Phe Thr Leu Pro Ser Asn Gln Val Asn
   405 410 415
   Ala Lys Lys Pro Leu Thr His Leu Arg Lys Leu Val Phe Asp Asp Ser
   420 425 430
   Pro Lys Lys Arg Lys Pro Asn Gly Phe Ser Asn Ala Asn Asn Asn Gly
   435 440 445
   Gly Leu Ser Lys Pro Phe Asn Glu Pro Lys Ser Glu Tyr Trp Ala Cys
   450 455 460
   Glu Val Lys His Lys His Ser Arg Asp Lys Leu Lys Glu Ser Tyr Val
   465 470 475 480
   Gln Leu Glu Ser Leu Lys Asn Lys Glu Gly Gln Ile Leu Gln Ala Leu
   485 490 495
   Pro Ser Leu Ser Ser Glu Glu Gly Asn Arg Thr Thr Thr Thr Ser Glu
   500 505 510
   Ser Ser Met Asp Ile Thr Phe Leu Asp Val Asn Lys Leu Asp Ser Met
   515 520 525
   Met Ser Thr Leu Glu Gly Asn Glu Asn Pro His Tyr His Ser Val Lys
   530 535 540
   Ser Asp Ala Val Met His Ser Asn Ser Gln Pro Arg Asn Gly Leu Gln
   545 550 555 560
   Ala Leu Tyr Ser Asn Pro His Ser Gln Cys Ala Gly Gly Leu Leu Ser
   565 570 575
   His Glu Ser Arg Ser Ser Asp Lys Ser His Asn Asn Asp Phe His Thr
               580                 585                 590
   Ser Arg Ser Thr Ser Ser Leu Leu Thr Pro Phe Tyr Ala Thr Ala Gln
           595                 600                 605
   Ser Tyr Ser Ser Lys Asn Leu Met Asp Leu Met Ala Pro Glu Met Thr
       610                 615                 620
   Ser Asn Ser Gly Ser Thr Ser Tyr Thr Asp Asn Lys Asn Gly Leu Thr
   625                 630                 635                 640
   Ser Lys Arg Lys Leu Phe Asn Pro Ile Cys Asp Ser Pro Pro Lys Ser
                   645                 650                 655
   Phe Lys Pro
<210> 25
   <211> 717
   <212> PRT
   <213> Felis catus
<400> 25
   Met Ala Gln Thr Val Gln Asn Val Thr Leu Ser Leu Thr Leu Pro Ile
   1               5                   10                  15
   Thr Cys His Ile Cys Leu Gly Lys Val Arg Gln Pro Val Ile Cys Ile
               20                  25                  30
   Asn Asn His Val Phe Cys Ser Ile Cys Ile Asp Leu Trp Leu Lys Asn
           35                  40                  45
   Asn Ser Gln Cys Pro Ala Cys Arg Val Pro Ile Thr Pro Glu Asn Pro
       50                  55                  60
   Cys Lys Glu Ile Ile Gly Gly Thr Ser Glu Ser Glu Pro Met Leu Ser
   65                  70                  75                  80
   His Thr Val Arg Lys His Leu Arg Lys Thr Arg Leu Glu Leu Leu His
                   85                  90                  95
   Lys Glu Tyr Glu Asp Glu Ile Asp Cys Leu Gln Lys Glu Val Glu Asp
               100                 105                 110
   Leu Lys Ser Lys Asn Leu Ser Leu Glu Ser Gln Ile Lys Thr Ile Leu
           115                 120                 125
   Asp Pro Leu Thr Leu Met Gln Ser Ser Gln Thr Glu Asp Lys His Pro
   130 135 140
   Ile Ala Asp Asn Pro Ser Lys Ile Asp Pro Glu Thr Val Ala Glu Trp
   145 150 155 160
   Lys Lys Lys Leu Arg Thr Ala Asn Glu Ile Tyr Glu Lys Val Lys Asp
   165 170 175
   Asp Val Asp Lys Leu Lys Glu Ala Asn Lys Lys Leu Lys Leu Glu Asn
   180 185 190
   Gly Gly Leu Val Arg Glu Asn Leu Arg Leu Lys Ala Glu Val Asp Asn
   195 200 205
   Arg Ser Pro Gln Lys Phe Gly Arg Phe Thr Val Ala Ala Leu Gln Ser
   210 215 220
   Lys Val Glu Gln His Glu Arg Glu Thr Asn Arg Leu Lys Lys Ala Leu
   225 230 235 240
   Glu Arg Ser Asp Lys Tyr Ile Glu Glu Leu Glu Ser Gln Ile Ala Gln
   245 250 255
   Leu Lys Asn Ser Ser Glu Glu Lys Glu Ala Ile Asn Ser Ile Cys Gln
   260 265 270
   Arg Ala Leu Ser Thr Asp Gly Lys Gly Ser Leu Gly Gly Glu Glu Asp
   275 280 285
   Met Ala Ser Lys Asn Gln Gly Asp Gly Ala Arg Lys Gln Leu Gly Ser
   290 295 300
   Ser Thr Ser Ser Ser His Leu Pro Lys Pro Ser Ser Ser Ser Ala Arg
   305 310 315 320
   Gln Glu Ser Thr Ser Lys Thr Glu Pro Asn Cys Ser Lys Asn Lys Asp
   325 330 335
   Leu Tyr Gln Lys Arg Val Glu Ile Met Leu Asp Val Thr Asp Thr Ser
   340 345 350
   Met Asp Thr Tyr Leu Glu Arg Glu Trp Gly Asn Lys Pro Ser Asp Cys
   355 360 365
   Ile Pro Tyr Lys Asp Glu Glu Leu Tyr Asp Leu Pro Ala Pro Cys Thr
           370                 375                 380
   Pro Leu Ser Leu Ser Cys Leu Gln Leu Ser Thr Pro Glu Asn Arg Glu
   385                 390                 395                 400
   Ser Ser Val Val Lys Ala Val Gly Ser Lys Lys His Ser Asn His Leu
                   405                 410                 415
   Arg Lys Leu Val Phe Asp Asp Phe Cys Asp Ser Ser Asn Val Cys Thr
               420                 425                 430
   Lys Asp Ser Ser Glu Asp Asp Arg Ser Glu Asn Glu Lys Lys Ser Glu
           435                 440                 445
   Cys Phe Thr Ser Ser Lys Thr Gly Phe Trp Asp Cys Cys Ser Thr Ser
       450                 455                 460
   Tyr Ala Gln Ser Leu Asp Phe Glu Ser Ser Glu Gly Asn Thr Ile Ala
   465                 470                 475                 480
   Asn Ser Val Gly Glu Ile Ser Leu Lys Leu Ser Glu Lys Ser Gly Ser
                   485                 490                 495
   Cys Ile Ser Lys Arg Leu Asn Ser Ile Arg Cys Phe Glu Met Asn Arg
               500                 505                 510
   Thr Arg Thr Ser Ser Glu Ala Ser Met Asp Ala Ala Tyr Leu Asp Lys
           515                 520                 525
   Ile Ser Glu Leu Asp Ser Met Met Ser Glu Ser Asp Asn Ser Lys Ser
       530                 535                 540
   Pro Cys Asn Asn Gly Phe Lys Ser Val Asp Leu Asp Gly Leu Ser Lys
   545                 550                 555                 560
   Ser Ser Gln Gly Ser Glu Phe Leu Glu Glu Pro Asp Lys Leu Asp Glu
                   565                 570                 575
   Arg Thr Lys Pro Asn Leu Ser Lys Gly Ser Leu Thr Thr Asp Gln Leu
               580                 585                 590
   Glu Asn Gly Asn Glu Trp Lys Pro Thr Ser Phe Phe Leu Leu Ser Pro
           595                 600                 605
   Ser Asp Gln Glu Met Asn Glu Asp Phe Ser Leu His Thr Ser Ser Asn
       610                 615                 620
   Pro Gly Thr Asn Glu Ile Lys Pro Pro Ser Cys Leu Phe Gln Thr Glu
   625 630 635 640
   Phe Ser Gln Gly Val Leu Leu Ser Ser Ser His Arg Leu Phe Glu Asp
   645 650 655
   Gln Arg Phe Gly Ser Ser Leu Phe Lys Met Thr Ser Glu Met His Gly
   660 665 670
   Leu His Asn His Leu Gln Ser Pro Trp Ser Thr Ser Phe Val Pro Glu
   675 680 685
   Lys Arg Asn Lys Asn Ile Thr Gln Ser Thr Lys Arg Lys Ile Gln Ser
   690 695 700
   Ser Leu Ser Asn Ala Ser Pro Ser Lys Ala Thr Lys Ser
   705 710 715
<210> 26
   <211> 724
   <212> PRT
   <213> Rattus norvegicus
<400> 26
   Met Ala Gln Thr Val Gln Asn Val Thr Leu Ser Leu Thr Leu Pro Ile
   1 5 10 15
   Thr Cys His Ile Cys Leu Gly Lys Val Arg Gln Pro Val Val Cys Thr
   20 25 30
   Asn Asn His Val Phe Cys Ser Ile Cys Ile Asp Leu Trp Leu Lys Asn
   35 40 45
   Asn Ser Gln Cys Pro Ala Cys Arg Val Pro Ile Thr Thr Glu Asn Pro
   50 55 60
   Cys Lys Glu Ile Ile Gly Gly Thr Ser Glu Ser Glu Pro Met Leu Ser
   65 70 75 80
   His Thr Val Arg Arg His Leu Arg Arg Thr Arg Leu Glu Leu Leu His
   85 90 95
   Arg Glu Tyr Glu Asp Glu Ile Asp Cys Leu Gln Lys Glu Val Glu Glu
   100 105 110
   Leu Lys Ser Lys Asn Leu Ser Leu Glu Ser Gln Ile Lys Thr Ile Leu
   115 120 125
   Asp Pro Leu Ala Leu Met Gln Gly Ser Gln Asn Glu Asp Lys His Pro
       130                 135                 140
   Met Ala Asp Asn Pro Ser Lys Ile Asp Pro Glu Ser Val Val Glu Trp
   145                 150                 155                 160
   Lys Lys Lys Leu Arg Thr Ala Asn Glu Ile Tyr Glu Lys Val Lys Asp
                   165                 170                 175
   Asp Val Asp Lys Leu Lys Glu Ala Asn Lys Lys Leu Lys Leu Glu Asn
               180                 185                 190
   Gly Gly Leu Leu Arg Glu Asn Leu Arg Leu Lys Ala Glu Val Asp Asn
           195                 200                 205
   Arg Ser Pro Gln Lys Phe Gly Arg Phe Thr Val Ala Ala Leu Gln Ser
       210                 215                 220
   Lys Val Glu Gln Tyr Glu Arg Glu Thr Asn Arg Leu Lys Lys Ala Leu
   225                 230                 235                 240
   Glu Arg Ser Asp Lys Tyr Ile Glu Glu Leu Glu Ser Gln Val Ala Gln
                   245                 250                 255
   Leu Arg His Ser Glu Glu Thr Glu Glu Gly Met Gly Ala Leu Cys Gln
               260                 265                 270
   Arg Ala Pro Ser Ala Asp Gly Lys Gly Pro Lys Gly Ser Asp Asp Val
           275                 280                 285
   Ala Ala Ser Lys Asn Pro Ser Glu Gly Ala Gly Lys Gln Ala Gly Ser
       290                 295                 300
   Ala Asn Ser Ala Ser His Leu Ala Thr Pro Ser Ser Ser Arg Leu Ala
   305                 310                 315                 320
   Asp Thr Ser Ser Ala Arg Gln Glu Ser Thr Ser Arg Thr Glu Pro Asn
                   325                 330                 335
   Cys Leu Gln Asn Lys Asp Arg Tyr Pro Lys Pro Thr Glu Ala Gln Leu
               340                 345                 350
   Gly Ala Arg Glu Thr Pro Met Asp Thr Tyr Leu Glu Arg Gln Trp Gly
           355                 360                 365
   Ser Lys Pro Ser Asp Cys Ala Pro Tyr Lys Glu Glu Glu Leu Tyr Gly
       370                 375                 380
   Leu Gln Ala Ser Cys Thr Pro Leu Ser Leu Ser Cys Leu Gln Leu Asn
   385                 390                 395                 400
   Thr Pro Glu Asn Arg Glu Asn Pro Val Ile Lys Ala Gly Gly Ser Lys
                   405                 410                 415
   Lys His Ala Asn His Leu Arg Lys Leu Val Phe Asp Asp Phe Cys Asp
               420                 425                 430
   Ser Pro Asn Thr Cys Asn Asn Asn Ser Ser Glu Asp Asp Arg Ser Gly
           435                 440                 445
   Asn Glu Lys Lys Ser Asp Cys Phe Ala Ser Ser Lys Thr Glu Phe Trp
       450                 455                 460
   Asp Cys Cys Ser Thr Ser Tyr Ala Gln Ser Leu Glu Phe Asp Gly Ser
   465                 470                 475                 480
   Glu Gly Asn Thr Ile Ala Asn Pro Val Gly Glu Val Pro Ser Lys Leu
                   485                 490                 495
   Ser Glu Lys Ser Gly Phe Cys Leu Ser Lys Arg Leu Ser Cys Ile Arg
               500                 505                 510
   Ser Leu Glu Met Asn Arg Thr Arg Thr Ser Ser Glu Ala Ser Met Asp
           515                 520                 525
   Ala Ala Tyr Leu Asp Lys Ile Ser Glu Leu Asp Ser Met Met Ser Glu
       530                 535                 540
   Ser Asp Asn Ser Lys Ser Pro Cys Asn Asn Gly Phe Lys Ser Val Glu
   545                 550                 555                 560
   Ser Glu Gly Ser Ser Lys Ser Pro Gln Gly Arg Glu Phe Leu Glu Glu
                   565                 570                 575
   Pro Asp Lys Leu Gln Glu Gly Ala Lys Leu Ser Leu Pro Lys Asn Ala
               580                 585                 590
   Leu Glu Ala Asp Gly Leu Glu Gly Gly Ser Glu Trp Lys Pro Ser Ser
           595                 600                 605
   Phe Phe Leu Leu Ser Pro Ser Asp His Glu Met Ser Glu Asp Phe Ser
       610                 615                 620
   Leu His Thr Thr Ser His Ser Gly Ala Ser Glu Val Lys Ser Gln Asn
   625                 630                 635                 640
   Cys Leu Phe Gln Thr Glu Phe Ser Gln Gly Val Gln Leu Leu Ser Ser
   645 650 655
   Ser Gln Gly Leu Phe Glu Asp Gln Arg Phe Gly Ser Ser Leu Phe Lys
   660 665 670
   Met Ser Ser Asp Met Gln Ser Leu Pro Gly Pro Leu Gln Ser Pro Trp
   675 680 685
   Ser Ala Ala Phe Val Ser Glu Lys Arg Ser Lys Asn Gly Asn Gln Ser
   690 695 700
   Ala Lys Arg Lys Ile Gln Ser Ser Leu Ser Asn Ala Ser Pro Ser Lys
   705 710 715 720
   Ala Thr Lys Ser
<210> 27
   <211> 654
   <212> PRT
   <213> Xenopus tropicalis
<400> 27
   Met Ala Gln Asn Val Gln Asn Val Thr Leu Ser Leu Thr Leu Pro Ile
   1 5 10 15
   Thr Cys His Ile Cys Leu Gly Lys Val Arg Gln Pro Val Ile Cys Val
   20 25 30
   Asn Tyr His Val Phe Cys Thr Val Cys Ile Glu Leu Trp Leu Lys Asn
   35 40 45
   Asn Ser Gln Cys Pro Val Cys Arg Val Pro Ile Thr Ala Glu Asn Pro
   50 55 60
   Cys Lys Asp Ile Ile Gly Gly Thr Gly Glu Asn Glu Cys Ser Leu Asp
   65 70 75 80
   His Ser Val Arg Lys His Leu Arg Lys Thr Arg Leu Glu Leu Leu His
   85 90 95
   Asn Glu Tyr Glu Glu Glu Ile Glu Ser Leu Leu Lys Glu Leu Glu Glu
   100 105 110
   Leu Lys Gln Lys Asn Ile Ile Leu Glu Arg Glu Arg Ser Pro Thr Thr
   115 120 125
   Thr Val Ser Leu Gly Ile Asp Cys Asn Cys Gly Thr Lys Leu Arg Thr
       130                 135                 140
   Glu Asn Thr Ser Ser Glu Gln Thr Thr Ser Glu Lys Trp Lys Lys Lys
   145                 150                 155                 160
   Leu Glu Glu Thr Asn Ala Ala Ser Arg Lys Leu Ser Ala Asp Val Glu
                   165                 170                 175
   Lys Leu Lys Glu Glu Asn Ala Lys Leu Ile Asn Glu Asn Ile Asp Tyr
               180                 185                 190
   Val Arg Glu Asn Phe Arg Leu Lys Thr Glu Val Asp Ser Arg Ser Pro
           195                 200                 205
   Gln Lys Phe Gly Arg Phe Thr Val Ala Ala Leu His Ala Lys Ile Asp
       210                 215                 220
   Gln Tyr Glu Lys Glu Met Asn Arg Leu Lys Lys Ala Leu Glu Arg Ser
   225                 230                 235                 240
   Asp Lys Tyr Ile Glu Glu Leu Glu Val Gln Val Asp Gln Leu Lys Arg
                   245                 250                 255
   Ala Ala Asp Leu Lys Gln Asp Glu Ser Ser Gln Asn Glu Asn Ser Val
               260                 265                 270
   His Asp Lys Asp Thr Val Asn Ser Glu Ile Gly Cys Thr Glu Leu Tyr
           275                 280                 285
   Glu Gly Val Lys Leu Glu Gln Met Gly Glu Lys Pro Glu Lys His Asp
       290                 295                 300
   Ile Asn Lys Glu Asn Ser Ser Asp Thr Cys Pro Ala Ala Asn Asn Glu
   305                 310                 315                 320
   Lys Ser His Val His Arg Ser Glu Tyr Leu Thr Gln Thr Gly Cys Ser
                   325                 330                 335
   Leu Asn Phe Gln Lys Glu Arg Lys Met Phe Leu Ser Ser Gly Ser Ala
               340                 345                 350
   Met Trp Lys Glu Ser Met Lys Gly Pro His Asp Asn Ile Thr Ser Gln
           355                 360                 365
   Asn Lys Gln Thr Glu Thr Tyr Thr Pro Thr Lys Glu Lys Glu Ile His
   370 375 380
   Glu Tyr Asn Ser Pro Ser Thr Ser Leu Ser Phe Ser Ser Leu Gln Leu
   385 390 395 400
   Asn Ser Pro Asp Cys Lys Leu Ser Leu Pro Ser Asn Gln Val Asn Ala
   405 410 415
   Lys Lys Pro Leu Thr Tyr Leu Arg Lys Leu Val Phe Glu Asp Tyr Pro
   420 425 430
   Lys Lys Gly Lys Pro Asn Ser Phe Ser Ser Ala Asn Asn Asn Ile Pro
   435 440 445
   Lys Pro Phe Asn Glu Gln Lys Ser Glu Tyr Met Thr Cys Glu Val Lys
   450 455 460
   His Lys His Gln Arg Gly Asn Leu Lys Glu Ser Asp Met Gln Ser Glu
   465 470 475 480
   Gly Gln Ile Leu Gln Ala Phe Ser Leu Pro Ser Ser Gly Glu Val His
   485 490 495
   His Thr Thr Thr Thr Ser Glu Ser Pro Met Asp Thr Phe Leu His Asn
   500 505 510
   Val Asn Asn Leu Asp Ser Leu Met Ser Thr Leu Glu Gly Asn Lys Ser
   515 520 525
   Leu His Tyr His Ser Val Thr Ala Asp Ala Val Met His Leu Asn Ser
   530 535 540
   Gln Pro Gly Asn Gly Leu Gln Ala Phe Phe Ser Asn Pro His Thr Arg
   545 550 555 560
   Asp Gly His Phe Pro Leu Glu Ser Gln Ser Ser Asp Lys Ser His Ile
   565 570 575
   Asn Asp Phe His Thr Ser Gly Arg Thr Ser Ser Leu Val Leu Pro Phe
   580 585 590
   Asn Asp Met Pro Gln Ile Lys Tyr Gln Asn Thr Thr Glu Ser Ser Asn
   595 600 605
   Ile Leu Thr Asn Leu Asn Ala Ser Glu Leu Ala Ser Asn Ser Cys Cys
   610 615 620
   Thr Ser Tyr Thr Asn Asn Lys His Gly Pro Ala Ser Lys Arg Lys Leu
   625 630 635 640
   Leu Asn Thr Ile Cys Glu Ser Pro Pro Lys Thr Met Lys Pro
   645 650
<210> 28
   <211> 39
   <212> PRT
   <213> Artificial Sequence
<220>
   <223> ring finger - zinc finger of OBI1 proteins
<220>
   <221> misc-feature
   <222> (13)..(13)
   <223> Xaa can be any naturally occurring amino acid
<220>
   <221> misc-feature
   <222> (15)..(15)
   <223> Xaa can be any naturally occurring amino acid
<220>
   <221> misc-feature
   <222> (23)..(23)
   <223> Xaa can be any naturally occurring amino acid
<400> 28
   Cys His Ile Cys Leu Gly Lys Val Arg Gln Pro Val Xaa Cys Xaa Asn
   1 5 10 15
   Asn His Val Phe Cys Ser Xaa Cys Ile Asp Leu Trp Leu Lys Asn Asn
   20 25 30
   Ser Gln Cys Pro Ala Cys Arg
   35
<210> 29
   <211> 194
   <212> PRT
   <213> Artificial Sequence
<220>
   <223> Coiled-Coil domain of huaman Obi1
<400> 29
   Val Arg Lys His Leu Arg Lys Thr Arg Leu Glu Leu Leu His Lys Glu
   1               5                   10                  15
   Tyr Glu Asp Glu Ile Asp Cys Leu Gln Lys Glu Val Glu Glu Leu Lys
               20                  25                  30
   Ser Lys Asn Leu Ser Leu Glu Ser Gln Ile Lys Thr Ile Leu Asp Pro
           35                  40                  45
   Leu Thr Leu Val Gln Gly Asn Gln Asn Glu Asp Lys His Leu Val Thr
       50                  55                  60
   Asp Asn Pro Ser Lys Ile Asn Pro Glu Thr Val Ala Glu Trp Lys Lys
   65                  70                  75                  80
   Lys Leu Arg Thr Ala Asn Glu Ile Tyr Glu Lys Val Lys Asp Asp Val
                   85                  90                  95
   Asp Lys Leu Lys Glu Ala Asn Lys Lys Leu Lys Leu Glu Asn Gly Gly
               100                 105                 110
   Leu Val Arg Glu Asn Leu Arg Leu Lys Ala Glu Val Asp Asn Arg Ser
           115                 120                 125
   Pro Gln Lys Phe Gly Arg Phe Ala Val Ala Ala Leu Gln Ser Lys Val
       130                 135                 140
   Glu Gln Tyr Glu Arg Glu Thr Asn Arg Leu Lys Lys Ala Leu Glu Arg
   145                 150                 155                 160
   Ser Asp Lys Tyr Ile Glu Glu Leu Glu Ser Gln Val Ala Gln Leu Lys
                   165                 170                 175
   Asn Ser Ser Glu Glu Lys Glu Ala Met Asn Ser Ile Cys Gln Thr Ala
               180                 185                 190
   Leu Ser
<210> 30
   <211> 450
   <212> PRT
   <213> Artificial Sequence
<220>
   <223> C-terminal part of human Obi1
<400> 30
   Ala Asp Gly Lys Gly Ser Lys Gly Ser Glu Glu Asp Val Val Ser Lys
   1               5                   10                  15
   Asn Gln Gly Asp Ser Ala Arg Lys Gln Pro Gly Ser Ser Thr Ser Ser
               20                  25                  30
   Ser Ser His Leu Ala Lys Pro Ser Ser Ser Arg Leu Cys Asp Thr Ser
           35                  40                  45
   Ser Ala Arg Gln Glu Ser Thr Ser Lys Ala Asp Leu Asn Cys Ser Lys
       50                  55                  60
   Asn Lys Asp Leu Tyr Gln Glu Gln Val Glu Val Met Leu Asp Val Thr
   65                  70                  75                  80
   Asp Thr Ser Met Asp Thr Tyr Leu Glu Arg Glu Trp Gly Asn Lys Pro
                   85                  90                  95
   Ser Asp Cys Val Pro Tyr Lys Asp Glu Glu Leu Tyr Asp Leu Pro Ala
               100                 105                 110
   Pro Cys Thr Pro Leu Ser Leu Ser Cys Leu Gln Leu Ser Thr Pro Glu
           115                 120                 125
   Asn Arg Glu Ser Ser Val Val Gln Ala Gly Gly Ser Lys Lys His Ser
       130                 135                 140
   Asn His Leu Arg Lys Leu Val Phe Asp Asp Phe Cys Asp Ser Ser Asn
   145                 150                 155                 160
   Val Ser Asn Lys Asp Ser Ser Glu Asp Asp Ile Ser Arg Ser Glu Asn
                   165                 170                 175
   Glu Lys Lys Ser Glu Cys Phe Ser Ser Pro Lys Thr Gly Phe Trp Asp
               180                 185                 190
   Cys Cys Ser Thr Ser Tyr Ala Gln Asn Leu Asp Phe Glu Ser Ser Glu
           195                 200                 205
   Gly Asn Thr Ile Ala Asn Ser Val Gly Glu Ile Ser Ser Lys Leu Ser
       210                 215                 220
   Glu Lys Ser Gly Leu Cys Leu Ser Lys Arg Leu Asn Ser Ile Arg Ser
   225                 230                 235                 240
   Phe Glu Met Asn Arg Thr Arg Thr Ser Ser Glu Ala Ser Met Asp Ala
                   245                 250                 255
   Ala Tyr Leu Asp Lys Ile Ser Glu Leu Asp Ser Met Met Ser Glu Ser
               260                 265                 270
   Asp Asn Ser Lys Ser Pro Cys Asn Asn Gly Phe Lys Ser Leu Asp Leu
           275                 280                 285
   Asp Gly Leu Ser Lys Ser Ser Gln Gly Ser Glu Phe Leu Glu Glu Pro
       290                 295                 300
   Asp Lys Leu Glu Glu Lys Thr Glu Leu Asn Leu Ser Lys Gly Ser Leu
   305                 310                 315                 320
   Thr Asn Asp Gln Leu Glu Asn Gly Ser Glu Trp Lys Pro Thr Ser Phe
                   325                 330                 335
   Phe Leu Leu Ser Pro Ser Asp Gln Glu Met Asn Glu Asp Phe Ser Leu
               340                 345                 350
   His Ser Ser Ser Cys Pro Val Thr Asn Glu Ile Lys Pro Pro Ser Cys
           355                 360                 365
   Leu Phe Gln Thr Glu Phe Ser Gln Gly Ile Leu Leu Ser Ser Ser His
       370                 375                 380
   Arg Leu Phe Glu Asp Gln Arg Phe Gly Ser Ser Leu Phe Lys Met Ser
   385                 390                 395                 400
   Ser Glu Met His Ser Leu His Asn His Leu Gln Ser Pro Trp Ser Thr
                   405                 410                 415
   Ser Phe Val Pro Glu Lys Arg Asn Lys Asn Val Asn Gln Ser Thr Lys
               420                 425                 430
   Arg Lys Ile Gln Ser Ser Leu Ser Ser Ala Ser Pro Ser Lys Ala Thr
           435                 440                 445
   Lys Ser
       450
<210> 31
   <211> 3552
   <212> DNA
   <213> Homo sapiens
<400> 31
   ggcgaagagt agcggtaggt cggcgggact tccgtgttgg cgggattctg aacgctgcca      60
   tggctcagac cgtgcagaat gttacattgt cgctcactct gcccatcacg tgccacattt     120
   gcttggggaa ggtacgtcag cctgtcatat gcatcaacaa ccatgtattt tgttcgattt     180
   gtattgattt gtggttgaag aataatagcc agtgtccagc ttgcagagtc cccatcactc     240
   ctgaaaatcc ttgcaaagaa attataggag gaacaagtga aagtgaacct atgctaagcc     300
   atacggtcag gaagcatctt cggaaaacta gacttgaatt actacacaaa gaatatgagg     360
   acgaaataga ttgtttacag aaagaagtag aagagcttaa gagtaaaaat ctcagcttgg 420
   agtcacagat caaaactatt ctggatcctt taaccttggt gcagggcaac caaaatgaag 480
   acaaacatct agtcacagat aatccaagta aaattaaccc agaaactgta gcagagtgga 540
   agaaaaaact cagaacagct aatgaaatct atgaaaaagt gaaagatgat gtggataagc 600
   taaaggaggc aaataaaaaa ttgaaattgg aaaatggtgg tctggtgagg gagaatttac 660
   gactgaaggc tgaagttgat aacagatcac ctcaaaagtt tggaaggttt gcagttgctg 720
   ctcttcagtc caaagtagaa cagtatgagc gtgaaaccaa tcgcctcaag aaagccctgg 780
   aacgaagtga taagtatata gaggaactag aatctcaagt tgcacagcta aaaaattcaa 840
   gtgaagagaa agaagctatg aattccattt gccagacagc actttctgca gatggcaaag 900
   ggagcaaagg cagtgaggag gatgtggtgt caaagaatca aggcgatagt gccagaaagc 960
   agcctggctc atccacctcc agttcttctc acctagcgaa gccttccagc agcagactgt 1020
   gtgacaccag ttctgcaagg caggaaagta ccagcaaagc agaccttaac tgttctaaga 1080
   acaaagacct atatcaagaa caggtagaag taatgttaga tgtgacagat acaagtatgg 1140
   atacttattt ggaaagagaa tgggggaata aaccaagtga ctgtgtaccc tacaaagatg 1200
   aagaacttta tgatcttcca gctccttgta ctcctttgtc ccttagttgc cttcagctca 1260
   gtactccaga aaatagagag agctctgtgg tccaagcagg aggttccaaa aagcactcaa 1320
   accatctcag aaaattggtg tttgatgatt tttgtgattc ttcaaatgtt tctaataaag 1380
   attcttcaga agatgatata agtagaagtg aaaatgaaaa gaaatcagaa tgtttttctt 1440
   ccccaaagac aggattttgg gactgttgtt ccacaagcta tgcccaaaac ttagattttg 1500
   aaagttcaga ggggaacacg atagcaaatt ctgttggaga aatatcttca aaattgagtg 1560
   agaaatcagg cttatgttta tccaaaaggt tgaattctat tcgctctttt gaaatgaacc 1620
   ggacaagaac atccagtgaa gcatcgatgg atgctgctta ccttgacaaa atctctgagt 1680
   tggattcaat gatgtcagag tcagacaaca gcaagagccc ttgtaataac ggttttaagt 1740
   cactggattt ggatgggtta tcaaagtcat ctcaaggcag tgaatttctt gaggaacctg 1800
   ataagttgga agaaaaaact gagctaaacc tttccaaagg ttctctaact aatgatcagt 1860
   tagaaaatgg aagtgaatgg aaacccactt ctttttttct cctctctcca tctgaccaag 1920
   aaatgaatga agatttttca ctccattcca gttcttgtcc agtaactaat gaaatcaaac 1980
   ccccaagctg cttgtttcag acagagtttt cccagggcat tttgttaagc agttcacatc 2040
   gactatttga agatcaaaga tttgggtcat ctttgtttaa gatgtcctca gagatgcaca 2100
   gtcttcataa ccaccttcag tctccttggt ctacttcctt tgtgcctgaa aagaggaata 2160
   aaaatgtgaa tcaatcaaca aaaagaaaaa tccagagcag cctttccagt gccagcccat 2220
   caaaagcaac taaaagttga ctcattagaa aggtgtcatt tgtggttttg tcctgagaga 2280
   aatagaaaag ttgttaaagt tacctttttt cctcataaaa gttctataca aattggaatt     2340
   gataatcttt agtcaagtat caagtcagga tggtggatta acctgtaccc agaatactta     2400
   ttgttcattt tgaaaagact ttgttctttt catttttatt tgggagtctt tgtgaccaga     2460
   gaagttaggg aggaggttat ttttgtgttt tggggttggt tggttggttg gttttgtttt     2520
   tggttttgtt tttttactga atttgatatg tatctcggtt ggatatacat tgttttttta     2580
   aaaaatgtta tttaactgtt agatacagtg gcctgttgat aagccccact tgtcttcaga     2640
   acttggattt cttaaataaa acttttagtg ttgtctatac actgctcaat aagacacttg     2700
   agtttaagct tttcccaggg tggaaattat tttacctgtc cctttttatt tatgtttagt    2760
   gatggcctag tttttctgca gggccatgat ggagaaatag cactctagcc ttagtccaat     2820
   attgatttac tttctttttt taggttttat gtatatgttt gcatttttta gcattgtgtt     2880
   ttgtccagtt ttgtgaaaat gttctgctag tatgaaagaa aacattttct atatgaagac     2940
   atttgtttta tgttaggtag cttacatttt ctcctctgcg tgtgtgtgta tgtgtgtaaa     3000
   atcagaaatt tagcatacta tggaaagaag gcatggagca cttgggttta gaggaaccta     3060
   aaacatcata gcttcattgt tccagatgta acaggtttga aagagctcat cgccaagttc     3120
   ttgatccact tgcattccag gggagttttc ttttgagtag tatgtttctt gtttgcatgt     3180
   tcctgttctt tgtggaaact atgcatggta gcatttttgc ttgctgtgtt ttccatactt     3240
   aagaaaaaga ggtttcagtt ggctgataga atatctttta tgtaggacaa aacttttctg     3300
   tgaagagtgt tgagggggtg aagataggta agaggtaagc acaattttta atttaggctc      3360
   tgaaaaagtg tattgttcta aacgtatttg gtatgcctat ataggtcttt aaaaatgggt     3420
   atgtatgctg tttaatgtgc actgaacatt ttacattaat attgtactgt tttacattaa     3480
   tactgcatgc ttttctatgt gaattgaata aagaatgtca taagcactgt gttcaaaaaa     3540
   aaaaaaaaaa aa                                                         3552
<210> 32
   <211> 19
   <212> DNA
   <213> Artificial sequence
<220>
   <223> siRNA inhibiting OBI1
<400> 32
   gtagaagtaa tgttagatg 19
<210> 33
   <211> 19
   <212> DNA
   <213> Artificial sequence
<220>
   <223> siRNA inhibiting OBI1
<400> 33
   ccaaaggttc tctaactaa 19
<210> 34
   <211> 19
   <212> DNA
   <213> Artificial sequence
<220>
   <223> siRNA inhibiting OBI1
<400> 34
   ggatttggat gggttatca 19
<210> 35
   <211> 19
   <212> DNA
   <213> Artificial sequence
<220>
   <223> siRNA inhibiting OBI1
<400> 35
   gaacgaagtg ataagtata 19
<210> 36
   <211> 19
   <212> RNA
   <213> Artificial sequence
<220>
   <223> siRNA inhibiting OBI1
<400> 36
   guagaaguaa uguuagaug 19
<210> 37
   <211> 19
   <212> RNA
   <213> Artificial sequence
<220>
   <223> siRNA inhibiting OBI1
<400> 37
   ccaaagguuc ucuaacuaa 19
<210> 38
   <211> 19
   <212> RNA
   <213> Artificial sequence
<220>
   <223> siRNA inhibiting OBI1
<400> 38
   ggauuuggau ggguuauca 19
<210> 39
   <211> 19
   <212> RNA
   <213> Artificial sequence
<220>
   <223> siRNA inhibiting OBI1
<400> 39
   gaacgaagug auaaguaua 19
<210> 40
   <211> 19
   <212> DNA
   <213> Artificial sequence
<220>
   <223> siRNA inhibiting OBI1
<400> 40
   catctaacat tacttctac 19
<210> 41
   <211> 19
   <212> DNA
   <213> Artificial sequence
<220>
   <223> siRNA inhibiting OBI1
<400> 41
   ttagttagag aacctttgg 19
<210> 42
   <211> 19
   <212> DNA
   <213> Artificial sequence
<220>
   <223> siRNA inhibiting OBI1
<400> 42
   tgataaccca tccaaatcc 19
<210> 43
   <211> 19
   <212> DNA
   <213> Artificial sequence
<220>
   <223> siRNA inhibiting OBI1
<400> 43
   tatacttatc acttcgttc 19
<210> 44
   <211> 19
   <212> RNA
   <213> Artificial sequence
<220>
   <223> siRNA inhibiting OBI1
<400> 44
   caucuaacau uacuucuac 19
<210> 45
   <211> 19
   <212> RNA
   <213> Artificial sequence
<220>
   <223> siRNA inhibiting OBI1
<400> 45
   uuaguuagag aaccuuugg 19
<210> 46
   <211> 19
   <212> RNA
   <213> Artificial sequence
<220>
   <223> siRNA inhibiting OBI1
<400> 46
   ugauaaccca uccaaaucc 19
<210> 47
   <211> 19
   <212> RNA
   <213> Artificial sequence
<220>
   <223> siRNA inhibiting OBI1
<400> 47
   uauacuuauc acuucguuc 19
<210> 48
   <211> 21
   <212> DNA
   <213> Artificial sequence
<220>
   <223> siRNA inhibiting OBI1
<400> 48
   guagaaguaa uguuagaugt t 21
<210> 49
   <211> 21
   <212> DNA
   <213> Artificial sequence
<220>
   <223> siRNA inhibiting OBI1
<400> 49
   ccaaagguuc ucuaacuaat t 21
<210> 50
   <211> 21
   <212> DNA
   <213> Artificial sequence
<220>
   <223> siRNA inhibiting OBI1
<400> 50
   ggauuuggau ggguuaucat t 21
<210> 51
   <211> 21
   <212> DNA
   <213> Artificial sequence
<220>
   <223> siRNA inhibiting OBI1
<400> 51
   gaacgaagug auaaguauat t 21
<210> 52
   <211> 21
   <212> DNA
   <213> Artificial sequence
<220>
   <223> siRNA inhibiting OBI1
<400> 52
   caucuaacau uacuucuact t 21
<210> 53
   <211> 21
   <212> DNA
   <213> Artificial sequence
<220>
   <223> siRNA inhibiting OBI1
<400> 53
   uuaguuagag aaccuuuggt t 21
<210> 54
   <211> 21
   <212> DNA
   <213> Artificial sequence
<220>
   <223> siRNA inhibiting OBI1
<400> 54
   ugauaaccca uccaaaucct t 21
<210> 55
   <211> 21
   <212> DNA
   <213> Artificial Sequence
<220>
   <223> siRNA inhibiting OBI1
<400> 55
   uauacuuauc acuucguuct t 21
<210> 56
   <211> 10
   <212> RNA
   <213> Artificial Sequence
<220>
   <223> shRNA loop
<400> 56
   cuuccuguca 10
<210> 57
   <211> 861
   <212> PRT
   <213> Homo sapiens
<400> 57
<210> 58
   <211> 647
   <212> PRT
   <213> Homo sapiens
<400> 58
   Met Gly Pro Leu Ser Ala Arg Leu Leu Met Gln Arg Gly Arg Pro Lys
   1               5                   10                  15
   Ser Asp Arg Leu Gly Lys Ile Arg Ser Leu Asp Leu Ser Gly Leu Glu
               20                  25                  30
   Leu Leu Ser Glu His Leu Asp Pro Lys Leu Leu Cys Arg Leu Thr Gln
           35                  40                  45
   Leu Gln Glu Leu Asp Leu Ser Asn Asn His Leu Glu Thr Leu Pro Asp
       50                  55                  60
   Asn Leu Gly Leu Ser His Leu Arg Val Leu Arg Cys Ala Asn Asn Gln
   65                  70                  75                  80
   Leu Gly Asp Val Thr Ala Leu Cys Gln Phe Pro Lys Leu Glu Glu Leu
                   85                  90                  95
   Ser Leu Glu Gly Asn Pro Phe Leu Thr Val Asn Asp Asn Leu Lys Val
               100                 105                 110
   Ser Phe Leu Leu Pro Thr Leu Arg Lys Val Asn Gly Lys Asp Ala Ser
           115                 120                 125
   Ser Thr Tyr Ser Gln Val Glu Asn Leu Asn Arg Glu Leu Thr Ser Arg
       130                 135                 140
   Val Thr Ala His Trp Glu Lys Phe Met Ala Thr Leu Gly Pro Glu Glu
   145                 150                 155                 160
   Glu Ala Glu Lys Ala Gln Ala Asp Phe Val Lys Ser Ala Val Arg Asp
                   165                 170                 175
   Val Arg Tyr Gly Pro Glu Ser Leu Ser Glu Phe Thr Gln Trp Arg Val
               180                 185                 190
   Arg Met Ile Ser Glu Glu Leu Val Ala Ala Ser Arg Thr Gln Val Gln
           195                 200                 205
   Lys Ala Asn Ser Pro Glu Lys Pro Pro Glu Ala Gly Ala Ala His Lys
       210                 215                 220
   Pro Arg Ala Arg Leu Ala Ala Leu Lys Arg Pro Asp Asp Val Pro Leu
   225                 230                 235                 240
   Ser Leu Ser Pro Ser Lys Arg Ala Cys Ala Ser Pro Ser Ala Gln Val
                   245                 250                 255
   Glu Gly Ser Pro Val Ala Gly Ser Asp Gly Ser Gln Pro Ala Val Lys
               260                 265                 270
   Leu Glu Pro Leu His Phe Leu Gln Cys His Ser Lys Asn Asn Ser Pro
           275                 280                 285
   Gln Asp Leu Glu Thr Gln Leu Trp Ala Cys Ala Phe Glu Pro Ala Trp
       290                 295                 300
   Glu Glu Gly Ala Thr Ser Gln Thr Val Ala Thr Cys Gly Gly Glu Ala
   305                 310                 315                 320
   Val Cys Val Ile Asp Cys Gln Thr Gly Ile Val Leu His Lys Tyr Lys
                   325                 330                 335
   Ala Pro Gly Glu Glu Phe Phe Ser Val Ala Trp Thr Ala Leu Met Val
               340                 345                 350
   Val Thr Gln Ala Gly His Lys Lys Arg Trp Ser Val Leu Ala Ala Ala
           355                 360                 365
   Gly Leu Arg Gly Leu Val Arg Leu Leu His Val Arg Ala Gly Phe Cys
       370                 375                 380
   Cys Gly Val Ile Arg Ala His Lys Lys Ala Ile Ala Thr Leu Cys Phe
   385                 390                 395                 400
   Ser Pro Ala His Glu Thr His Leu Phe Thr Ala Ser Tyr Asp Lys Arg
                   405                 410                 415
   Ile Ile Leu Trp Asp Ile Gly Val Pro Asn Gln Asp Tyr Glu Phe Gln
               420                 425                 430
   Ala Ser Gln Leu Leu Thr Leu Asp Thr Thr Ser Ile Pro Leu Arg Leu
           435                 440                 445
   Cys Pro Val Ala Ser Cys Pro Asp Ala Arg Leu Leu Ala Gly Cys Glu
       450                 455                 460
   Gly Gly Cys Cys Cys Trp Asp Val Arg Leu Asp Gln Pro Gln Lys Arg
   465                 470                 475                 480
   Arg Val Cys Glu Val Glu Phe Val Phe Ser Glu Gly Ser Glu Ala Ser
                   485                 490                 495
   Gly Arg Arg Val Asp Gly Leu Ala Phe Val Asn Glu Asp Ile Val Ala
               500                 505                 510
   Ser Lys Gly Ser Gly Leu Gly Thr Ile Cys Leu Trp Ser Trp Arg Gln
           515                 520                 525
   Thr Trp Gly Gly Arg Gly Ser Gln Ser Thr Val Ala Val Val Val Leu
       530                 535                 540
   Ala Arg Leu Gln Trp Ser Ser Thr Glu Leu Ala Tyr Phe Ser Leu Ser
   545                 550                 555                 560
   Ala Cys Pro Asp Lys Gly Ile Val Leu Cys Gly Asp Glu Glu Gly Asn
                   565                 570                 575
   Val Trp Leu Tyr Asp Val Ser Asn Ile Leu Lys Gln Pro Pro Leu Leu
               580                 585                 590
   Pro Ala Ala Leu Gln Ala Pro Thr Gln Ile Leu Lys Trp Pro Gln Pro
           595                 600                 605
   Trp Ala Leu Gly Gln Val Val Thr Lys Thr Met Val Asn Thr Val Val
       610                 615                 620
   Ala Asn Ala Ser Phe Thr Tyr Leu Thr Ala Leu Thr Asp Ser Asn Ile
   625                 630                 635                 640
   Val Ala Ile Trp Gly Arg Met
                   645
<210> 59
   <211> 43
   <212> PRT
   <213> Artificial Sequence
<220>
   <223> Ring finger domain of humain Obi1
<400> 59
   Thr Cys His Ile Cys Leu Gly Lys Val Arg Gln Pro Val Ile Cys Ile
   1               5                   10                  15
   Asn Asn His Val Phe Cys Ser Ile Cys Ile Asp Leu Trp Leu Lys Asn
               20                  25                  30
   Asn Ser Gln Cys Pro Ala Cys Arg Val Pro Ile
           35                  40
<210> 60
   <211> 259
   <212> PRT
   <213> Artificial Sequence
<220>
   <223> RING + Coil-coiled domain
<400> 60
   Thr Cys His Ile Cys Leu Gly Lys Val Arg Gln Pro Val Ile Cys Ile
   1               5                   10                  15
   Asn Asn His Val Phe Cys Ser Ile Cys Ile Asp Leu Trp Leu Lys Asn
               20                  25                  30
   Asn Ser Gln Cys Pro Ala Cys Arg Val Pro Ile Thr Pro Glu Asn Pro
           35                  40                  45
   Cys Lys Glu Ile Ile Gly Gly Thr Ser Glu Ser Glu Pro Met Leu Ser
       50                  55                  60
   His Thr Val Arg Lys His Leu Arg Lys Thr Arg Leu Glu Leu Leu His
   65                  70                  75                  80
   Lys Glu Tyr Glu Asp Glu Ile Asp Cys Leu Gln Lys Glu Val Glu Glu
                   85                  90                  95
   Leu Lys Ser Lys Asn Leu Ser Leu Glu Ser Gln Ile Lys Thr Ile Leu
               100                 105                 110
   Asp Pro Leu Thr Leu Val Gln Gly Asn Gln Asn Glu Asp Lys His Leu
           115                 120                 125
   Val Thr Asp Asn Pro Ser Lys Ile Asn Pro Glu Thr Val Ala Glu Trp
       130                 135                 140
   Lys Lys Lys Leu Arg Thr Ala Asn Glu Ile Tyr Glu Lys Val Lys Asp
   145                 150                 155                 160
   Asp Val Asp Lys Leu Lys Glu Ala Asn Lys Lys Leu Lys Leu Glu Asn
                   165                 170                 175
   Gly Gly Leu Val Arg Glu Asn Leu Arg Leu Lys Ala Glu Val Asp Asn
               180                 185                 190
   Arg Ser Pro Gln Lys Phe Gly Arg Phe Ala Val Ala Ala Leu Gln Ser
   195 200 205
   Lys Val Glu Gln Tyr Glu Arg Glu Thr Asn Arg Leu Lys Lys Ala Leu
   210 215 220
   Glu Arg Ser Asp Lys Tyr Ile Glu Glu Leu Glu Ser Gln Val Ala Gln
   225 230 235 240
   Leu Lys Asn Ser Ser Glu Glu Lys Glu Ala Met Asn Ser Ile Cys Gln
   245 250 255
   Thr Ala Leu
<210> 61
   <211> 237
   <212> PRT
   <213> Artificial Sequence
<220>
   <223> RING domain and coil-coiled domain
<400> 61
   Thr Cys His Ile Cys Leu Gly Lys Val Arg Gln Pro Val Ile Cys Ile
   1 5 10 15
   Asn Asn His Val Phe Cys Ser Ile Cys Ile Asp Leu Trp Leu Lys Asn
   20 25 30
   Asn Ser Gln Cys Pro Ala Cys Arg Val Pro Ile Val Arg Lys His Leu
   35 40 45
   Arg Lys Thr Arg Leu Glu Leu Leu His Lys Glu Tyr Glu Asp Glu Ile
   50 55 60
   Asp Cys Leu Gln Lys Glu Val Glu Glu Leu Lys Ser Lys Asn Leu Ser
   65 70 75 80
   Leu Glu Ser Gln Ile Lys Thr Ile Leu Asp Pro Leu Thr Leu Val Gln
   85 90 95
   Gly Asn Gln Asn Glu Asp Lys His Leu Val Thr Asp Asn Pro Ser Lys
   100 105 110
   Ile Asn Pro Glu Thr Val Ala Glu Trp Lys Lys Lys Leu Arg Thr Ala
   115 120 125
   Asn Glu Ile Tyr Glu Lys Val Lys Asp Asp Val Asp Lys Leu Lys Glu
       130                 135                 140
   Ala Asn Lys Lys Leu Lys Leu Glu Asn Gly Gly Leu Val Arg Glu Asn
   145                 150                 155                 160
   Leu Arg Leu Lys Ala Glu Val Asp Asn Arg Ser Pro Gln Lys Phe Gly
                   165                 170                 175
   Arg Phe Ala Val Ala Ala Leu Gln Ser Lys Val Glu Gln Tyr Glu Arg
               180                 185                 190
   Glu Thr Asn Arg Leu Lys Lys Ala Leu Glu Arg Ser Asp Lys Tyr Ile
           195                 200                 205
   Glu Glu Leu Glu Ser Gln Val Ala Gln Leu Lys Asn Ser Ser Glu Glu
       210                 215                 220
   Lys Glu Ala Met Asn Ser Ile Cys Gln Thr Ala Leu Ser
   225                 230                 235
<210> 62
   <211> 493
   <212> PRT
   <213> Artificial Sequence
<220>
   <223> Ring and C-ter domain
<400> 62
   Thr Cys His Ile Cys Leu Gly Lys Val Arg Gln Pro Val Ile Cys Ile
   1               5                   10                  15
   Asn Asn His Val Phe Cys Ser Ile Cys Ile Asp Leu Trp Leu Lys Asn
               20                  25                  30
   Asn Ser Gln Cys Pro Ala Cys Arg Val Pro Ile Ala Asp Gly Lys Gly
           35                  40                  45
   Ser Lys Gly Ser Glu Glu Asp Val Val Ser Lys Asn Gln Gly Asp Ser
       50                  55                  60
   Ala Arg Lys Gln Pro Gly Ser Ser Thr Ser Ser Ser Ser His Leu Ala
   65                  70                  75                  80
   Lys Pro Ser Ser Ser Arg Leu Cys Asp Thr Ser Ser Ala Arg Gln Glu
                   85                  90                  95
   Ser Thr Ser Lys Ala Asp Leu Asn Cys Ser Lys Asn Lys Asp Leu Tyr
   100 105 110
   Gln Glu Gln Val Glu Val Met Leu Asp Val Thr Asp Thr Ser Met Asp
   115 120 125
   Thr Tyr Leu Glu Arg Glu Trp Gly Asn Lys Pro Ser Asp Cys Val Pro
   130 135 140
   Tyr Lys Asp Glu Glu Leu Tyr Asp Leu Pro Ala Pro Cys Thr Pro Leu
   145 150 155 160
   Ser Leu Ser Cys Leu Gln Leu Ser Thr Pro Glu Asn Arg Glu Ser Ser
   165 170 175
   Val Val Gln Ala Gly Gly Ser Lys Lys His Ser Asn His Leu Arg Lys
   180 185 190
   Leu Val Phe Asp Asp Phe Cys Asp Ser Ser Asn Val Ser Asn Lys Asp
   195 200 205
   Ser Ser Glu Asp Asp Ile Ser Arg Ser Glu Asn Glu Lys Lys Ser Glu
   210 215 220
   Cys Phe Ser Ser Pro Lys Thr Gly Phe Trp Asp Cys Cys Ser Thr Ser
   225 230 235 240
   Tyr Ala Gln Asn Leu Asp Phe Glu Ser Ser Glu Gly Asn Thr Ile Ala
   245 250 255
   Asn Ser Val Gly Glu Ile Ser Ser Lys Leu Ser Glu Lys Ser Gly Leu
   260 265 270
   Cys Leu Ser Lys Arg Leu Asn Ser Ile Arg Ser Phe Glu Met Asn Arg
   275 280 285
   Thr Arg Thr Ser Ser Glu Ala Ser Met Asp Ala Ala Tyr Leu Asp Lys
   290 295 300
   Ile Ser Glu Leu Asp Ser Met Met Ser Glu Ser Asp Asn Ser Lys Ser
   305 310 315 320
   Pro Cys Asn Asn Gly Phe Lys Ser Leu Asp Leu Asp Gly Leu Ser Lys
   325 330 335
   Ser Ser Gln Gly Ser Glu Phe Leu Glu Glu Pro Asp Lys Leu Glu Glu
   340 345 350
   Lys Thr Glu Leu Asn Leu Ser Lys Gly Ser Leu Thr Asn Asp Gln Leu
   355 360 365
   Glu Asn Gly Ser Glu Trp Lys Pro Thr Ser Phe Phe Leu Leu Ser Pro
   370 375 380
   Ser Asp Gln Glu Met Asn Glu Asp Phe Ser Leu His Ser Ser Ser Cys
   385 390 395 400
   Pro Val Thr Asn Glu Ile Lys Pro Pro Ser Cys Leu Phe Gln Thr Glu
   405 410 415
   Phe Ser Gln Gly Ile Leu Leu Ser Ser Ser His Arg Leu Phe Glu Asp
   420 425 430
   Gln Arg Phe Gly Ser Ser Leu Phe Lys Met Ser Ser Glu Met His Ser
   435 440 445
   Leu His Asn His Leu Gln Ser Pro Trp Ser Thr Ser Phe Val Pro Glu
   450 455 460
   Lys Arg Asn Lys Asn Val Asn Gln Ser Thr Lys Arg Lys Ile Gln Ser
   465 470 475 480
   Ser Leu Ser Ser Ala Ser Pro Ser Lys Ala Thr Lys Ser
   485 490
<210> 63
   <211> 644
   <212> PRT
   <213> Artificial Sequence
<220>
   <223> Coil-coiled domain and Cter domain
<400> 63
   Val Arg Lys His Leu Arg Lys Thr Arg Leu Glu Leu Leu His Lys Glu
   1               5                   10                  15
   Tyr Glu Asp Glu Ile Asp Cys Leu Gln Lys Glu Val Glu Glu Leu Lys
               20                  25                  30
   Ser Lys Asn Leu Ser Leu Glu Ser Gln Ile Lys Thr Ile Leu Asp Pro
           35                  40                  45
   Leu Thr Leu Val Gln Gly Asn Gln Asn Glu Asp Lys His Leu Val Thr
       50                  55                  60
   Asp Asn Pro Ser Lys Ile Asn Pro Glu Thr Val Ala Glu Trp Lys Lys
   65                  70                  75                  80
   Lys Leu Arg Thr Ala Asn Glu Ile Tyr Glu Lys Val Lys Asp Asp Val
                   85                  90                  95
   Asp Lys Leu Lys Glu Ala Asn Lys Lys Leu Lys Leu Glu Asn Gly Gly
               100                 105                 110
   Leu Val Arg Glu Asn Leu Arg Leu Lys Ala Glu Val Asp Asn Arg Ser
           115                 120                 125
   Pro Gln Lys Phe Gly Arg Phe Ala Val Ala Ala Leu Gln Ser Lys Val
       130                 135                 140
   Glu Gln Tyr Glu Arg Glu Thr Asn Arg Leu Lys Lys Ala Leu Glu Arg
   145                 150                 155                 160
   Ser Asp Lys Tyr Ile Glu Glu Leu Glu Ser Gln Val Ala Gln Leu Lys
                   165                 170                 175
   Asn Ser Ser Glu Glu Lys Glu Ala Met Asn Ser Ile Cys Gln Thr Ala
               180                 185                 190
   Leu Ser Ala Asp Gly Lys Gly Ser Lys Gly Ser Glu Glu Asp Val Val
           195                 200                 205
   Ser Lys Asn Gln Gly Asp Ser Ala Arg Lys Gln Pro Gly Ser Ser Thr
       210                 215                 220
   Ser Ser Ser Ser His Leu Ala Lys Pro Ser Ser Ser Arg Leu Cys Asp
   225                 230                 235                 240
   Thr Ser Ser Ala Arg Gln Glu Ser Thr Ser Lys Ala Asp Leu Asn Cys
                   245                 250                 255
   Ser Lys Asn Lys Asp Leu Tyr Gln Glu Gln Val Glu Val Met Leu Asp
               260                 265                 270
   Val Thr Asp Thr Ser Met Asp Thr Tyr Leu Glu Arg Glu Trp Gly Asn
           275                 280                 285
   Lys Pro Ser Asp Cys Val Pro Tyr Lys Asp Glu Glu Leu Tyr Asp Leu
       290                 295                 300
   Pro Ala Pro Cys Thr Pro Leu Ser Leu Ser Cys Leu Gln Leu Ser Thr
   305                 310                 315                 320
   Pro Glu Asn Arg Glu Ser Ser Val Val Gln Ala Gly Gly Ser Lys Lys
                   325                 330                 335
   His Ser Asn His Leu Arg Lys Leu Val Phe Asp Asp Phe Cys Asp Ser
               340                 345                 350
   Ser Asn Val Ser Asn Lys Asp Ser Ser Glu Asp Asp Ile Ser Arg Ser
           355                 360                 365
   Glu Asn Glu Lys Lys Ser Glu Cys Phe Ser Ser Pro Lys Thr Gly Phe
       370                 375                 380
   Trp Asp Cys Cys Ser Thr Ser Tyr Ala Gln Asn Leu Asp Phe Glu Ser
   385                 390                 395                 400
   Ser Glu Gly Asn Thr Ile Ala Asn Ser Val Gly Glu Ile Ser Ser Lys
                   405                 410                 415
   Leu Ser Glu Lys Ser Gly Leu Cys Leu Ser Lys Arg Leu Asn Ser Ile
               420                 425                 430
   Arg Ser Phe Glu Met Asn Arg Thr Arg Thr Ser Ser Glu Ala Ser Met
           435                 440                 445
   Asp Ala Ala Tyr Leu Asp Lys Ile Ser Glu Leu Asp Ser Met Met Ser
       450                 455                 460
   Glu Ser Asp Asn Ser Lys Ser Pro Cys Asn Asn Gly Phe Lys Ser Leu
   465                 470                 475                 480
   Asp Leu Asp Gly Leu Ser Lys Ser Ser Gln Gly Ser Glu Phe Leu Glu
                   485                 490                 495
   Glu Pro Asp Lys Leu Glu Glu Lys Thr Glu Leu Asn Leu Ser Lys Gly
               500                 505                 510
   Ser Leu Thr Asn Asp Gln Leu Glu Asn Gly Ser Glu Trp Lys Pro Thr
           515                 520                 525
   Ser Phe Phe Leu Leu Ser Pro Ser Asp Gln Glu Met Asn Glu Asp Phe
       530                 535                 540
   Ser Leu His Ser Ser Ser Cys Pro Val Thr Asn Glu Ile Lys Pro Pro
   545                 550                 555                 560
   Ser Cys Leu Phe Gln Thr Glu Phe Ser Gln Gly Ile Leu Leu Ser Ser
                   565                 570                 575
   Ser His Arg Leu Phe Glu Asp Gln Arg Phe Gly Ser Ser Leu Phe Lys
               580                 585                 590
   Met Ser Ser Glu Met His Ser Leu His Asn His Leu Gln Ser Pro Trp
           595                 600                 605
   Ser Thr Ser Phe Val Pro Glu Lys Arg Asn Lys Asn Val Asn Gln Ser
       610                 615                 620
   Thr Lys Arg Lys Ile Gln Ser Ser Leu Ser Ser Ala Ser Pro Ser Lys
   625                 630                 635                 640
   Ala Thr Lys Ser
<210> 64
   <211> 35
   <212> DNA
   <213> Artificial sequence
<220>
   <223> derived from ORC1
<400> 64
   ctggcggccg caccatggca cactacccca caagg 35
<210> 65
   <211> 30
   <212> DNA
   <213> Artificial sequence
<220>
   <223> derived from ORC1
<400> 65
   ctggcggccg cctcgtcttt cagcgcatac 30
<210> 66
   <211> 40
   <212> DNA
   <213> Artificial sequence
<220>
   <223> derived from ORC2
<400> 66
   ctggcggccg caccatgagt aaaccagaat taaaggaaga 40
<210> 67
   <211> 32
   <212> DNA
   <213> Artificial sequence
<220>
   <223> derived from ORC2
<400> 67
   ctggcggccg cagcctcctc ttcttccttt tc 32
<210> 68
   <211> 30
   <212> DNA
   <213> Artificial sequence
<220>
   <223> derived from LRWD1
<400> 68
   ctggcggccg caccatgggc cccctctcgg 30
<210> 69
   <211> 28
   <212> DNA
   <213> Artificial sequence
<220>
   <223> derived from LRWD1
<400> 69
   ctggcggccg ccatcctccc ccagatgg 28
<210> 70
   <211> 50
   <212> DNA
   <213> Artificial sequence
<220>
   <223> derived from FLAG tag
<400> 70
   gatccgccaa catggactac aaggacgacg atgacaagtc catggctggg 50
<210> 71
   <211> 50
   <212> DNA
   <213> Artificial sequence
<220>
   <223> derived from FLAG tag
<400> 71
   aattcccagc catggacttg tcatcgtcgt ccttgtagtc catgttggcg 50
<210> 72
   <211> 34
   <212> DNA
   <213> Artificial sequence
<220>
   <223> derived from ORC1
<400> 72
   ctgtctagag ccatggcaca ctaccccaca aggc 34
<210> 73
   <211> 31
   <212> DNA
   <213> Artificial sequence
<220>
   <223> derived from ORC1
<400> 73
   ctgtctagat tactcgtctt tcagcgcata c 31
<210> 74
   <211> 35
   <212> DNA
   <213> Artificial sequence
<220>
   <223> derived from ORC2
<400> 74
   ctgccatggg aagtaaacca gaattaaagg aagac 35
<210> 75
   <211> 30
   <212> DNA
   <213> Artificial sequence
<220>
   <223> derived from ORC2
<400> 75
   ctgctcgagt caagcctcct cttcttcctt 30
<210> 76
   <211> 22
   <212> DNA
   <213> Artificial sequence
<220>
   <223> derived from ORC3
<400> 76
   ctgccatggc tacgtcctcg at 22
<210> 77
   <211> 30
   <212> DNA
   <213> Artificial sequence
<220>
   <223> derived from ORC3
<400> 77
   ctgtctagat tagcagcctc cccatgttag 30
<210> 78
   <211> 29
   <212> DNA
   <213> Artificial sequence
<220>
   <223> derived from ORC4
<400> 78
   ctgctcgaga gcagtcgtaa atcaaagag 29
<210> 79
   <211> 32
   <212> DNA
   <213> Artificial sequence
<220>
   <223> derived from ORC4
<400> 79
   ctgtctagat tataaccagc ttagtgagga tg 32
<210> 80
   <211> 29
   <212> DNA
   <213> Artificial sequence
<220>
   <223> derived from ORC5
<400> 80
   ctggaattct ccccacttgg aaaacgtgg 29
<210> 81
   <211> 33
   <212> DNA
   <213> Artificial sequence
<220>
   <223> derived from ORC5
<400> 81
   ctgctcgagt cacaagaaat catacaagta ttt 33
<210> 82
   <211> 25
   <212> DNA
   <213> Artificial sequence
<220>
   <223> derived from ORC6
<400> 82
   ctgctcgagg ggtcggagct gatcg 25
<210> 83
   <211> 31
   <212> DNA
   <213> Artificial sequence
<220>
   <223> derived from ORC6
<400> 83
   ctgtctagat tactctgctg tagccttttg a 31
<210> 84
   <211> 18
   <212> DNA
   <213> Artificial sequence
<220>
   <223> derived from obi1
<400> 84
   ctgccatggc tcagaccg 18
<210> 85
   <211> 34
   <212> DNA
   <213> Artificial sequence
<220>
   <223> derived from obi1
<400> 85
   ctgtctagat taacttttag ttgcttttga tggg 34
<210> 86
   <211> 27
   <212> DNA
   <213> Artificial sequence
<220>
   <223> derived from obi1
<400> 86
   ctgccatggc ttgcagagtc cccatca 27
<210> 87
   <211> 32
   <212> DNA
   <213> Artificial sequence
<220>
   <223> derived from obi1
<400> 87
   ctgtctagat tatttgccat ctgcagaaag tg 32
<210> 88
   <211> 26
   <212> DNA
   <213> Artificial sequence
<220>
   <223> derived from obi1
<400> 88
   ctgccatggg gagcaaaggc agtgag 26
<210> 89
   <211> 32
   <212> DNA
   <213> Artificial sequence
<220>
   <223> derived from obi1
<400> 89
   ctgccatgga tcctcctata atttctttgc aa 32
<210> 90
   <211> 30
   <212> DNA
   <213> Artificial sequence
<220>
   <223> derived from obi1
<400> 90
   ctggaattct atggcgcaga ctgttcagaa 30
<210> 91
   <211> 32
   <212> DNA
   <213> Artificial sequence
<220>
   <223> derived from obi1
<400> 91
   ctgctcgagt catggcttaa aggactttgg ag 32
<210> 92
   <211> 40
   <212> DNA
   <213> Artificial sequence
<220>
   <223> derived from cdt1
<400> 92
   aattctatgt atgatgttcc tgattatgct agcctctaat 40
<210> 93
   <211> 40
   <212> DNA
   <213> Artificial sequence
<220>
   <223> derived from cdt1
<400> 93
   ctagattaga ggctagcata atcaggaaca tcatacatag 40
<210> 94
   <211> 27
   <212> DNA
   <213> Artificial sequence
<220>
   <223> derived from cdt1
<400> 94
   ctgaagctta ccatggagca gcgccgc 27
<210> 95
   <211> 25
   <212> DNA
   <213> Artificial Sequence
<220>
   <223> derived from cdt1
<400> 95
   ctggaattca gcccctcctc agcac 25
<210> 96
   <211> 3552
   <212> DNA
   <213> Homo sapiens
<400> 96
   ggcgaagagt agcggtaggt cggcgggact tccgtgttgg cgggattctg aacgctgcca      60
   tggctcagac cgtgcagaat gttacattgt cgctcactct gcccatcacg tgccacattt     120
   gcttggggaa ggtacgtcag cctgtcatat gcatcaacaa ccatgtattt tgttcgattt     180
   gtattgattt gtggttgaag aataatagcc agtgtccagc ttgcagagtc cccatcactc     240
   ctgaaaatcc ttgcaaagaa attataggag gaacaagtga aagtgaacct atgctaagcc     300
   atacggtcag gaagcatctt cggaaaacta gacttgaatt actacacaaa gaatatgagg     360
   acgaaataga ttgtttacag aaagaagtag aagagcttaa gagtaaaaat ctcagcttgg     420
   agtcacagat caaaactatt ctggatcctt taaccttggt gcagggcaac caaaatgaag     480
   acaaacatct agtcacagat aatccaagta aaattaaccc agaaactgta gcagagtgga     540
   agaaaaaact cagaacagct aatgaaatct atgaaaaagt gaaagatgat gtggataagc     600
   taaaggaggc aaataaaaaa ttgaaattgg aaaatggtgg tctggtgagg gagaatttac     660
   gactgaaggc tgaagttgat aacagatcac ctcaaaagtt tggaaggttt gcagttgctg     720
   ctcttcagtc caaagtagaa cagtatgagc gtgaaaccaa tcgcctcaag aaagccctgg     780
   aacgaagtga taagtatata gaggaactag aatctcaagt tgcacagcta aaaaattcaa     840
   gtgaagagaa agaagctatg aattccattt gccagacagc actttctgca gatggcaaag     900
   ggagcaaagg cagtgaggag gatgtggtgt caaagaatca aggcgatagt gccagaaagc 960
   agcctggctc atccacctcc agttcttctc acctagcgaa gccttccagc agcagactgt 1020
   gtgacaccag ttctgcaagg caggaaagta ccagcaaagc agaccttaac tgttctaaga 1080
   acaaagacct atatcaagaa caggtagaag taatgttaga tgtgacagat acaagtatgg 1140
   atacttattt ggaaagagaa tgggggaata aaccaagtga ctgtgtaccc tacaaagatg 1200
   aagaacttta tgatcttcca gctccttgta ctcctttgtc ccttagttgc cttcagctca 1260
   gtactccaga aaatagagag agctctgtgg tccaagcagg aggttccaaa aagcactcaa 1320
   accatctcag aaaattggtg tttgatgatt tttgtgattc ttcaaatgtt tctaataaag 1380
   attcttcaga agatgatata agtagaagtg aaaatgaaaa gaaatcagaa tgtttttctt 1440
   ccccaaagac aggattttgg gactgttgtt ccacaagcta tgcccaaaac ttagattttg 1500
   aaagttcaga ggggaacacg atagcaaatt ctgttggaga aatatcttca aaattgagtg 1560
   agaaatcagg cttatgttta tccaaaaggt tgaattctat tcgctctttt gaaatgaacc 1620
   ggacaagaac atccagtgaa gcatcgatgg atgctgctta ccttgacaaa atctctgagt 1680
   tggattcaat gatgtcagag tcagacaaca gcaagagccc ttgtaataac ggttttaagt 1740
   cactggattt ggatgggtta tcaaagtcat ctcaaggcag tgaatttctt gaggaacctg 1800
   ataagttgga agaaaaaact gagctaaacc tttccaaagg ttctctaact aatgatcagt 1860
   tagaaaatgg aagtgaatgg aaacccactt ctttttttct cctctctcca tctgaccaag 1920
   aaatgaatga agatttttca ctccattcca gttcttgtcc agtaactaat gaaatcaaac 1980
   ccccaagctg cttgtttcag acagagtttt cccagggcat tttgttaagc agttcacatc 2040
   gactatttga agatcaaaga tttgggtcat ctttgtttaa gatgtcctca gagatgcaca 2100
   gtcttcataa ccaccttcag tctccttggt ctacttcctt tgtgcctgaa aagaggaata 2160
   aaaatgtgaa tcaatcaaca aaaagaaaaa tccagagcag cctttccagt gccagcccat 2220
   caaaagcaac taaaagttga ctcattagaa aggtgtcatt tgtggttttg tcctgagaga 2280
   aatagaaaag ttgttaaagt tacctttttt cctcataaaa gttctataca aattggaatt 2340
   gataatcttt agtcaagtat caagtcagga tggtggatta acctgtaccc agaatactta 2400
   ttgttcattt tgaaaagact ttgttctttt catttttatt tgggagtctt tgtgaccaga 2460
   gaagttaggg aggaggttat ttttgtgttt tggggttggt tggttggttg gttttgtttt 2520
   tggttttgtt tttttactga atttgatatg tatctcggtt ggatatacat tgttttttta 2580
   aaaaatgtta tttaactgtt agatacagtg gcctgttgat aagccccact tgtcttcaga 2640
   acttggattt cttaaataaa acttttagtg ttgtctatac actgctcaat aagacacttg 2700
   agtttaagct tttcccaggg tggaaattat tttacctgtc cctttttatt tatgtttagt 2760
   gatggcctag tttttctgca gggccatgat ggagaaatag cactctagcc ttagtccaat 2820
   attgatttac tttctttttt taggttttat gtatatgttt gcatttttta gcattgtgtt 2880
   ttgtccagtt ttgtgaaaat gttctgctag tatgaaagaa aacattttct atatgaagac 2940
   atttgtttta tgttaggtag cttacatttt ctcctctgcg tgtgtgtgta tgtgtgtaaa 3000
   atcagaaatt tagcatacta tggaaagaag gcatggagca cttgggttta gaggaaccta 3060
   aaacatcata gcttcattgt tccagatgta acaggtttga aagagctcat cgccaagttc 3120
   ttgatccact tgcattccag gggagttttc ttttgagtag tatgtttctt gtttgcatgt 3180
   tcctgttctt tgtggaaact atgcatggta gcatttttgc ttgctgtgtt ttccatactt 3240
   aagaaaaaga ggtttcagtt ggctgataga atatctttta tgtaggacaa aacttttctg 3300
   tgaagagtgt tgagggggtg aagataggta agaggtaagc acaattttta atttaggctc 3360
   tgaaaaagtg tattgttcta aacgtatttg gtatgcctat ataggtcttt aaaaatgggt 3420
   atgtatgctg tttaatgtgc actgaacatt ttacattaat attgtactgt tttacattaa 3480
   tactgcatgc ttttctatgt gaattgaata aagaatgtca taagcactgt gttcaaaaaa 3540
   aaaaaaaaaa aa 3552
<210> 97
   <211> 2181
   <212> DNA
   <213> Homo sapiens
<400> 97
   atggctcaga ccgtgcagaa tgttacattg tcgctcactc tgcccatcac gtgccacatt      60
   tgcttgggga aggtacgtca gcctgtcata tgcatcaaca accatgtatt ttgttcgatt     120
   tgtattgatt tgtggttgaa gaataatagc cagtgtccag cttgcagagt ccccatcact     180
   cctgaaaatc cttgcaaaga aattatagga ggaacaagtg aaagtgaacc tatgctaagc     240
   catacggtca ggaagcatct tcggaaaact agacttgaat tactacacaa agaatatgag     300
   gacgaaatag attgtttaca gaaagaagta gaagagctta agagtaaaaa tctcagcttg     360
   gagtcacaga tcaaaactat tctggatcct ttaaccttgg tgcagggcaa ccaaaatgaa     420
   gacaaacatc tagtcacaga taatccaagt aaaattaacc cagaaactgt agcagagtgg     480
   aagaaaaaac tcagaacagc taatgaaatc tatgaaaaag tgaaagatga tgtggataag     540
   ctaaaggagg caaataaaaa attgaaattg gaaaatggtg gtctggtgag ggagaattta     600
   cgactgaagg ctgaagttga taacagatca cctcaaaagt ttggaaggtt tgcagttgct     660
   gctcttcagt ccaaagtaga acagtatgag cgtgaaacca atcgcctcaa gaaagccctg     720
   gaacgaagtg ataagtatat agaggaacta gaatctcaag ttgcacagct aaaaaattca     780
   agtgaagaga aagaagctat gaattccatt tgccagacag cactttctgc agatggcaaa     840
   gggagcaaag gcagtgagga ggatgtggtg tcaaagaatc aaggcgatag tgccagaaag     900
   cagcctggct catccacctc cagttcttct cacctagcga agccttccag cagcagactg      960
   tgtgacacca gttctgcaag gcaggaaagt accagcaaag cagaccttaa ctgttctaag     1020
   aacaaagacc tatatcaaga acaggtagaa gtaatgttag atgtgacaga tacaagtatg     1080
   gatacttatt tggaaagaga atgggggaat aaaccaagtg actgtgtacc ctacaaagat     1140
   gaagaacttt atgatcttcc agctccttgt actcctttgt cccttagttg ccttcagctc     1200
   agtactccag aaaatagaga gagctctgtg gtccaagcag gaggttccaa aaagcactca     1260
   aaccatctca gaaaattggt gtttgatgat ttttgtgatt cttcaaatgt ttctaataaa     1320
   gattcttcag aagatgatat aagtagaagt gaaaatgaaa agaaatcaga atgtttttct     1380
   tccccaaaga caggattttg ggactgttgt tccacaagct atgcccaaaa cttagatttt     1440
   gaaagttcag aggggaacac gatagcaaat tctgttggag aaatatcttc aaaattgagt     1500
   gagaaatcag gcttatgttt atccaaaagg ttgaattcta ttcgctcttt tgaaatgaac     1560
   cggacaagaa catccagtga agcatcgatg gatgctgctt accttgacaa aatctctgag     1620
   ttggattcaa tgatgtcaga gtcagacaac agcaagagcc cttgtaataa cggttttaag     1680
   tcactggatt tggatgggtt atcaaagtca tctcaaggca gtgaatttct tgaggaacct     1740
   gataagttgg aagaaaaaac tgagctaaac ctttccaaag gttctctaac taatgatcag     1800
   ttagaaaatg gaagtgaatg gaaacccact tctttttttc tcctctctcc atctgaccaa     1860
   gaaatgaatg aagatttttc actccattcc agttcttgtc cagtaactaa tgaaatcaaa     1920
   cccccaagct gcttgtttca gacagagttt tcccagggca ttttgttaag cagttcacat     1980
   cgactatttg aagatcaaag atttgggtca tctttgttta agatgtcctc agagatgcac     2040
   agtcttcata accaccttca gtctccttgg tctacttcct ttgtgcctga aaagaggaat     2100
   aaaaatgtga atcaatcaac aaaaagaaaa atccagagca gcctttccag tgccagccca     2160
   tcaaaagcaa ctaaaagttg a                                               2181
<210> 98
   <211> 3550
   <212> DNA
   <213> Homo sapiens
<400> 98
   ggcgaagagt agcggtaggt cggcgggact tccgtgttgg cgggattctg aacgctgcca      60
   tggctcagac cgtgcagaat gttacattgt cgctcactct gcccatcacg tgccacattt     120
   gcttggggaa ggtacgtcag cctgtcatat gcatcaacaa ccatgtattt tgttcgattt     180
   gtattgattt gtggttgaag aataatagcc agtgtccagc ttgcagagtc cccatcactc     240
   ctgaaaatcc ttgcaaagaa attataggag gaacaagtga aagtgaacct atgctaagcc     300
   atacggtcag gaagcatctt cggaaaacta gacttgaatt actacacaaa gaatatgagg     360
   acgaaataga ttgtttacag aaagaagtag aagagcttaa gagtaaaaat ctcagcttgg 420
   agtcacagat caaaactatt ctggatcctt taaccttggt gcagggcaac caaaatgaag 480
   acaaacatct agtcacagat aatccaagta aaattaaccc agaaactgta gcagagtgga 540
   agaaaaaact cagaacagct aatgaaatct atgaaaaagt gaaagatgat gtggataagc 600
   taaaggaggc aaataaaaaa ttgaaattgg aaaatggtgg tctggtgagg gagaatttac 660
   gactgaaggc tgaagttgat aacagatcac ctcaaaagtt tggaaggttt gcagttgctg 720
   ctcttcagtc caaagtagaa cagtatgagc gtgaaaccaa tcgcctcaag aaagccctgg 780
   aacgaagtga taagtatata gaggaactag aatctcaagt tgcacagcta aaaaattcaa 840
   gtgaagagaa agaagctatg aattccattt gccagacagc actttctgca gatggcaaag 900
   ggagcaaagg cagtgaggag gatgtggtgt caaagaatca aggcgatagt gccagaaagc 960
   agcctggctc atccacctcc agttcttctc acctagcgaa gccttccagc agcagactgt 1020
   gtgacaccag ttctgcaagg caggaaagta ccagcaaagc agaccttaac tgttctaaga 1080
   acaaagacct atatcaagaa caggtagaag taatgttaga tgtgacagat acaagtatgg 1140
   atacttattt ggaaagagaa tgggggaata aaccaagtga ctgtgtaccc tacaaagatg 1200
   aagaacttta tgatcttcca gctccttgta ctcctttgtc ccttagttgc cttcagctca 1260
   gtactccaga aaatagagag agctctgtgg tccaagcagg aggttccaaa aagcactcaa 1320
   accatctcag aaaattggtg tttgatgatt tttgtgattc ttcaaatgtt tctaataaag 1380
   attcttcaga agatgatata agtagaagtg aaaatgaaaa gaaatcagtg tttttcttcc 1440
   ccaaagacag gattttggga ctgttgttcc acaagctatg cccaaaactt agattttgaa 1500
   agttcagagg ggaacacgat agcaaattct gttggagaaa tatcttcaaa attgagtgag 1560
   aaatcaggct tatgtttatc caaaaggttg aattctattc gctcttttga aatgaaccgg 1620
   acaagaacat ccagtgaagc atcgatggat gctgcttacc ttgacaaaat ctctgagttg 1680
   gattcaatga tgtcagagtc agacaacagc aagagccctt gtaataacgg ttttaagtca 1740
   ctggatttgg atgggttatc aaagtcatct caaggcagtg aatttcttga ggaacctgat 1800
   aagttggaag aaaaaactga gctaaacctt tccaaaggtt ctctaactaa tgatcagtta 1860
   gaaaatggaa gtgaatggaa acccacttct ttttttctcc tctctccatc tgaccaagaa 1920
   atgaatgaag atttttcact ccattccagt tcttgtccag taactaatga aatcaaaccc 1980
   ccaagctgct tgtttcagac agagttttcc cagggcattt tgttaagcag ttcacatcga 2040
   ctatttgaag atcaaagatt tgggtcatct ttgtttaaga tgtcctcaga gatgcacagt 2100
   cttcataacc accttcagtc tccttggtct acttcctttg tgcctgaaaa gaggaataaa 2160
   aatgtgaatc aatcaacaaa aagaaaaatc cagagcagcc tttccagtgc cagcccatca 2220
   aaagcaacta aaagttgact cattagaaag gtgtcatttg tggttttgtc ctgagagaaa 2280
   tagaaaagtt gttaaagtta ccttttttcc tcataaaagt tctatacaaa ttggaattga 2340
   taatctttag tcaagtatca agtcaggatg gtggattaac ctgtacccag aatacttatt 2400
   gttcattttg aaaagacttt gttcttttca tttttatttg ggagtctttg tgaccagaga 2460
   agttagggag gaggttattt ttgtgttttg gggttggttg gttggttggt tttgtttttg 2520
   gttttgtttt tttactgaat ttgatatgta tctcggttgg atatacattg tttttttaaa 2580
   aaatgttatt taactgttag atacagtggc ctgttgataa gccccacttg tcttcagaac 2640
   ttggatttct taaataaaac ttttagtgtt gtctatacac tgctcaataa gacacttgag 2700
   tttaagcttt tcccagggtg gaaattattt tacctgtccc tttttattta tgtttagtga 2760
   tggcctagtt tttctgcagg gccatgatgg agaaatagca ctctagcctt agtccaatat 2820
   tgatttactt tcttttttta ggttttatgt atatgtttgc attttttagc attgtgtttt 2880
   gtccagtttt gtgaaaatgt tctgctagta tgaaagaaaa cattttctat atgaagacat 2940
   ttgttttatg ttaggtagct tacattttct cctctgcgtg tgtgtgtatg tgtgtaaaat 3000
   cagaaattta gcatactatg gaaagaaggc atggagcact tgggtttaga ggaacctaaa 3060
   acatcatagc ttcattgttc cagatgtaac aggtttgaaa gagctcatcg ccaagttctt 3120
   gatccacttg cattccaggg gagttttctt ttgagtagta tgtttcttgt ttgcatgttc 3180
   ctgttctttg tggaaactat gcatggtagc atttttgctt gctgtgtttt ccatacttaa 3240
   gaaaaagagg tttcagttgg ctgatagaat atcttttatg taggacaaaa cttttctgtg 3300
   aagagtgttg agggggtgaa gataggtaag aggtaagcac aatttttaat ttaggctctg 3360
   aaaaagtgta ttgttctaaa cgtatttggt atgcctatat aggtctttaa aaatgggtat 3420
   gtatgctgtt taatgtgcac tgaacatttt acattaatat tgtactgttt tacattaata 3480
   ctgcatgctt ttctatgtga attgaataaa gaatgtcata agcactgtgt tcaaaaaaaa 3540
   aaaaaaaaaa 3550
<210> 99
   <211> 1440
   <212> DNA
   <213> Homo sapiens
<400> 99
   atggctcaga ccgtgcagaa tgttacattg tcgctcactc tgcccatcac gtgccacatt 60
   tgcttgggga aggtacgtca gcctgtcata tgcatcaaca accatgtatt ttgttcgatt 120
   tgtattgatt tgtggttgaa gaataatagc cagtgtccag cttgcagagt ccccatcact 180
   cctgaaaatc cttgcaaaga aattatagga ggaacaagtg aaagtgaacc tatgctaagc 240
   catacggtca ggaagcatct tcggaaaact agacttgaat tactacacaa agaatatgag 300
   gacgaaatag attgtttaca gaaagaagta gaagagctta agagtaaaaa tctcagcttg 360
   gagtcacaga tcaaaactat tctggatcct ttaaccttgg tgcagggcaa ccaaaatgaa 420
   gacaaacatc tagtcacaga taatccaagt aaaattaacc cagaaactgt agcagagtgg 480
   aagaaaaaac tcagaacagc taatgaaatc tatgaaaaag tgaaagatga tgtggataag 540
   ctaaaggagg caaataaaaa attgaaattg gaaaatggtg gtctggtgag ggagaattta 600
   cgactgaagg ctgaagttga taacagatca cctcaaaagt ttggaaggtt tgcagttgct 660
   gctcttcagt ccaaagtaga acagtatgag cgtgaaacca atcgcctcaa gaaagccctg 720
   gaacgaagtg ataagtatat agaggaacta gaatctcaag ttgcacagct aaaaaattca 780
   agtgaagaga aagaagctat gaattccatt tgccagacag cactttctgc agatggcaaa 840
   gggagcaaag gcagtgagga ggatgtggtg tcaaagaatc aaggcgatag tgccagaaag 900
   cagcctggct catccacctc cagttcttct cacctagcga agccttccag cagcagactg 960
   tgtgacacca gttctgcaag gcaggaaagt accagcaaag cagaccttaa ctgttctaag 1020
   aacaaagacc tatatcaaga acaggtagaa gtaatgttag atgtgacaga tacaagtatg 1080
   gatacttatt tggaaagaga atgggggaat aaaccaagtg actgtgtacc ctacaaagat 1140
   gaagaacttt atgatcttcc agctccttgt actcctttgt cccttagttg ccttcagctc 1200
   agtactccag aaaatagaga gagctctgtg gtccaagcag gaggttccaa aaagcactca 1260
   aaccatctca gaaaattggt gtttgatgat ttttgtgatt cttcaaatgt ttctaataaa 1320
   gattcttcag aagatgatat aagtagaagt gaaaatgaaa agaaatcagt gtttttcttc 1380
   cccaaagaca ggattttggg actgttgttc cacaagctat gcccaaaact tagattttga 1440
<210> 100
   <211> 479
   <212> PRT
   <213> Homo sapiens
<400> 100
   Met Ala Gln Thr Val Gln Asn Val Thr Leu Ser Leu Thr Leu Pro Ile
   1               5                   10                  15
   Thr Cys His Ile Cys Leu Gly Lys Val Arg Gln Pro Val Ile Cys Ile
               20                  25                  30
   Asn Asn His Val Phe Cys Ser Ile Cys Ile Asp Leu Trp Leu Lys Asn
           35                  40                  45
   Asn Ser Gln Cys Pro Ala Cys Arg Val Pro Ile Thr Pro Glu Asn Pro
       50                  55                  60
   Cys Lys Glu Ile Ile Gly Gly Thr Ser Glu Ser Glu Pro Met Leu Ser
   65                  70                  75                  80
   His Thr Val Arg Lys His Leu Arg Lys Thr Arg Leu Glu Leu Leu His
                   85                  90                  95
   Lys Glu Tyr Glu Asp Glu Ile Asp Cys Leu Gln Lys Glu Val Glu Glu
               100                 105                 110
   Leu Lys Ser Lys Asn Leu Ser Leu Glu Ser Gln Ile Lys Thr Ile Leu
           115                 120                 125
   Asp Pro Leu Thr Leu Val Gln Gly Asn Gln Asn Glu Asp Lys His Leu
       130                 135                 140
   Val Thr Asp Asn Pro Ser Lys Ile Asn Pro Glu Thr Val Ala Glu Trp
   145                 150                 155                 160
   Lys Lys Lys Leu Arg Thr Ala Asn Glu Ile Tyr Glu Lys Val Lys Asp
                   165                 170                 175
   Asp Val Asp Lys Leu Lys Glu Ala Asn Lys Lys Leu Lys Leu Glu Asn
               180                 185                 190
   Gly Gly Leu Val Arg Glu Asn Leu Arg Leu Lys Ala Glu Val Asp Asn
           195                 200                 205
   Arg Ser Pro Gln Lys Phe Gly Arg Phe Ala Val Ala Ala Leu Gln Ser
       210                 215                 220
   Lys Val Glu Gln Tyr Glu Arg Glu Thr Asn Arg Leu Lys Lys Ala Leu
   225                 230                 235                 240
   Glu Arg Ser Asp Lys Tyr Ile Glu Glu Leu Glu Ser Gln Val Ala Gln
                   245                 250                 255
   Leu Lys Asn Ser Ser Glu Glu Lys Glu Ala Met Asn Ser Ile Cys Gln
               260                 265                 270
   Thr Ala Leu Ser Ala Asp Gly Lys Gly Ser Lys Gly Ser Glu Glu Asp
           275                 280                 285
   Val Val Ser Lys Asn Gln Gly Asp Ser Ala Arg Lys Gln Pro Gly Ser
       290                 295                 300
   Ser Thr Ser Ser Ser Ser His Leu Ala Lys Pro Ser Ser Ser Arg Leu
   305                 310                 315                 320
   Cys Asp Thr Ser Ser Ala Arg Gln Glu Ser Thr Ser Lys Ala Asp Leu
                   325                 330                 335
   Asn Cys Ser Lys Asn Lys Asp Leu Tyr Gln Glu Gln Val Glu Val Met
               340                 345                 350
   Leu Asp Val Thr Asp Thr Ser Met Asp Thr Tyr Leu Glu Arg Glu Trp
           355                 360                 365
   Gly Asn Lys Pro Ser Asp Cys Val Pro Tyr Lys Asp Glu Glu Leu Tyr
       370                 375                 380
   Asp Leu Pro Ala Pro Cys Thr Pro Leu Ser Leu Ser Cys Leu Gln Leu
   385                 390                 395                 400
   Ser Thr Pro Glu Asn Arg Glu Ser Ser Val Val Gln Ala Gly Gly Ser
                   405                 410                 415
   Lys Lys His Ser Asn His Leu Arg Lys Leu Val Phe Asp Asp Phe Cys
               420                 425                 430
   Asp Ser Ser Asn Val Ser Asn Lys Asp Ser Ser Glu Asp Asp Ile Ser
           435                 440                 445
   Arg Ser Glu Asn Glu Lys Lys Ser Val Phe Phe Phe Pro Lys Asp Arg
       450                 455                 460
   Ile Leu Gly Leu Leu Phe His Lys Leu Cys Pro Lys Leu Arg Phe
   465                 470                 475
<210> 101
   <211> 27
   <212> PRT
   <213> Artificial Sequence
<220>
   <223> derived from human Obi1 protein
<400> 101
   Lys Ser Glu Cys Phe Ser Ser Pro Lys Thr Gly Phe Trp Asp Cys Cys
   1               5                   10                  15
   Ser Thr Ser Tyr Ala Gln Asn Leu Asp Phe Glu
               20                  25
<210> 102
   <211> 81
   <212> DNA
   <213> Artificial Sequence
<220>
   <223> derived from human Obi1 gene
<400> 102
   aaatcagaat gtttttcttc cccaaagaca ggattttggg actgttgttc cacaagctat        60
   gcccaaaact tagattttga a                                                  81
<210> 103
   <211> 79
   <212> DNA
   <213> Artificial Sequence
<220>
   <223> derived from human mutated Obi1 gene
<400> 103
   aaatcagtgt ttttcttccc caaagacagg attttgggac tgttgttcca caagctatgc        60
   ccaaaactta gattttgaa                                                     79
<210> 104
   <211> 25
   <212> PRT
   <213> Artificial Sequence
<220>
   <223> derived from mutated Obi1 protein
<400> 104
   Lys Ser Val Phe Phe Phe Pro Lys Asp Arg Ile Leu Gly Leu Leu Phe
   1               5                   10                  15
   His Lys Leu Cys Pro Lys Leu Arg Phe
               20                  25
<210> 105
   <211> 1104
   <212> DNA
   <213> Artificial Sequence
<220>
   <223> ucléic acid coding for a dominant negative form of Obi1
<400> 105
   atggagcaaa agctcatttc tgaagaggac ttgaatgaaa tggagcaaaa gctcatttct      60
   gaagaggact tgaatgaaat ggagcaaaag ctcatttctg aagaggactt gaatgaaatg     120
   gagcaaaagc tcatttctga agaggacttg aatgaaatgg agcaaaagct catttctgaa     180
   gaggacttga atgaaatgga gagcttgggc gacctcacca tgtctctggc tctcaaactg     240
   gctggactgg acatcggatc catggctcag accgtgcaga atgttacatt gtcgctcact     300
   ctgcccatca cgtgccacat ttgcttgggg aaggtacgtc agcctgtcat atgcatcaac     360
   aaccatgtat tttgttcgat ttgtattgat ttgtggttga agaataatag ccagtgtcca     420
   gcttgcagag tccccatcac tcctgaaaat ccttgcaaag aaattatagg aggaacaagt     480
   gaaagtgaac ctatgctaag ccatacggtc aggaagcatc ttcggaaaac tagacttgaa     540
   ttactacaca aagaatatga ggacgaaata gattgtttac agaaagaagt agaagagctt     600
   aagagtaaaa atctcagctt ggagtcacag atcaaaacta ttctggatcc tttaaccttg     660
   gtgcagggca accaaaatga agacaaacat ctagtcacag ataatccaag taaaattaac     720
   ccagaaactg tagcagagtg gaagaaaaaa ctcagaacag ctaatgaaat ctatgaaaaa     780
   gtgaaagatg atgtggataa gctaaaggag gcaaataaaa aattgaaatt ggaaaatggt     840
   ggtctggtga gggagaattt acgactgaag gctgaagttg ataacagatc acctcaaaag     900
   tttggaaggt ttgcagttgc tgctcttcag tccaaagtag aacagtatga gcgtgaaacc     960
   aatcgcctca agaaagccct ggaacgaagt gataagtata tagaggaact agaatctcaa    1020
   gttgcacagc taaaaaattc aagtgaagag aaagaagcta tgaattccat ttgccagaca    1080
   gcactttctg cagatggcaa ataa                                           1104
<210> 106
   <211> 367
   <212> PRT
   <213> Artificial Sequence
<220>
   <223> dominant negative form of OBI1
<400> 106
   Met Glu Gln Lys Leu Ile Ser Glu Glu Asp Leu Asn Glu Met Glu Gln
   1               5                   10                  15
   Lys Leu Ile Ser Glu Glu Asp Leu Asn Glu Met Glu Gln Lys Leu Ile
               20                  25                  30
   Ser Glu Glu Asp Leu Asn Glu Met Glu Gln Lys Leu Ile Ser Glu Glu
           35                  40                  45
   Asp Leu Asn Glu Met Glu Gln Lys Leu Ile Ser Glu Glu Asp Leu Asn
       50                  55                  60
   Glu Met Glu Ser Leu Gly Asp Leu Thr Met Ser Leu Ala Leu Lys Leu
   65                  70                  75                  80
   Ala Gly Leu Asp Ile Gly Ser Met Ala Gln Thr Val Gln Asn Val Thr
                   85                  90                  95
   Leu Ser Leu Thr Leu Pro Ile Thr Cys His Ile Cys Leu Gly Lys Val
               100                 105                 110
   Arg Gln Pro Val Ile Cys Ile Asn Asn His Val Phe Cys Ser Ile Cys
           115                 120                 125
   Ile Asp Leu Trp Leu Lys Asn Asn Ser Gln Cys Pro Ala Cys Arg Val
       130                 135                 140
   Pro Ile Thr Pro Glu Asn Pro Cys Lys Glu Ile Ile Gly Gly Thr Ser
   145                 150                 155                 160
   Glu Ser Glu Pro Met Leu Ser His Thr Val Arg Lys His Leu Arg Lys
                   165                 170                 175
   Thr Arg Leu Glu Leu Leu His Lys Glu Tyr Glu Asp Glu Ile Asp Cys
               180                 185                 190
   Leu Gln Lys Glu Val Glu Glu Leu Lys Ser Lys Asn Leu Ser Leu Glu
           195                 200                 205
   Ser Gln Ile Lys Thr Ile Leu Asp Pro Leu Thr Leu Val Gln Gly Asn
       210                 215                 220
   Gln Asn Glu Asp Lys His Leu Val Thr Asp Asn Pro Ser Lys Ile Asn
   225                 230                 235                 240
   Pro Glu Thr Val Ala Glu Trp Lys Lys Lys Leu Arg Thr Ala Asn Glu
                   245                 250                 255
   Ile Tyr Glu Lys Val Lys Asp Asp Val Asp Lys Leu Lys Glu Ala Asn
               260                 265                 270
   Lys Lys Leu Lys Leu Glu Asn Gly Gly Leu Val Arg Glu Asn Leu Arg
           275                 280                 285
   Leu Lys Ala Glu Val Asp Asn Arg Ser Pro Gln Lys Phe Gly Arg Phe
       290                 295                 300
   Ala Val Ala Ala Leu Gln Ser Lys Val Glu Gln Tyr Glu Arg Glu Thr
   305                 310                 315                 320
   Asn Arg Leu Lys Lys Ala Leu Glu Arg Ser Asp Lys Tyr Ile Glu Glu
                   325                 330                 335
   Leu Glu Ser Gln Val Ala Gln Leu Lys Asn Ser Ser Glu Glu Lys Glu
               340                 345                 350
   Ala Met Asn Ser Ile Cys Gln Thr Ala Leu Ser Ala Asp Gly Lys
           355                 360                 365
<210> 107
   <211> 4352
   <212> DNA
   <213> Artificial Sequence
<220>
   <223> vector pCS2 comprising Myc tag
<400> 107
   cgccattctg cctggggacg tcggagcaag cttgatttag gtgacactat agaatacaag      60
   ctacttgttc tttttgcagg atcccatcga tttaaagcta tggagcaaaa gctcatttct     120
   gaagaggact tgaatgaaat ggagcaaaag ctcatttctg aagaggactt gaatgaaatg     180
   gagcaaaagc tcatttctga agaggacttg aatgaaatgg agcaaaagct catttctgaa     240
   gaggacttga atgaaatgga gcaaaagctc atttctgaag aggacttgaa tgaaatggag     300
   agcttgggcg acctcaccat ggagcaaaag ctcatttctg aagaggactt gaattcaagg     360
   cctctcgagc ctctagaact atagtgagtc gtattacgta gatccagaca tgataagata     420
   cattgatgag tttggacaaa ccacaactag aatgcagtga aaaaaatgct ttatttgtga     480
   aatttgtgat gctattgctt tatttgtaac cattataagc tgcaataaac aagttaacaa     540
   caacaattgc attcatttta tgtttcaggt tcagggggag gtgtgggagg ttttttaatt     600
   cgcggccgcg gcgccaatgc attgggcccg gtacccagct tttgttccct ttagtgaggg     660
   ttaattgcgc gcttggcgta atcatggtca tagctgtttc ctgtgtgaaa ttgttatccg     720
   ctcacaattc cacacaacat acgagccgga agcataaagt gtaaagcctg gggtgcctaa     780
   tgagtgagct aactcacatt aattgcgttg cgctcactgc ccgctttcca gtcgggaaac     840
   ctgtcgtgcc agctgcatta atgaatcggc caacgcgcgg ggagaggcgg tttgcgtatt     900
   gggcgctctt ccgcttcctc gctcactgac tcgctgcgct cggtcgttcg gctgcggcga     960
   gcggtatcag ctcactcaaa ggcggtaata cggttatcca cagaatcagg ggataacgca    1020
   ggaaagaaca tgtgagcaaa aggccagcaa aaggccagga accgtaaaaa ggccgcgttg    1080
   ctggcgtttt tccataggct ccgcccccct gacgagcatc acaaaaatcg acgctcaagt    1140
   cagaggtggc gaaacccgac aggactataa agataccagg cgtttccccc tggaagctcc    1200
   ctcgtgcgct ctcctgttcc gaccctgccg cttaccggat acctgtccgc ctttctccct    1260
   tcgggaagcg tggcgctttc tcatagctca cgctgtaggt atctcagttc ggtgtaggtc    1320
   gttcgctcca agctgggctg tgtgcacgaa ccccccgttc agcccgaccg ctgcgcctta    1380
   tccggtaact atcgtcttga gtccaacccg gtaagacacg acttatcgcc actggcagca    1440
   gccactggta acaggattag cagagcgagg tatgtaggcg gtgctacaga gttcttgaag    1500
   tggtggccta actacggcta cactagaagg acagtatttg gtatctgcgc tctgctgaag    1560
   ccagttacct tcggaaaaag agttggtagc tcttgatccg gcaaacaaac caccgctggt 1620
   agcggtggtt tttttgtttg caagcagcag attacgcgca gaaaaaaagg atctcaagaa 1680
   gatcctttga tcttttctac ggggtctgac gctcagtgga acgaaaactc acgttaaggg 1740
   attttggtca tgagattatc aaaaaggatc ttcacctaga tccttttaaa ttaaaaatga 1800
   agttttaaat caatctaaag tatatatgag taaacttggt ctgacagtta ccaatgctta 1860
   atcagtgagg cacctatctc agcgatctgt ctatttcgtt catccatagt tgcctgactc 1920
   cccgtcgtgt agataactac gatacgggag ggcttaccat ctggccccag tgctgcaatg 1980
   ataccgcgag acccacgctc accggctcca gatttatcag caataaacca gccagccgga 2040
   agggccgagc gcagaagtgg tcctgcaact ttatccgcct ccatccagtc tattaattgt 2100
   tgccgggaag ctagagtaag tagttcgcca gttaatagtt tgcgcaacgt tgttgccatt 2160
   gctacaggca tcgtggtgtc acgctcgtcg tttggtatgg cttcattcag ctccggttcc 2220
   caacgatcaa ggcgagttac atgatccccc atgttgtgca aaaaagcggt tagctccttc 2280
   ggtcctccga tcgttgtcag aagtaagttg gccgcagtgt tatcactcat ggttatggca 2340
   gcactgcata attctcttac tgtcatgcca tccgtaagat gcttttctgt gactggtgag 2400
   tactcaacca agtcattctg agaatagtgt atgcggcgac cgagttgctc ttgcccggcg 2460
   tcaatacggg ataataccgc gccacatagc agaactttaa aagtgctcat cattggaaaa 2520
   cgttcttcgg ggcgaaaact ctcaaggatc ttaccgctgt tgagatccag ttcgatgtaa 2580
   cccactcgtg cacccaactg atcttcagca tcttttactt tcaccagcgt ttctgggtga 2640
   gcaaaaacag gaaggcaaaa tgccgcaaaa aagggaataa gggcgacacg gaaatgttga 2700
   atactcatac tcttcctttt tcaatattat tgaagcattt atcagggtta ttgtctcatg 2760
   agcggataca tatttgaatg tatttagaaa aataaacaaa taggggttcc gcgcacattt 2820
   ccccgaaaag tgccacctaa attgtaagcg ttaatatttt gttaaaattc gcgttaaatt 2880
   tttgttaaat cagctcattt tttaaccaat aggccgaaat cggcaaaatc ccttataaat 2940
   caaaagaata gaccgagata gggttgagtg ttgttccagt ttggaacaag agtccactat 3000
   taaagaacgt ggactccaac gtcaaagggc gaaaaaccgt ctatcagggc gatggcccac 3060
   tacgtgaacc atcaccctaa tcaagttttt tggggtcgag gtgccgtaaa gcactaaatc 3120
   ggaaccctaa agggagcccc cgatttagag cttgacgggg aaagccggcg aacgtggcga 3180
   gaaaggaagg gaagaaagcg aaaggagcgg gcgctagggc gctggcaagt gtagcggtca 3240
   cgctgcgcgt aaccaccaca cccgccgcgc ttaatgcgcc gctacagggc gcgtcccatt 3300
   cgccattcag gctgcgcaac tgttgggaag ggcgatcggt gcgggcctct tcgctattac 3360
   gccagtcgac catagccaat tcaatatggc gtatatggac tcatgccaat tcaatatggt 3420
   ggatctggac ctgtgccaat tcaatatggc gtatatggac tcgtgccaat tcaatatggt 3480
   ggatctggac cccagccaat tcaatatggc ggacttggca ccatgccaat tcaatatggc     3540
   ggacttggca ctgtgccaac tggggagggg tctacttggc acggtgccaa gtttgaggag     3600
   gggtcttggc cctgtgccaa gtccgccata ttgaattggc atggtgccaa taatggcggc     3660
   catattggct atatgccagg atcaatatat aggcaatatc caatatggcc ctatgccaat     3720
   atggctattg gccaggttca atactatgta ttggccctat gccatatagt attccatata     3780
   tgggttttcc tattgacgta gatagcccct cccaatgggc ggtcccatat accatatatg     3840
   gggcttccta ataccgccca tagccactcc cccattgacg tcaatggtct ctatatatgg     3900
   tctttcctat tgacgtcata tgggcggtcc tattgacgta tatggcgcct cccccattga     3960
   cgtcaattac ggtaaatggc ccgcctggct caatgcccat tgacgtcaat aggaccaccc     4020
   accattgacg tcaatgggat ggctcattgc ccattcatat ccgttctcac gccccctatt     4080
   gacgtcaatg acggtaaatg gcccacttgg cagtacatca atatctatta atagtaactt     4140
   ggcaagtaca ttactattgg aaggacgcca gggtacattg gcagtactcc cattgacgtc     4200
   aatggcggta aatggcccgc gatggctgcc aagtacatcc ccattgacgt caatggggag     4260
   gggcaatgac gcaaatgggc gttccattga cgtaaatggg cggtaggcgt gcctaatggg     4320
   aggtctatat aagcaatgct cgtttaggga ac                                   4352
<210> 108
   <211> 52
   <212> DNA
   <213> Artificial Sequence
<220>
   <223> derived from NES of PKI
<400> 108
   catgtctctg gctctcaaac tggctggact ggacatcgga tccatggtct tg 52
<210> 109
   <211> 52
   <212> DNA
   <213> Artificial Sequence
<220>
   <223> derived from NES of PKI
<400> 109
   aattcaagac catggatccg atgtccagtc cagccagttt gagagccaga ga 52

## Description

The present invention relates to a protein involved in DNA replication as drug. The invention also relates to compounds inhibiting the expression or the activity of said protein. Chromosomal DNA replication in eukaryotic cells is highly complicated and sophisticatedly regulated. Owing to its large size, a typical eukaryotic genome contains hundreds to tens of thousands of initiation sites called DNA replication origins where DNA synthesis takes place. Multiple initiation sites remove the constraint of a genome size because only a certain amount of DNA can be replicated from a single origin in a limited time. The activation of these multiple origins must be coordinated so that each segment of chromosomal DNA is precisely duplicated only once per cell cycle. Although DNA replication is a vital process for cell growth and its mechanism is highly conserved, recent studies also reveal significant diversity in origin structure, assembly of pre-replication complex (pre-RC) and regulation of replication initiation along evolutionary lines.

It is crucial to maintain genomic integrity in eukaryotic cells. During DNA replication, errors in pre-RC assembly often result in substantial changes in the amount of genetic material, which will cause cell death, or transform cells to become malignant tumors.

The ORC proteins
The first origin recognition complex (ORC) proteins to be identified were purified from cell extracts as a heterohexameric complex that specifically binds to origins of DNA replication and the subunits were named Orc1 through Orc6. Subsequently, orthologs of *ORC1-ORC5* were identified in organisms as diverse as *Drosophila melanogaster, Arabidopsis thaliana* and *Homo sapiens,* strongly suggesting that these genes are likely to exist in all eukaryotes.

The ORC needs to be in an ATP-bound state so that it can interact with Cdc6p creating events that will produce the unwinding of the double stranded DNA which will promote the hydrolysis of the ATP by the ORC. Once the ORC is bound to the origin, the complex is retained in an ATP-bound state and the ATP hydrolysis is reserved for a downstream step in initiation. When the ORC binds to DNA at replication origin sites in an ATP-dependent manner it then serves as a scaffold for the assembly of other key initiation factors of the pre-replicative complex (pre-RC) which include Cdc6, Cdt1, and Mcm proteins.

Recently, mutations in genes encoding ORC1, ORC4, ORC6, CDT1, and CDC6 were identified in patients displaying Seckel syndrome (SS) and/or Meier-Gorlin syndrome (MGS) [Bicknell LS et al. (2011) Nat Genet 43: 356-359*;* Bicknell LS, et al. (2011) Nat Genet 43: 350-355*;* Guernsey DL, et al. (2011) Nat Genet 43: 360-364*].*

Seckel syndrome, Majewski osteodysplastic primordial dwarfism (MOPD) type II and Meier-Gorlin syndrome represent three disorders which share overlapping clinical features that include pronounced microcephaly, severe intrauterine growth retardation and post natal growth delay.

However, even if these mutations are associated with some of patients afflicted by these syndromes, some orphan individuals do not harbor any mutations or expression abnormality in the genes encoding ORC1, ORC4, ORC6, CDT1, and CDC6.

Therefore, there is a need to identify other genes in which mutations could be responsible of the syndromes developed by the above orphan individuals.

One aim of the invention is to identify such gene.

Another aim of the invention is to identify proteins that could be responsible of the above syndromes, or other syndrome linked to defects in unknown protein controlling DNA replication.

Still another aim of the invention is to provide a new efficient new medicine intended for the treatment of the above syndromes.

The disclosure relates to a composition comprising at least one of the following compounds:
- a protein consisting of an amino acid sequence as set forth in SEQ ID NO: 1, or a fragment thereof provided that said fragment retains properties to interact with the origin recognition complex, or eventually retains oncogenic properties or dimerization properties,
- a protein derived from the protein as set forth in SEQ ID :1 by substitution, deletion, or insertion of at least one nucleotide provided that this protein retains ubiquitin ligase activity and/or participate to DNA replication,
- a nucleic acid molecule coding for said proteins or said fragments,
- a compound affecting, i.e. inhibiting or activating, the expression or the activity of said protein,
a salt or a solvate thereof,

in association with a pharmaceutically acceptable carrier.

The disclosure is based on the characterization by the inventors of a new protein that physically interacts with the ORC protein complex and that is involved in DNA replication. By a purification process of the ORC protein complex, the inventors have identified a new ORC protein complex partner which has been identified to specifically interact with both ORC1 and LRWD1 proteins. The process for purifying the protein is described hereafter. The ORC1 protein is a component of the origin recognition complex (ORC) that binds origins of replication. The DNA-binding of ORC1 is ATP-dependent.

The LRW1 protein is an associated component of the ORC required to recruit and stabilize the ORC complex to chromatin during G1 to establish pre-replication complex (preRC) and to heterochromatic sites in post-replicated cells. This protein binds a combination of DNA and histone methylation repressive marks on heterochromatin: it binds histone H3 and H4 trimethylation marks H3K9me3, H3K27me3 and H4K20me3 in a cooperative manner with DNA methylation.

The inventors have identified that the protein disclosed in databases as the RNF219 protein specifically interacts with only the above proteins (ORC1 and LRWD1) and is binding the ORC complex.

In view of the characterization of the interaction, RNF219 is now named: ORC Ubiquitin-ligase 1 or OBI1).

The human OBI1 protein consists of the amino acid sequence as set forth in SEQ ID NO: 1.

The composition according to the disclosure can therefore contain, as active substance, the OBI1 protein as set forth in SEQ ID NO: 1.

The disclosure also encompasses a protein derived from the protein as set forth in SEQ ID :1, said protein consisting of the amino acid sequence as set forth in SEQ ID NO: 1 and having at least one substitution and/or one deletion and/or one insertion of at least one nucleotide provided that said protein retains ubiquitin ligase activity and/or participates to DNA replication.

The disclosure encompasses also a composition as defined above, comprising as active substance a protein having at least 20% identity with SEQ ID NO: 1, in particular a protein having at least 20% identity with SEQ ID NO: 1 and containing the amino acid sequence SEQ ID NO: 28. SEQ ID NO: 28 represents the amino acid sequence of the zinc finger/ring finger of the OBI1 proteins.

Thus, in the disclosure, a protein having at least 20 % identity with SEQ ID NO: 1 defines a proteins having 20% identity, 21% identity, 22% identity, 23% identity, 24% identity, 25% identity, 26% identity, 27% identity, 28% identity, 29% identity, 30% identity, 31% identity, 32% identity, 33% identity, 34% identity, 35% identity, 36% identity, 37% identity, 38% identity, 39% identity, 40% identity, 41% identity , 42% identity, 43% identity, 44% identity, 45% identity, 46% identity, 47% identity, 48% identity, 49% identity, 50% identity, 51% identity, 52% identity, 53% identity, 54% identity, 55% identity, 56% identity, 57% identity, 58% identity, 59% identity, 60% identity, 61% identity, 62% identity, 63% identity, 64% identity, 65% identity, 66% identity, 67% identity, 68% identity, 69% identity, 70% identity, 71% identity, 72% identity, 73% identity, 74% identity, 75% identity, 76% identity, 77% identity, 78% identity, 79% identity, 80% identity, 81% identity, 82% identity, 83% identity, 84% identity, 85% identity, 86% identity, 87% identity, 88% identity, 89% identity, 90% identity, 91% identity, 92% identity, 93% identity, 94% identity, 95% identity, 96% identity, 97% identity, 98% identity or 99% identity with SEQ ID NO: 1. Such proteins are, for instance, the proteins consisting of the amino acid sequence as set forth in SEQ ID NO : 2, SEQ ID NO : 3, SEQ ID NO : 4, SEQ ID NO : 5, SEQ ID NO : 6, SEQ ID NO : 7, SEQ ID NO : 8, SEQ ID NO : 9, SEQ ID NO : 10, SEQ ID NO : 11, SEQ ID NO : 12, SEQ ID NO : 13, SEQ ID NO : 14, SEQ ID NO : 15, SEQ ID NO : 16, SEQ ID NO : 17, SEQ ID NO : 18, SEQ ID NO : 19, SEQ ID NO : 20, SEQ ID NO : 21, SEQ ID NO : 22, SEQ ID NO : 23, SEQ ID NO : 24, SEQ ID NO : 25, SEQ ID NO : 26 or SEQ ID NO : 27. All these proteins represent the OBI1 proteins of different eukaryotic species. The above lists are not limitative, and the skilled person is able to determine any new Obi1 protein.

The disclosure also encompasses a composition comprising fragments of the above proteins, fragments retaining at least one of the function of the protein: i.e. said fragments retaining at least an oncogenic function, an ubiquitin ligase function or a dimerization function.

The advantageous fragments contained in the composition according to the disclosure are :
- the peptide consisting of the amino acid sequence SEQ ID NO: 59,
- the peptide consisting of the amino acid sequence SEQ ID NO: 29,
- the peptide consisting of the amino acid sequence SEQ ID NO: 30,
- the peptide consisting of the amino acid sequence SEQ ID NO: 60,
- the peptide consisting of the amino acid sequence SEQ ID NO: 61,
- the peptide consisting of the amino acid sequence SEQ ID NO: 62,
- the peptide consisting of the amino acid sequence SEQ ID NO: 63, and
- the peptide consisting of the amino acid sequence SEQ ID NO: 100.

All the proteins and fragments as defined above can also be tagged with specific tags, in order to enhance and/or facilitate their purification. The tags can be located at the N-terminus part or at the C-terminus part of the proteins or fragments. In some advantageous embodiments, the proteins can be tagged at both their N- and C-terminus parts, such protein being advantageously tagged with two different tags.

The skilled person is able, with his common knowledge, to determine the most appropriated tags. The following list, which not intend to restrict the scope of the invention, represents the common tags that can be used: GFP, EGFP, YFP, CFP, dsRed, Myc tag, E tag, FLAG tag, Glu-Glu tag, GST tag, HA tag, His tag, in particular 6xHIS, HSV tag, luciferase, MBP, protein C tag, S tag, T7 tag, V5 tag, VSV-g tag, avidin/streptavidin/strep tag, thioredoxin, His-patch thioredoxin, β-galactosidase, chloramphenicol acetyltransferase, cellulose binding domains (CBDs), chitin binding domain, staphylococcal protein A, streptococcal protein G, neo, hyg, pac, zeo, gpt, ble, dhfr, hpt and npt II.

It is also possible to use in the protein according to the invention nuclear sequences such as Nuclear Export Sequence (NES) or Nuclear Localization Signal (NLS), in order to specifically address respectively protein comprising them to the cytoplasm or the nucleus. The disclosure also encompasses a composition comprising nucleic acid molecules coding for the proteins or the fragments as defined above.

In the invention the nucleic acid coding for the protein consisting of SEQ ID NO : 1 consists of the mRNA nucleic sequence as set forth in SEQ ID NO : 31.

The composition as defined above may also contain inhibitors of the expression or of the activity of the above mentioned protein.

In the invention salt(s), as used herein, means those salts of compounds of the invention that are safe and effective for use in mammals and that possess the desired biological activity. Pharmaceutically acceptable salts include salts of acidic or basic groups present in compounds of the invention. Pharmaceutically acceptable acid addition salts include, but are not limited to, hydrochloride, hydrobromide, hydroiodide, nitrate, sulfate, bisulfate, phosphate, acid phosphate, isonicotinate, acetate, lactate, salicylate, citrate, tartrate, oxalate, maleate, pantothenate, bitartrate, ascorbate, succinate, maleate, gentisinate, fumarate, gluconate, glucaronate, saccharate, formate, benzoate, glutamate, methanesulfonate, ethanesulfonate, benzensulfonate, p-toluenesulfonate, pamoate (i.e., 1,1'-methylene-bis-(2-hydroxy-3-naphthoate)), mesylate, besylate, tosylate and tartrate salts. Suitable base salts include, but are not limited to, aluminum, calcium, lithium, magnesium, potassium, sodium, zinc, and diethanolamine salts.

Advantageously, the disclosure relates to a composition as defined above, wherein said nucleic acid molecule comprises the nucleic acid sequence SEQ ID NO : 31, or a nucleic acid sequence having at least 20% identity, i.e. 20% identity, 21% identity, 22% identity, 23% identity, 24% identity, 25% identity, 26% identity, 27% identity, 28% identity, 29% identity, 30% identity, 31% identity, 32% identity, 33% identity, 34% identity, 35% identity, 36% identity, 37% identity, 38% identity, 39% identity, 40% identity, 41% identity , 42% identity, 43% identity, 44% identity, 45% identity, 46% identity, 47% identity, 48% identity, 49% identity, 50% identity, 51% identity, 52% identity, 53% identity, 54% identity, 55% identity, 56% identity, 57% identity, 58% identity, 59% identity, 60% identity, 61% identity, 62% identity, 63% identity, 64% identity, 65% identity, 66% identity, 67% identity, 68% identity, 69% identity, 70% identity, 71% identity, 72% identity, 73% identity, 74% identity, 75% identity, 76% identity, 77% identity, 78% identity, 79% identity, 80% identity, 81% identity, 82% identity, 83% identity, 84% identity, 85% identity, 86% identity, 87% identity, 88% identity, 89% identity, 90% identity, 91% identity, 92% identity, 93% identity, 94% identity, 95% identity, 96% identity, 97% identity, 98% identity or 99% identity, with the nucleic acid sequence SEQ ID NO: 31, provided that he protein coded by said nucleic acid sequences retains ubiquitin ligase activity, and/or participates to DNA replication. In one another advantageous embodiment, the disclosure relates to a composition previously defined, wherein said nucleic acid molecule is contained in a vector, said vector comprising means allowing the expression of said nucleic acid molecule.

In the invention the vector is advantageously an expression vector which is a plasmid or virus designed for protein expression in cells. The vector is used to introduce a specific gene into a target cell, and can command the cell mechanism for protein synthesis to produce the protein encoded by the gene.

The vector is engineered to contain regulatory sequences that act as enhancer and promoter regions and lead to efficient transcription of the gene carried on the expression vector.

An expression vector have elements necessary for protein expression that include a strong promoter, the correct translation initiation sequence such as a ribosomal binding site and start codon, a strong termination codon, and a transcription termination sequence. There are differences in the machinery for protein synthesis between prokaryotes and eukaryotes, therefore the expression vectors must have the elements for expression that is appropriate for the chosen host. For example, prokaryotes expression vectors would have a Shine-Dalgarno sequence at its translation initiation site for the binding of ribosomes, while eukaryotes expression vectors contains the Kozak consensus sequence.

Vector may also comprise replication origin allowing the replication of the vector in bacteria. Furthermore, the vector as defined above may contain at least one selection marker allowing selection of transformed cells. These markers are well known in the art and the skilled person is able to determine which marker is more appropriate for a determined experiment.

In still another advantageous embodiment, the disclosure relates to a composition as previously defined wherein said compound inhibiting the expression of the protein consisting of an amino acid sequence as set forth in SEQ ID NO: 1, or the protein derived from the protein as set forth in SEQ ID :1 by substitution, deletion, or insertion of at least one nucleotide provided that this protein retains ubiquitin ligase activity and/or participate to DNA replication, in particular a protein having at least 20% identity with SEQ ID NO: 1 and containing the amino acid sequence SEQ ID NO: 28, as defined above, is chosen among a siRNA, miRNA, shRNA, RNA antisense, DNA antisense, antibodies and a fragment of the protein consisting of an amino acid sequence as set forth in SEQ ID NO: 1,said fragment comprising the amino acid sequence SEQ ID NO: 29, SEQ ID NO 30, SEQ ID NO: 59, SEQ ID NO: 60, SEQ ID NO: 61, SEQ ID NO: 62, SEQ ID NO: 63, SEQ ID NO: 100 or SEQ ID NO: 106.

siRNA, shRNA or miRNA according to the invention inhibit OBI1 gene expression by RNA interference mechanism.

RNA interference is a highly conserved biological mechanism inducing specific repression of genes by specifically destroying mRNA, or inhibition translation of said RNA.

In 1998, Fire et al [Fire et al., Nature. 1998 Feb 19;391(6669):806-11] demonstrated that a double-stranded is produced in cells by a Class III RNA endonuclease, the DICER complex, and small inhibiting double-stranded RNA (siRNA) of about 19 to 28 nucleotides are produced.

Incorporated to the enzymatic « RNA- Induced Silencing Complex » RISC complex, said siRNA are deshybridized and can therefore hybridize with the complementary sequence contained in mRNA. The "captured" mRNA is then destroyed, or its translation by ribosomal particles is inhibited.

Small hairpin RiboNucleic Acid- shRNA are double-stranded molecules comprising both the sense and the antisense strand of a siRNA, said sense and antisense strands being linked by a linker. These molecules form a hairpin, and the linker is eliminated to allow the liberation of a siRNA.

Micro RNA - miRNA are encoded by eukaryotic nuclear DNA and act as si- or mi-RNA. The invention also relates to the composition as defined above, for its use as medicine. The invention further relates to the composition as defined above, for its use for the treatment or the prevention of a human or animal pathology linked to a dysfunction of the OBI1 gene or to the DNA replication.

In other words, the invention relates to a composition comprising at least one of the following compounds:
- the protein consisting of the amino acid sequence as set forth in SEQ ID NO: 1,
- a protein derived from the protein as set forth in SEQ ID NO:1 by substitution of at least one nucleotide provided that said protein retains ubiquitin ligase activity and/or participates to DNA replication, said protein consisting of the amino acid sequence as set forth in SEQ ID NO : 2-27,
- a nucleic acid molecule coding for said proteins or said fragments,
- a compound affecting the expression of said protein, said compound being a small interfering molecule comprising the nucleic acid sequence as set forth in SEQ ID NO : 32 to 55,
- a compound affecting the activity of said protein, said compound being
   ∘ a fragment of said protein provided that said fragment retains properties to interact with the origin recognition complex, said fragment consisting of the amino acid sequence SEQ ID NO: 59, SEQ ID NO: 29, SEQ ID NO: 30, SEQ ID NO: 60, SEQ ID NO: 61, SEQ ID NO: 62, SEQ ID NO: 63,SEQ ID NO: 100 or SEQ ID NO : 106, or
   ∘ a nucleic acid molecule coding for said fragment
   a salt or a solvate thereof,
   possibly in association with a pharmaceutically acceptable carrier,
   as defined above
   for its use for the treatment or the prevention of a human or animal pathology linked to DNA replication.

The Disclosure encompasses both :
- the composition as defined above for its use for the treatment or the prevention of a human or animal pathology linked to a dysfunction of the OBI1 gene, or OBI1 protein, and
- the composition according to the invention as defined above for its use for the treatment or the prevention of a human or animal pathology linked to DNA replication.

The disclosure relates also to a method for treating human or animal pathology linked to a dysfunction of the OBI1 gene or linked to the DNA replication, said method comprising the administration of an effective amount, in a patient in a need thereof, of at least one of the following compounds:
- a protein consisting of an amino acid sequence as set forth in SEQ ID NO: 1, or a fragment thereof provided that said fragment retains properties to interact with the origin recognition complex, or eventually retains oncogenic properties or dimerization properties,
- a protein derived from the protein as set forth in SEQ ID :1 by substitution, deletion, or insertion of at least one nucleotide provided that this protein retains ubiquitin ligase activity and/or participate to DNA replication,
- a nucleic acid molecule coding for said proteins or said fragments, and
- a compound inhibiting the expression or the activity of said protein,
or a salt or a solvate thereof.

Advantageously, the invention relates to the composition for its use according to the previous definition, wherein said pathology is a neoplastic disease or cancer, and wherein said composition comprises a compound inhibiting the expression of said nucleic acid molecule or the activity of said protein.

The compounds inhibiting the expression of the protein, i.e. the Obi1 protein, can be for instance the molecule inhibiting the expression by RNA interference. Such compounds are for instance those described hereafter.

The compounds inhibiting the activity of the protein, i.e. the Obi1 protein, are for instance compounds inhibiting the ubiquitin ligase activity of said protein.

Examples of such compounds are those discloses in Ceccarelli et al 2011 Cell 145 :1075-87.

In one another advantageous embodiment, the invention relates to the composition for its use previously defined, wherein said pathology is a pathology linked to DNA replication is chosen among Meier-Gorlin syndrome, Seckel syndrome and Majewski osteodysplastic primordial dwarfism and wherein said composition comprises:
- the protein consisting of the amino acid sequence as set forth in SEQ ID NO: 1,
- a protein derived from the protein as set forth in SEQ ID NO:1 by substitution of at least one nucleotide provided that said protein retains ubiquitin ligase activity and/or participates to DNA replication, said protein consisting of the amino acid sequence as set forth in SEQ ID NO : 2- 27, or
- a nucleic acid molecule coding for said proteins.

The invention also relates to a nucleic acid molecule inhibiting the expression of the OBI1 gene, as defined above by RNA interference, said a nucleic acid molecule comprising or consisting essentially of, or consisting of one of the following sequences:
5'- GTAGAAGTAATGTTAGATG -3' (SEQ ID NO : 32),
5'- CCAAAGGTTCTCTAACTAA -3' (SEQ ID NO : 33),
5'- GGATTTGGATGGGTTATCA -3' (SEQ ID NO : 34),
5'- GAACGAAGTGATAAGTATA -3' (SEQ ID NO : 35),
5'- GUAGAAGUAAUGUUAGAUG -3' (SEQ ID NO : 36),
5'- CCAAAGGUUCUCUAACUAA -3' (SEQ ID NO : 37),
5'- GGAUUUGGAUGGGUUAUCA -3' (SEQ ID NO : 38),
5'- GAACGAAGUGAUAAGUAUA -3' (SEQ ID NO : 39),
5'- CATCTAACATTACTTCTAC -3' (SEQ ID NO : 40),
5'- TTAGTTAGAGAACCTTTGG -3' (SEQ ID NO : 41),
5'- TGATAACCCATCCAAATCC -3' (SEQ ID NO : 42),
5'- TATACTTATCACTTCGTTC -3' (SEQ ID NO : 43),
5'- CAUCUAACAUUACUUCUAC -3' (SEQ ID NO : 44),
5'- UUAGUUAGAGAACCUUUGG -3' (SEQ ID NO : 45),
5'- UGAUAACCCAUCCAAAUCC -3' (SEQ ID NO : 46), and
5'- UAUACUUAUCACUUCGUUC -3' (SEQ ID NO : 47).

The invention also relates to a composition, in particular for its use as defined above, comprising at least one of the nucleic acid molecules as defined above.

The complementary sequence of the nucleic acid molecule comprising or consisting of SEQ ID NO: 32 comprises or consists of the sequence SEQ ID NO : 40. The complementary sequence of the nucleic acid molecule comprising or consisting of SEQ ID NO: 33 comprises or consists of the sequence SEQ ID NO : 41. The complementary sequence of the nucleic acid molecule comprising or consisting of SEQ ID NO: 34 comprises or consists of the sequence SEQ ID NO : 42. The complementary sequence of the nucleic acid molecule comprising or consisting of SEQ ID NO: 35 comprises or consists of the sequence SEQ ID NO : 43. The complementary sequence of the nucleic acid molecule comprising or consisting of SEQ ID NO: 36 comprises or consists of the sequence SEQ ID NO : 44. The complementary sequence of the nucleic acid molecule comprising or consisting of SEQ ID NO: 37 comprises or consists of the sequence SEQ ID NO : 45. The complementary sequence of the nucleic acid molecule comprising or consisting of SEQ ID NO: 38 comprises or consists of the sequence SEQ ID NO : 46. The complementary sequence of the nucleic acid molecule comprising or consisting of SEQ ID NO: 39 comprises or consists of the sequence SEQ ID NO : 47.

Thus, most advantageous siRNA according to the invention are one of the following siRNA:
- siRNA comprising a sense strand comprising or consisting in SEQ ID NO: 36 and its complementary sequence, or antisense strand, comprising or consisting of SEQ ID NO : 44,
- siRNA comprising a sense strand comprising or consisting in SEQ ID NO: 37 and its complementary sequence, or antisense strand, comprising or consisting of SEQ ID NO : 45,
- siRNA comprising a sense strand comprising or consisting in SEQ ID NO: 38 and its complementary sequence, or antisense strand, comprising or consisting of SEQ ID NO : 46, and
- siRNA comprising a sense strand comprising or consisting in SEQ ID NO: 39 and its complementary sequence, or antisense strand, comprising or consisting of SEQ ID NO : 47.

The above siRNA can also be modified by addition of compounds stabilizing siRNA structure.

For instance, the above siRNA may contain, in their 3'- end, a dinucleotide: a dithymidine (TT). Therefore, the siRNA according to the invention comprise one of the following sequences:
5'- GUAGAAGUAAUGUUAGAUGTT -3' (SEQ ID NO : 48),
5'- CCAAAGGUUCUCUAACUAATT -3' (SEQ ID NO : 49),
5'- GGAUUUGGAUGGGUUAUCATT -3' (SEQ ID NO : 50),
5'- GAACGAAGUGAUAAGUAUATT -3' (SEQ ID NO : 51),
5'- CAUCUAACAUUACUUCUACTT -3' (SEQ ID NO : 52),
5'- UUAGUUAGAGAACCUUUGGTT -3' (SEQ ID NO : 53),
5'- UGAUAACCCAUCCAAAUCCTT -3' (SEQ ID NO : 54), and
5'- UAUACUUAUCACUUCGUUCTT -3' (SEQ ID NO : 55).

Thus, most advantageous siRNA according to the invention are one of the following siRNA:
- siRNA comprising a sense strand comprising or consisting in SEQ ID NO: 48 and its complementary sequence, or antisense strand, comprising or consisting of SEQ ID NO : 52,
- siRNA comprising a sense strand comprising or consisting in SEQ ID NO: 49 and its complementary sequence, or antisense strand, comprising or consisting of SEQ ID NO : 53,
- siRNA comprising a sense strand comprising or consisting in SEQ ID NO: 50 and its complementary sequence, or antisense strand, comprising or consisting of SEQ ID NO : 54, and
- siRNA comprising a sense strand comprising or consisting in SEQ ID NO: 51 and its complementary sequence, or antisense strand, comprising or consisting of SEQ ID NO : 55.

In one another advantageous embodiment, the invention relates to a product used as mentioned above, wherein said shRNA comprises or consists of one of the following nucleic acid molecules:
- a nucleic acid molecule comprising or being constituted by the sequence SEQ ID NO : 3 followed by the sequence SEQ ID NO: 48, the 3'-end of SEQ ID NO:3 being linked to the 5'-end of SEQ ID NO: 52 by a linker,
- a nucleic acid molecule comprising or being constituted by the sequence SEQ ID NO : 5 followed by the sequence SEQ ID NO: 49, the 3'-end of SEQ ID NO:5 being linked to the 5'-end of SEQ ID NO: 53 by a linker,
- a nucleic acid molecule comprising or being constituted by the sequence SEQ ID NO : 7 followed by the sequence SEQ ID NO: 50, the 3'-end of SEQ ID NO:7 being linked to the 5'-end of SEQ ID NO: 54 by a linker, and
- a nucleic acid molecule comprising or being constituted by the sequence SEQ ID NO : 9 followed by the sequence SEQ ID NO: 51, the 3'-end of SEQ ID NO:9 being linked to the 5'-end of SEQ ID NO: 55 by a linker.

The linker according to the invention can be chosen among the following linkers :
1) UUCAAGAGA (Brummelkamp, T.R., 2002 Science.296(5567):550-3),
2) AAGUUCUCU (Promega),
3) UUUGUGUAG (Scherr, M., Curr Med Chem. 2003 Feb;10(3):245-56.),
4) CUUCCUGUCA (SEQ ID NO : 56) (Schwarz D. S., 2003 Cell. 115(2):199-208.), and
5) CUCGAG.

The compounds inhibiting the activity of the protein, i.e. the Obi1 protein, can be, for instance, the protein fragments interfering with the activity of the wild type Obi1 protein. Such fragments are for instance those as set forth in SEQ ID NO: 29, SEQ ID NO 30, SEQ ID NO: 59, SEQ ID NO: 60, SEQ ID NO: 61, SEQ ID NO: 62, SEQ ID NO: 63, SEQ ID NO: 100 or SEQ ID NO: 106, in particular the fragments as set forth in SEQ ID NO: 29, SEQ ID NO: 61, SEQ ID NO: 100 and SEQ ID NO: 106.

The invention also relates to an inhibitor of the activity of the protein consisting of an amino acid sequence as set forth in SEQ ID NO: 1, or a protein derived from the protein as set forth in SEQ ID :1 by substitution, deletion, or insertion of at least one nucleotide provided that said protein retains ubiquitin ligase activity and/or participates to DNA replication, as defined above, characterized in that it comprises the amino acid sequence : SEQ ID NO: 29, SEQ ID NO 30, SEQ ID NO: 59, SEQ ID NO: 60, SEQ ID NO: 61, SEQ ID NO: 62, SEQ ID NO: 63, SEQ ID NO: 100 or SEQ ID NO: 106.

The above fragments may be used as purified proteins, in particular in association with an appropriate salt, or in a form of nucleic acid coding said fragment, possibly contained in a vector (viral vector), possibly in the vector comprising a Myc tag, and possibly derived from the vector as set forth in SEQ ID NO: 107.

These proteins, or fragments, and their nucleic acids are artificial, non naturally occurring, or purified from their natural context.

The invention further relates to the use of the protein consisting of an amino acid sequence as set forth in SEQ ID NO: 1, or protein derived from the protein as set forth in SEQ ID :1 by substitution, deletion, or insertion of at least one nucleotide provided that said protein retains ubiquitin ligase activity and/or participates to DNA replication, said protein consisting of the amino acid sequence as set forth in SEQ ID NO : 2- 27,as Lys6 ubiquitine ligase enzyme.

The disclosure also relates to a method for ubiquitinate at least one protein, in particular ORC3 and ORC5 subunits of the ORC complex, comprising a step of contacting said protein with the protein consisting of an amino acid sequence as set forth in SEQ ID NO: 1, protein derived from the protein as set forth in SEQ ID :1 by substitution, deletion, or insertion of at least one nucleotide provided that said protein retains ubiquitin ligase activity and/or participates to DNA replication, or a fragment thereof as defined above, said protein comprising a chain of Lys6-linked poly ubiquitin.

The RING ligases that have been characterized to date yield different patterns of substrate ubiquitination.

RING domains recruit E2 enzymes that are thioesterified with ubiquitin and also activate E2 to discharge its ubiquitin cargo to a substrate.

A number of RING ligases primarily monoubiquitinate or oligoubiquitinate their substrates. Ubiquitination may terminate after the transfer of only one or a few ubiquitins because the substrate simply falls off the E3 before the reaction can proceed further. However, oligoubiquitination is specified by an internal ubiquitin-binding domain that caps the growing chain and prevents its further extension. Yet other E3-E2 pairs transfer multiple ubiquitins to substrate, but in the form of several monoubiquitins rather than a polyubiquitin chain.

Multiple monoubiquitination can directly promote degradation of cyclin B in vitro but most often probably does not function as a proteasome-targeting signal in vivo, as exemplified by modification and lysosomal targeting of the EGF receptor by c-Cbl. Other RING-E2 complexes transfer multiple ubiquitins to substrate preferentially in the form of a polyubiquitin chain.

RING-E2 complexes can assemble chains linked exclusively via the Lys6, Lys48, and Lys63 residues of ubiquitin. Ultimately, all seven lysine residues of ubiquitin (Lys6, Lys11, Lys27, Lys29, Lys33, Lys48, Lys63) can form chain linkages in vivo. Thus, the number of topological isomers is potentially huge if chains of mixed linkage are produced. These modifications have a range of biological effects, from proteasome-dependent proteolysis (Lys48- and Lys11-linked polyubiquitin) to posttranslational control of protein function, structure, assembly, and/or localization (Lys63 and other linkages).

The inventors have demonstrated that OBI1 protein is an E3 ubiquitin ligase that essentially forms Lys6 polyubiquitin chains (see example section).

The disclosure also relates to the use of an inhibitor of the protein consisting of an amino acid sequence as set forth in SEQ ID NO: 1, protein derived from the protein as set forth in SEQ ID :1 by substitution, deletion, or insertion of at least one nucleotide provided that said protein retains ubiquitin ligase activity and/or participates to DNA replication, as defined above, for inhibiting cell proliferation.

The inventors have demonstrated that OBI1 gene is an oncogene, as set forth in the example section.

Therefore, the inventors propose, and have tested, inhibitors of the expression, and/or activity of OBI1 in order to abolish its oncogenic function and in order to inhibit deregulated cell proliferation.

The disclosure also encompasses a composition comprising at least one inhibitor of the protein consisting of an amino acid sequence as set forth in SEQ ID NO: 1, or protein derived from the protein as set forth in SEQ ID :1 by substitution, deletion, or insertion of at least one nucleotide provided that said protein retains ubiquitin ligase activity and/or participates to DNA replication, as defined above, for its use for treating cancer.

The disclosure relates also to a method for treating cancer comprising the administration of an effective amount, to a patient in a need thereof, of at least one inhibitor of the protein consisting of an amino acid sequence as set forth in SEQ ID NO: 1, or protein derived from the protein as set forth in SEQ ID :1 by substitution, deletion, or insertion of at least one nucleotide provided that said protein retains ubiquitin ligase activity and/or participates to DNA replication, as defined above.

The invention further relates to a process for purifying a protein consisting of the amino acid sequence SEQ ID NO: 1, or protein derived from the protein as set forth in SEQ ID :1 by substitution, deletion, or insertion of at least one nucleotide provided that said protein retains ubiquitin ligase activity and/or participates to DNA replication, said protein consisting of the amino acid sequence as set forth in SEQ ID NO : 2- 27, said process comprising :
- a step of purifying either the ORC1 protein or the LRWD1 protein, and
- a step of isolating the OBI1 protein.

In the invention, the ORC1 and LRWD1 proteins consist respectively of the amino acid sequence as set forth in SEQ ID NO: 57 and 58.

The invention further relate to a process for purifying a protein consisting of the amino acid sequence SEQ ID NO: 1, or protein derived from the protein as set forth in SEQ ID :1 by substitution, deletion, or insertion of at least one nucleotide provided that said protein retains ubiquitin ligase activity and/or participates to DNA replication, as defined above, said process comprising the steps of:
- immunoprecipitating with a first specific antibody either the ORC1 protein or the LRWD1 protein,
- eluting the purified proteins with a peptide which is specific of said first antibody,
- immunoprecipitating with a second antibody specific antibody either the ORC1 protein or the LRWD1 protein,
- eluting the purified proteins with a peptide which is specific of said second antibody, and
optionally further purifying the protein consisting of the amino acid sequence SEQ ID NO: 1, or protein derived from the protein as set forth in SEQ ID :1 by substitution, deletion, or insertion of at least one nucleotide provided that said protein retains ubiquitin ligase activity and/or participates to DNA replication, with a third specific antibody.

In one other advantageous embodiment, the invention relates to a process for purifying a protein consisting of the amino acid sequence SEQ ID NO: 1, or protein derived from the protein as set forth in SEQ ID :1 by substitution, deletion, or insertion of at least one nucleotide provided that said protein retains ubiquitin ligase activity and/or participates to DNA replication, said process comprising the steps of:
- purifying a tagged ORC1 protein or a tagged LRWD1 protein, said tagged ORC and LRWD1 proteins being tagged with two different tags, said purification step being carried out by using s first tag,
- eluting the purified proteins with the free tag used to purify said proteins,
- purifying a tagged ORC1 protein or a tagged LRWD1 protein obtained from the first step by using the second tag,
- eluting the purified proteins with the free tag used to purify said proteins,
- isolating the OBI1 protein.

The above method is well known in the art as TAP-TAG purification process as discloses in Puig et al. METHODS 24, 218-229 (2001).

Tandem affinity purification (TAP) is a technique for studying protein-protein interactions. It involves creating a fusion protein with a designed piece, the TAP tag, on the end.

In the original version of the technique, the protein of interest with the TAP tag first binds to beads coated with IgG, the TAP tag is then broken apart by an enzyme, and finally a different part of the TAP tag binds reversibly to beads of a different type. After the protein of interest has been washed through two affinity columns, it can be examined for binding partners.

The original TAP method involves the fusion of the TAP tag to the C-terminus of the protein under study. The TAP tag consists of calmodulin binding peptide (CBP) from the N-terminal, followed by tobacco etch virus protease (TEV protease) cleavage site and Protein A, which binds tightly to IgG. The relative order of the modules of the tag is important because Protein A needs to be at the extreme end of the fusion protein so that the entire complex can be retrieved using an IgG matrix.

Once the fusion protein is translated within the host, the new protein at one end of the fusion protein would be able to interact with other proteins. Subsequently, the fusion protein is retrieved from the host by breaking the cells and retrieving the fusion protein through affinity selection, together with the other constituents attached to the new protein, by means of an IgG matrix.

After washing, TEV protease is introduced to elute the bound material at the TEV protease cleavage site. This eluate is then incubated with calmodulin-coated beads in the presence of calcium. This second affinity step is required to remove the TEV protease as well as traces of contaminants remaining after the first affinity step.[2] After washing, the eluate is then released with ethylene glycol tetraacetic acid (EGTA).

The native elution, consisting of the new protein and its interacting protein partners as well as CBP, can now be analyzed by sodium dodecyl sulfate polyacrylamide gel electrophoresis (SDS-PAGE) or be identified by mass spectrometry.

The disclosure further relates to an isolated purified protein complex comprising the protein consisting of the amino acid sequence as set forth in SEQ ID NO: 1, or protein derived from the protein as set forth in SEQ ID :1 by substitution, deletion, or insertion of at least one nucleotide provided that said protein retains ubiquitin ligase activity and/or participates to DNA replication, as defined above, and the ORC1 protein or the LRWD1 protein, or both.

In the invention the ORC1 protein consists of the amino acid sequence SEQ ID NO: 57, and the LRWD1 protein consists of the amino acid sequence SEQ ID NO: 58.

The complex as defined above comprises, or consists essentially, or consists of
- the protein OBI1 (SEQ ID NO:1) and
- the protein ORC1 and/or the protein LRWD1.

This complex may contain nucleic acid molecules.

The disclosure further relates to a process for identifying compounds destabilizing the complex defined above, said method comprising the following step:
- contacting a cell with a compound liable to destabilize the complex as defined above, and
- purifying the OBI1 protein as defined above.

According to the above described method, it is possible to screen compounds able to destabilize the above complex, i.e. able to inhibit the interaction between Obi1 and ORC1 or between OBI1 and LRWD1.

The process as defined above is such that, if the purification of OBI1 is not effective, then the tested compound has destabilized the complex, and if the purification of OBI1 is effective, thus the tested compound is not able to destabilize the complex.

One advantageous embodiment of this process is to use fluorescent ORC proteins and OBI1 protein. Such fluorescent proteins are obtained by adding a fluorescent tag. In this embodiment, the fluorescent proteins are able to carry out FRET (Fluorescent rescue energy transfer), such that the emitting wavelength of one of the fluorescent protein corresponds to the absorption wavelength of the other protein.

Therefore, when proteins are close, the complex is detected by exposing cells with a wavelength corresponding to the absorption wavelength of the first fluorescent molecule and by detecting the light with a filter corresponding to the emission wavelength of the second molecule.

If the complex is destabilized, it is therefore impossible to detect the light with a filter corresponding to the emission wavelength of the second molecule, since no FRET is able. The disclosure also relates to a method for diagnosing, advantageously in vitro or ex vivo, Meier-Gorlin syndrome, Seckel syndrome and Majewski osteodysplastic primordial dwarfism, comprising a step of identifying the sequence of the Obi1 gene in cells originating from an individual, and a step of comparing said sequence with a reference sequence as set forth in SEQ ID NO: 97. Thus, the invention relates to a method for diagnosing *in vitro* or *ex vivo* Meier-Gorlin syndrome comprising :
a. a step of identifying the sequence of the Obi1 gene coding for a protein as set forth in SEQ ID NO : 1, in cells originating from an individual,
b. a step of comparing said sequence with a reference sequence as set forth in SEQ ID NO: 97 to determine if a mutation has occurred in the sequence of said Obi1 gene in said cells originating from said individual, and
c. a step of determining if said mutation affects the biological function of the protein coded by said Obi1 gene in said cells originating from said individual

As demonstrated in Example 12, the inventors have identified in a patient afflicted by Meier-Gorlin syndrome a mutation (a deletion) which leads to a frameshift. The mutation therefore leads to the expression of a truncated protein.

Thus, by identifying the sequence of the Obi1 gene, by sequencing said gene, it is possible to determine if an individual is liable to be afflicted by Meier-Gorlin syndrome.

From cells form an individual suspected to be afflicted by Meier-Gorlin syndrome, it is possible to specifically amplify, by PCR, the Obi1 gene, in particular the open reading frame (ORF) of said gene, and thus to sequence this ORF. By comparison with a reference sequence, in particular the sequence as set forth in SEQ ID NO: 97, it is possible to determine if a mutation has occurred, and if said mutation affect the biological function of the resulting protein.

It is also possible to diagnose Meier-Golin syndrome, Seckel syndrome and Majewski osteodysplastic primordial dwarfism, by measuring, possibly in vitro or ex vivo, in cell from an individual suspected to be afflicted by said syndromes, the total activity of the Obi1 protein consisting of the amino acids sequence SEQ ID NO: 1.

If the total Obi1 activity is less that the Obi1 activity of a control cell (originating from healthy individual), preferably at least 1.5x lower than the activity of the control cell, thus the individual is probably afflicted by said syndrome.

The total activity can be measured by determining the Lys6 ubiquitine ligase activity of the proteins co-immuno precipitating with ORC1.

In view of the above description, the skilled person may easily specifically identify such a total Obi1 activity in cells.

The disclosure advantageously relates to a method for diagnosing Meier-Gorlin syndrome, Seckel syndrome and Majewski osteodysplastic primordial dwarfism, in an individual suspected to have or suspected to further develop said syndrome, said method comprising a step of sequencing the Obi1 cDNA to obtain its sequence, and comparing the obtained sequence with a reference sequence, and in particular with SEQ ID NO : 97. The skilled person is able to easily determine the above mentioned sequence.

The invention will be better explained in view of the following examples and the following figures.

### Brief description of the drawings:

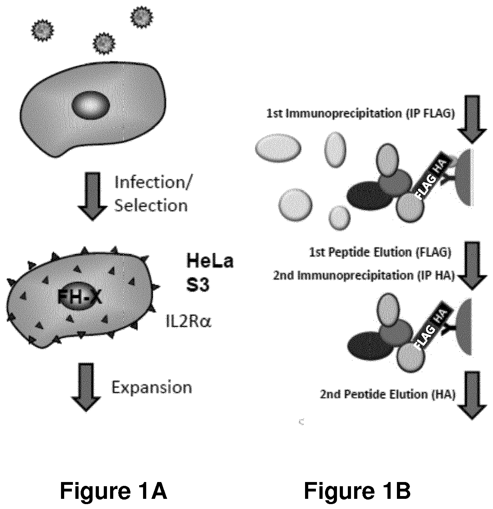

**Figures 1A-B** represent the Tandem affinity Purification (TAP)-TAG approach
Figure 1 A represents the infection of HeLa S3 wells with retroviruses encoding a tagged version of the protein of interest (bait). Infected cells were expanded after selection of IL2Rα-positive cells. Nuclei were isolated from exponentially growing cells and nuclear extract (Dignam) was prepared.
Figure 1B represents the Tandem affinity Purification (TAP)-TAG purification process which consists of tandem immunoprecipitation of the baits using the FLAG-HA tag added. Elutions (represented by the arrows) are done by competition with FLAG or HA peptide. Finally, purified complexes were separated on gel and bands were analyzed by mass spectrometry for protein identification.

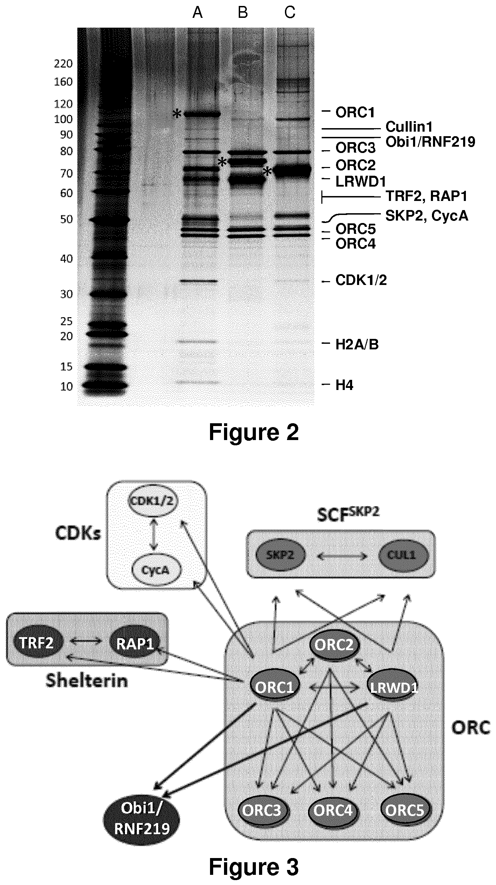

**Figure 2** represents a silver staining gel showing the proteins contained in the complexes purified according to the TAP-TAG process, by using FLAG-HA tagged ORC1 (A), ORC2 (B) or LRWD1 (C). The proteins that interact with the FLAG-HA tagged proteins are identified by mass spectrometry, and their corresponding name is indicated on the left side of the gel.
**Figure 3** shows a schematic representation of interactions found between the baits (ORC1, ORC2 or LRWD1) and cellular proteins. Several known interactions were rediscovered, including association of the ORC complex with the ubiquitin ligase SCFSkp2, cyclin-dependant kinases (CDK) and the telomere protection complex Shelterin.

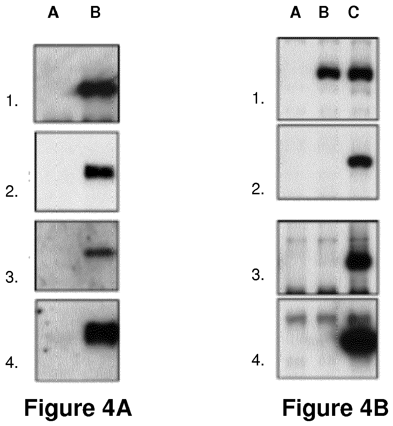

**Figures 4A-B** show the Interaction of Obi1 with the ORC complex.
Figure 4A represents a western blot gel showing proteins contained in TAP-TAG purification samples from HeLa S3 (column 1) or HeLa S3 stably expressing ORC1 FLAG-HA (column 2). Proteins were labeled with specific antibodies; 1.: anti Flag, 2.: anti ORC2, 3.: anti ORC4 and 4.: anti Obi1.
Figure 4B shows the association between ectopic OBI1 and ORC complex. U20S cells were transitently transfected with tagged Obi1 (column 2) or Obi1 and ORC1 (column 3). Untransfected cells are shown in column 1. The amount of transfected proteins is detected by specific antibodies 1.: anti Myc (detecting Obi1) and 2.: anti FLAG (detecting ORC1). 48 hours post-transfection, cell lysates were subjected to an anti-Flag immunoprecipitation (IP:Flag) (3. and 4.) and the proteins were detected with specific antibodies 3.: anti Myc (detecting Obi1) and 4.: anti FLAG (detecting ORC1)

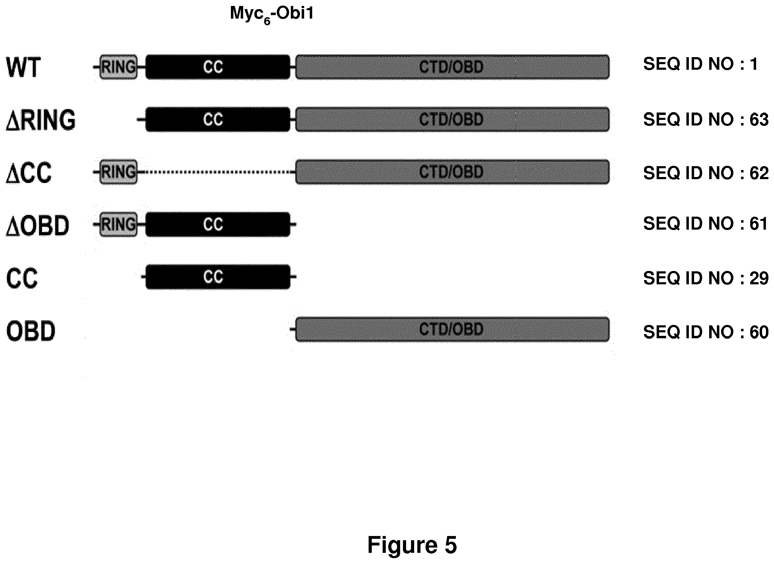

**Figure 5** shows a schematic representation of the protein and fragments of the invention. The corresponding SEQ ID are also indicated.

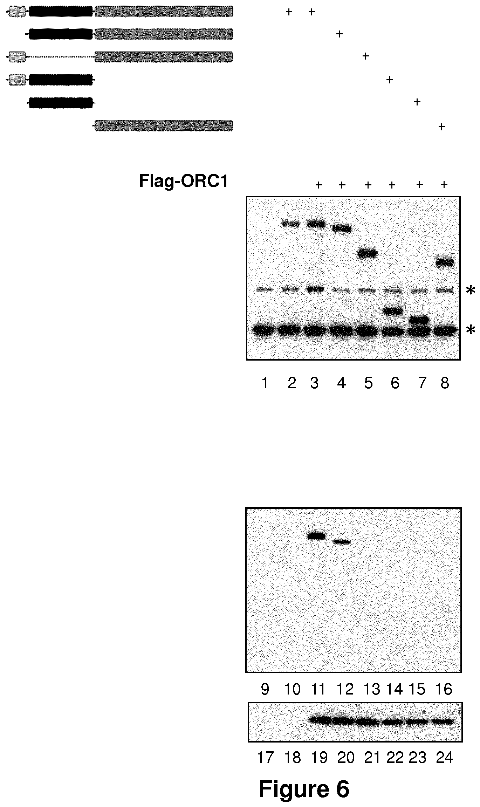

**Figure 6** represents the mapping of the Obi1/ORC interaction domains. U20S cells were transfected with the indicated Myc tagged Obi1 protein or fragments, as illustrated in figure 5, along with Flag tagged ORC1. Flag-ORC1 immunoprecipitation was performed to monitor specific Obi1 interaction. The co-immunoprecipitation of Obi1 is revealed using an anti Myc antibody (lanes 9 to 16). As control, the amount of immunoprecipitated ORC1 is revealed with an anti-Flag antibody (lanes 17 to 24), and expression of Obi1 is revealed with an anti Myc antibody (lanes 1 to 8). Non-specific bands are marked by an asterisk

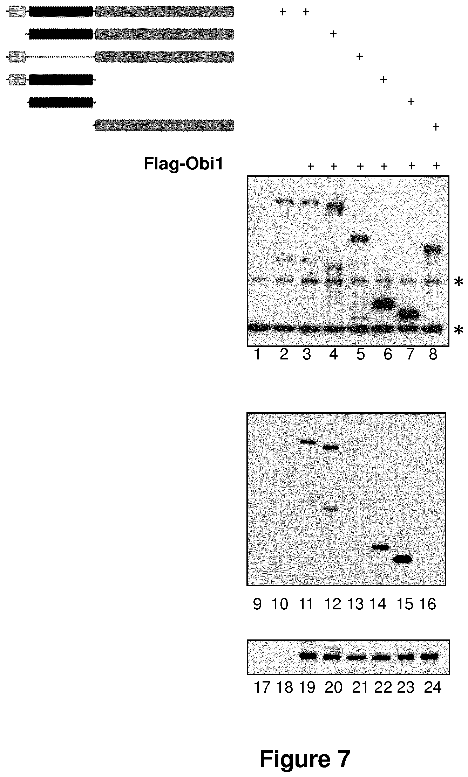

**Figure 7** represents the results showing the self-association of Obi1. U20S cells were transfected with the indicated Myc tagged Obi1 protein or fragments, as illustrated in figure 5, along with Flag tagged full length Obi1 protein. Flag-Obi1 immunoprecipitation was performed to monitor specific Obi1 dimerization. The co-immunoprecipitation of Myc Obi1 is revealed using an anti Myc antibody (lanes 9 to 16). As control, the amount of immunoprecipitated Flag Obi1 is revealed with an anti-Flag antibody (lanes 17 to 24), and expression of Myc Obi1 is revealed with an anti Myc antibody (lanes 1 to 8). Non-specific bands are marked by an asterisk.
**Figures 8A-D** represents the enhanced licensing induced by Obi1.
Figure 8A represents flow cytometry histograms showing the DNA content of U20S cells transfected with GFP protein along with 1.: empty vector, 2.: Cdt1 protein, 3.: Cdt1 and Obi1 proteins and 4.: Cdt1 protein and Obi1 devoid of RING domain. Percentages represent the proportion of cells having more than 4n DNA content (re-replicating cells).
Figure 8B represents the western blots of the transfected cells used in Figure 8A (columns 1-4). Panel a. represents a blot with an anti Myc antibody (Obi1), panel b. represents a blot with an anti Ha antibody (Cdt1). Panel c. is a control representing a blot with an anti actin antibody.
Figure 8C blots showing the chromatin loading of the replicative MCM2/MCM7 complex. U20S cells were transfected with 1.: empty vector, 2.: MCM2/MCM5 proteins (Flag tagged proteins), 3.: MCM2/MCM5 (Flag tagged proteins) and Cdt1 protein (Ha tagged protein), and 4.: MCM2/MCM5 (Flag tagged proteins), Cdt1 protein (Ha tagged protein) and Obi1 (Myc tagged protein). The soluble fraction is loaded on gel and revealed with : panel a.: anti-Flag antibody, panel b.: anti-Ha antibody, panel c.: anti Myc antibody. Panel d. is a loading control labeled with an anti actin antibody.
Figure 8D represents the chromatin fraction of the experiment described in figure 8C. panel a. is blotted with an anti-Flag antibody, and panel b., used as control, is blotted with an anti-histones antibody.
**Figures 9A-E** shows that Obi1 behaves like a proto oncogene.

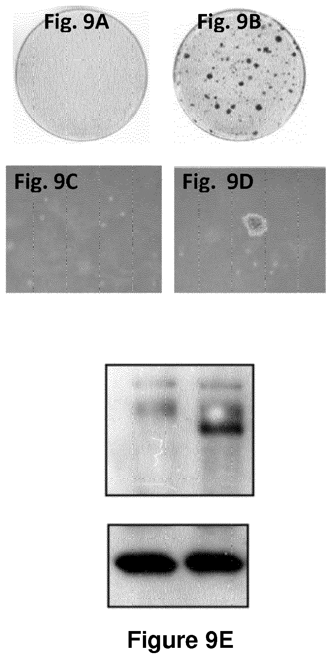

Figure 9A represents the foci formation assay of NIH 3T3 cells transfected with empty vector.
Figure 9B represents the foci formation assay of NIH 3T3 cells transfected with Obi1 protein.
Figure 9C represents the culture in soft agar of NIH 3T3 cells transfected with empty vector.
Figure 9D represents the culture in soft agar of NIH 3T3 cells transfected with Obi1 protein.
Figure 9 D represents the protein content (left column: empty vector; right column: transfected with Obi1 protein).Upper panel is a blotting with an anti-Myc antibody revealing the Obi1 protein, and the lower panel is a control of loading.

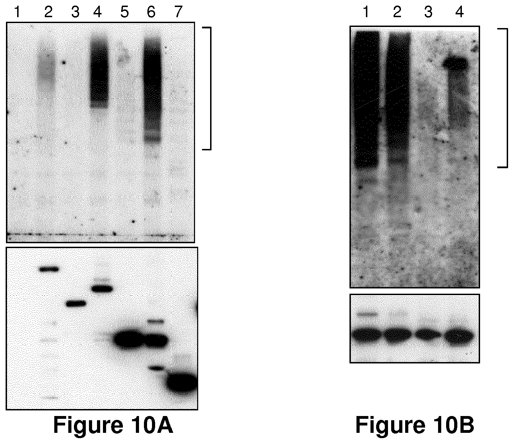

**Figures 10A-B** shows that ORC3 and ORC5 subunits of the ORC complex is polyubiquitylated via ubiquitin Lys6 linkage in vivo.
Figure 10A represents a blot of U20S cells transfected with 1: Ha-Ubiquitin alone, or with Ha-Ubiquitin along with the Flag tagged proteins: 2: ORC1, 3: ORC2, 4: ORC3, 5: ORC4, 6: ORC5 and 7: ORC6. After transfection, ORC proteins are immunoprecipitated and the ubiquitin adducts are revealed. The upper blot is a revelation with an anti-Ha antibody and the lower panel is a revelation with an anti-Flag antibody. Bracket represents the Ubiquitin adducts.
Figure 10B represents a blot of U20S cells transfected with Flag ORC5 along with 1: wild type Ha-Ubiquitin, 2: Ha-Ubiquitin having only the free K6, 2: Ha-Ubiquitin having only the free K48, 2: Ha-Ubiquitin having only the free K63. After transfection, ORC5 protein is immunoprecipitated and the ubiquitin adducts are revealed. The upper blot is a revelation with an anti-Ha antibody and the lower panel is a revelation with an anti-Flag antibody. Bracket represents the Ubiquitin adducts.

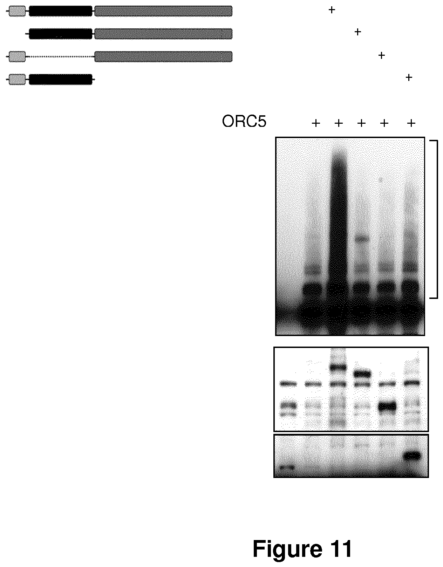

**Figures 11** represents the stimulation of ORC polyubiquitylation in vivo by Obi1. U20S cells were transfected with the indicated Myc tagged Obi1 protein or fragments, as illustrated in figure 5, along with Flag tagged ORC5. Flag-ORC5 immunoprecipitation was performed to monitor ubiquitination. The ubiquitination is revealed using an anti Ha antibody upper panel). As control, the amount of immunoprecipitated ORC5 is revealed with an anti-Flag antibody (lower panel).

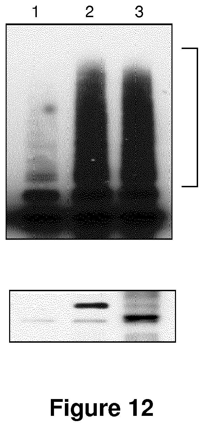

**Figure** 12 shows that the activity of Obi1 on ORC polyubiquitylation is conserved. U20S cells were transfected with Flag-ORC5 along with the human (column 2) or the xenopus Obi1 homologs (column 3). 48 hours post-transfection, Flag-ORC5 was immunoprecipitated and probed for the presence of high-molecular weight ubiquitin adducts (upper panel, anti Anti Flag). As control, the expression of human or xenopous Obi1 is evaluated (lower panel, anti Myc).

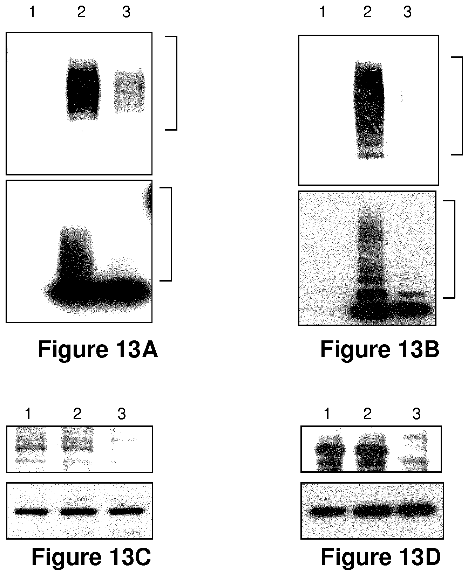

**Figures 13A-D** shows that Obi1 is the major ORC ubiquitin ligase in vivo.
Figure 13A represent a gel blotted with an anti-Ha antibody (upper panel ; Ubiquitin) or with an anti-Flag (lower panel; ORC3) of an immunipreciptation using an anti-Flag (ORC3) antibody from extracts of U20S cells transfected with Flag ORC3 along with empty vector (column 1), siRNAs against Obi1 (column 3) or a control sequence siRNA (column 2).
Figure 13B represents a gel blotted with an anti-Myc antibody (upper panel; Obi1) or with an anti-actin (lower panel; control) of total extract of the U20S cells transfected with empty vector (column 1), siRNAs against Obi1 (column 3) or a control sequence siRNA (column 2).
Figure 13C represent a gel blotted with an anti-Ha antibody (upper panel ; Ubiquitin) or with an anti-Flag (lower panel; ORC5) of an immunipreciptation using an anti-Flag (ORC5) antibody from extracts of U20S cells transfected with Flag ORC5 along with empty vector (column 1), siRNAs against Obi1 (column 3) or a control sequence siRNA (column 2).
Figure 13B represents a gel blotted with an anti-Myc antibody (upper panel; Obi1) or with an anti-actin (lower panel; control) of total extract of the U20S cells transfected with empty vector (column 1), siRNAs against Obi1 (column 3) or a control sequence siRNA (column 2).

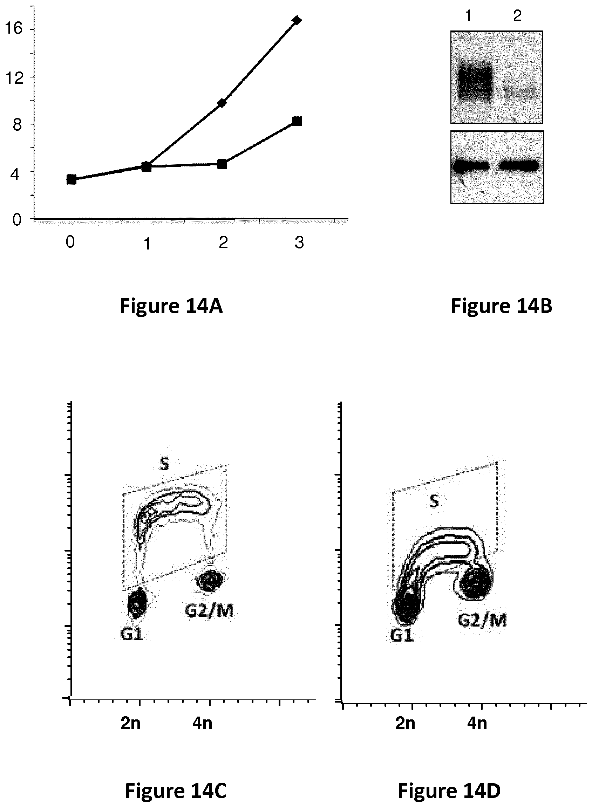

**Figures 14A-D** show the importance of Obi1 for efficient cell proliferation and efficient DNA replication
Figure 14A represents a graph showing cell proliferation of U20S cells transfected with siRNAs against Obi1 (curve with squares) or a control sequence (curve with diamonds). X-axis represents the time in days and Y axis represents the number of cells (x10⁵).
Figure 14B represents the expression of Obi1 in U20S cells transfected with siRNAs against Obi1 (left column; upper panel) or a control sequence (right column; upper panel). As control, the membrane is blotted with an anti-actin antibody (lower panel).
Figure 14C represents a graph showing the Bromodeoxyuridine (BrdU) content and the DNA content of cells transfected with non relevant siRNA. G1, S and G2 phases of the cell cycle are indicated.
Figure 14C represents a graph showing the Bromodeoxyuridine (BrdU) content (Y-axis) and the DNA content (X-axis) of cells transfected with non relevant siRNA. G1, S and G2 phases of the cell cycle are indicated.
Figure 14C represents a graph showing the Bromodeoxyuridine (BrdU) content (Y-axis) and the DNA content (X-axis) of cells transfected with Obi1 siRNAs. G1, S and G2 phases of the cell cycle are indicated.

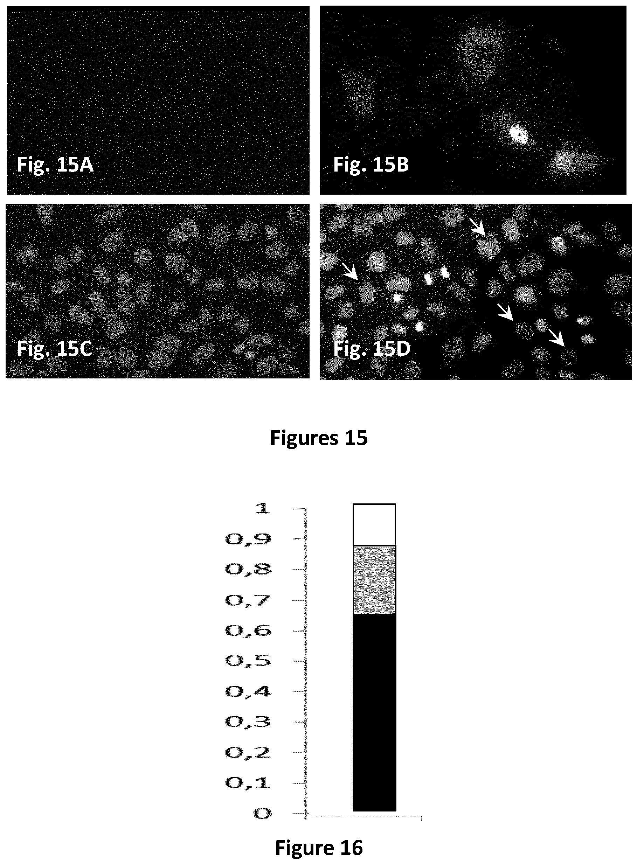

**Figures 15A-D** show the subcellular localization of Obi1.
Figure 15A represents the immunofluorescent result of U20S cells transfected with an empty vector and labelled with an anti-Myc antibody, and revealed with an anti body labelled with FITC.
Figure 15B represents the immunofluorescent result of U20S cells transfected with a vector expressing Myc-Obi1 and labelled with an anti-Myc antibody, and revealed with an anti body labelled with FITC.
Figure 15C represents the immunofluorescent result of U20S cells transfected with an empty vector and labelled with Hoescht to label DNA of cells shown in figure 15A
Figure 15D represents the immunofluorescent result of U20S cells transfected with a vector expressing Myc-Obi1 and labelled with Hoescht to label DNA of cells shown in figure 15B.
**Figure 16** represents a histogram showing the localization of Obi1 in cytoplasm or in the nucleus, scored on at least 100 cells based on the nuclear:/cytoplasm distribution of the signal observed as shown in figures 15. White represents a nuclear staining with low cytoplasmic signal (N>C); grey represents cells showing an equal distribution between the nuclear and cytosolic compartment (N=C) and black represents cells having exclusive cytoplasmic signal (N<C).

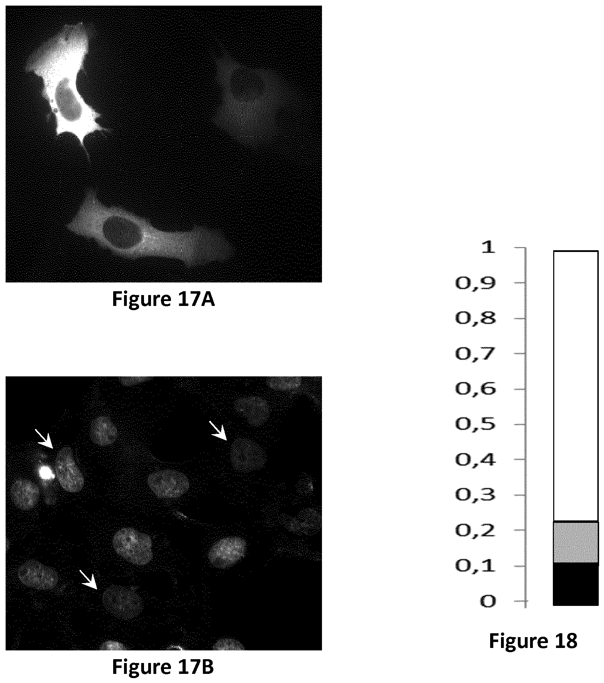

**Figures 17A and B** show the subcellular localization of Myc-NES-Obi1(1-280).
Figure 17A represents the immunofluorescent result of U20S cells transfected with a vector allowing the expression of the Myc-NES-Obi1(1-280) protein and labelled with an anti-Myc antibody, and revealed with an anti body labelled with FITC.
Figure 17B represents the immunofluorescent result of U20S cells transfected with a vector expressing Myc-Obi1 and labelled with Hoescht to label DNA of cells shown in figure 17A.
**Figure 18** represents a histogram showing the localization of the Myc-NES-Obi1(1-280) protein in cytoplasm or in the nucleus, scored on at least 100 cells based on the nuclear:/cytoplasm distribution of the signal observed as shown in figures 15. White represents a nuclear staining with low cytoplasmic signal (N>C); grey represents cells showing an equal distribution between the nuclear and cytosolic compartment (N=C) and black represents cells having exclusive cytoplasmic signal (N<C).

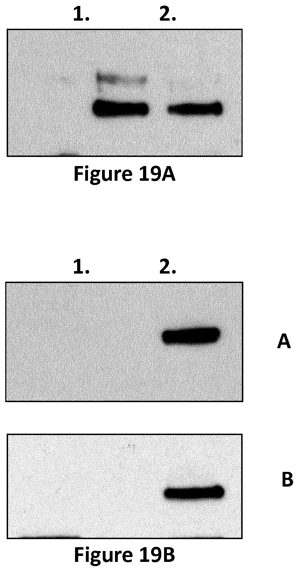

**Figures 19A and B** show that NES-Obi1(1-280) protein associates with Obi1 protein.
Figure 19A represents a western blot showing the expression the Myc tagged proteins of U20S cells transfected with Myc-NES-Obi1(1-280) (1.) or Myc-NES-Obi1(1-280) and Flag-Obi1 WT (2.). The blot was labelled with an anti-Myc antibody.
Figure 19B represents a western blot showing the immunoprecipitation of the Myc tagged proteins co-immunoprecipitated with anti Flag antibody of U20S cells transfected with Myc-NES-Obi1(1-280) (1.) or Myc-NES-Obi1(1-280) and Flag-Obi1 WT (2.). The blot was labeled with an anti-Myc antibody (upper panel). Lower panel is the same blot labelled with an anti Flag antibody.

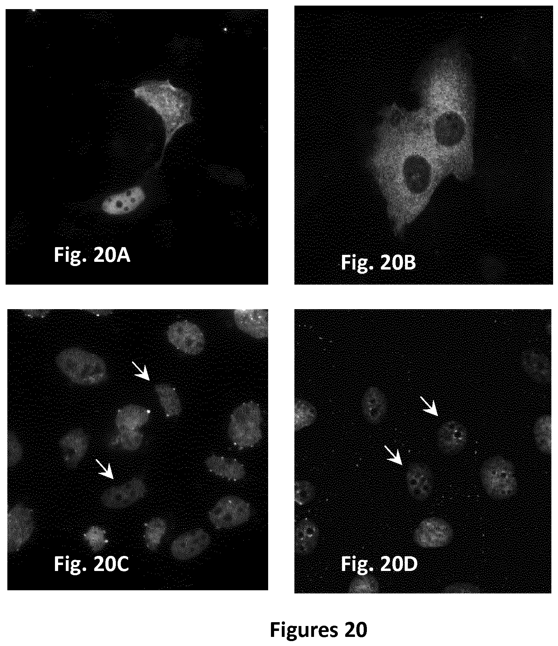

**Figures 20A-D** shows that the Myc-NES-Obi1(1-280) protein drives Obi1 away from the nucleus.
Figure 20A represents the immunofluorescent result of U20S cells transfected with an empty vector along with a vector allowing the expression of the Flag-WT Obi1 protein, and labelled with an anti-Flag antibody, and revealed with an anti body labelled with FITC.
Figure 20B represents the immunofluorescent result of U20S cells transfected with a vector expressing the Myc-NES-Obi1(1-280) protein along with a vector allowing the expression of the Flag-WT Obi1 protein, and labelled with an anti-Myc antibody, and revealed with an anti body labelled with FITC.
Figure 20C represents the immunofluorescent result of U20S cells transfected with an empty vector along with a vector allowing the expression of the Flag-WT Obi1 protein, and labelled with Hoescht to label DNA of cells shown in figure 20A
Figure 20D represents the immunofluorescent result of U20S cells transfected with a vector expressing the Myc-NES-Obi1(1-280) protein along with a vector allowing the expression of the Flag-WT Obi1 protein, and labelled with Hoescht to label DNA of cells shown in figure 20B.

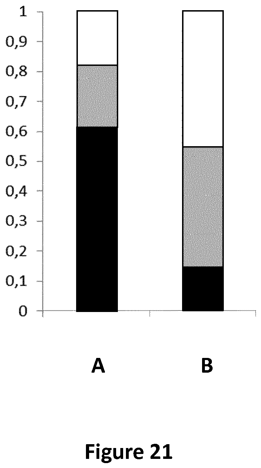

**Figure 21** represents histograms showing the localization of the Myc-NES-Obi1(1-280) protein in cytoplasm or in the nucleus, scored on at least 100 cells based on the nuclear:/cytoplasm distribution of the signal observed as shown in figures 20A and C (A) or figures 20B and D (B). White represents a nuclear staining with low cytoplasmic signal (N>C); grey represents cells showing an equal distribution between the nuclear and cytosolic compartment (N=C) and black represents cells having exclusive cytoplasmic signal (N<C).

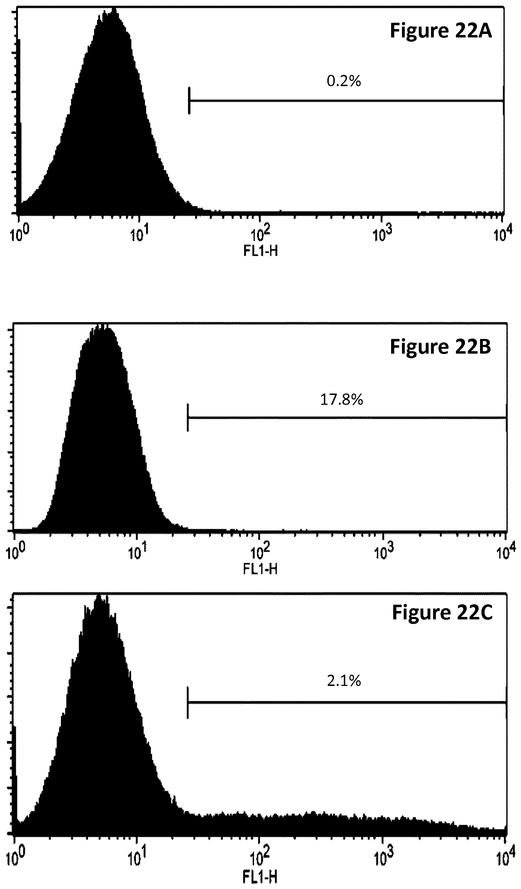

**Figures 22A-C** shows that the expression of NES-Obi1(1-280) induces a selective loss of transfected cells of the transfected vectors.
Figure 22A represents a graph measuring the intensity of GFP fluorescence (X-axis) of U20S cells transfected with empty vectors.
Figure 22B represents a graph measuring the intensity of GFP fluorescence (X-axis) of U20S cells transfected with an empty vector and a vector allowing the expression of the fluorescent EGFP protein.
Figure 22C represents a graph measuring the intensity of GFP fluorescence (X-axis) of U20S cells transfected with a vector expressing the Myc-tagged NES-Obi1(1-280) protein and a vector allowing the expression of the fluorescent EGFP protein.

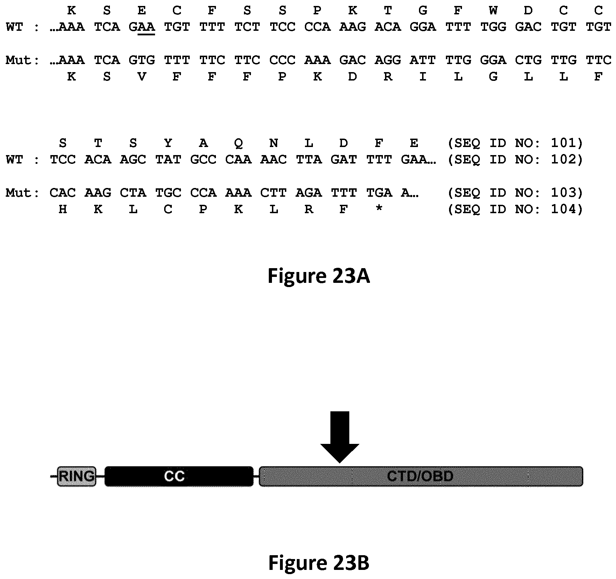

**Figures 23A-B** show the DNA and protein sequence of Obi1 in a patient afflicted by Meier-Gorlin syndrome.
Figure 23A represents the alignment of wild-type and mutated Obi1 coding sequences (SEQ ID NO : 101 and 104). The corresponding amino acids are also shown (SEQ ID NO : 102 and 103). The mutation is a two nucleotides deletion (AA, underlined) resulting in a frameshift and a premature STOP codon (* in SEQ ID NO ; 104).
Figure 25B represents the position of the mutation in a schematic representation of the Obi1 protein. The mutation, leading to a truncation, maps within the C-terminal domain of Obi1 (indicated by an arrow).

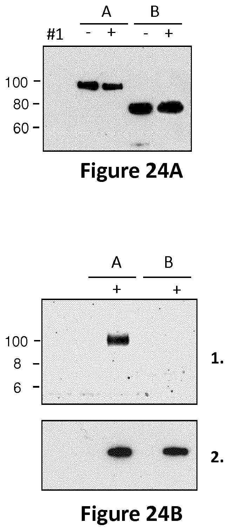

**Figures 24A-B** show that the mutated Obi1 found in the patient is impaired for ORC association.
Figure 24A represents a western blot showing the expression of WT Obi (1) and mutated Obi1 (2) proteins, when co-expressed (+) or not (-) with Flag-ORC1 protein (#1). Proteins were revealed by using anti-Myc antibody. Numbers (100, 80, 60) represent the molecular mass in kDa.
Figure 24B represents a western blot of a co- immunoprecipitation of Obi1 proteins (WT:A; mutated: B - panel 1.) by using an anti-Flag antibody that immunoprecipitates Flag-ORC1 protein.). Proteins were revealed by using anti-Myc antibody. Numbers (100, 80, 60) represent the molecular mass in kDa. Panel 2. shows the immunoprecipitated ORC1 proteins. Proteins were revealed by using anti-Flag antibody

### EXAMPLES

### Example 1 : Materials and methods

### Cell culture, transfections and infections

U20S, HeLa S3, NIH 3T3 and Platinum-E (Cell Biolabs) cells were grown in DMEM (Invitrogen) supplemented with 10% Foetal Bovine Serum (FBS), 2 mM glutamine and antibiotics. Cells were transfected using the Lipofectamine reagent (Invitrogen) as specified by the manufacturer. For TAP-TAG experiments, HeLa S3 cells stably expressing baits were generated by retroviral transduction. For this, viral particles were generated using Platinum-E ecotropic packaging cell line transiently transfected with the retroviral constructs. In parallel, HeLa S3 cells were transiently transfected with mouse Cationic Aminoacid Transporter 1 to render them susceptible to infection by ecotropic retroviruses. 48 hours after transfection, S3 cells were incubated with viral supernatant in presence of polybrene (10 um/mL, Sigma Aldrich) for 24 hours. 48 hours later, transduced cells express human IL2 receptor alpha chain which allows selection using mouse anti-IL2Rα antibody (Upstate Biotechnology) coated magnetic beads as specified by the manufacturer (Dynabeads M-450 Epoxy, Invitrogen). Knockdown experiments were done using a pool of 4 siRNAs (Smartpool, Dharmacon) targeting human Obi1 transfected using Interferin reagent (PolyPlus Transfection) as specified by the manufacturer. 10 nM siRNA was added to the cells.

### Antibodies

Anti-Flag M2 [# F1804; 1/1000], anti-Actin [# A5441; 1/5000] and anti-PCNA [# WH0005111 M2; 1/2500] monoclonal antibodies were from Sigma. The anti-Myc 9B11 monoclonal antibody [# 2276S; 1/1000] was from Cell Signaling Technologies. The polyclonal antibodies against HA [# sc-805; 1/100], Obi1/RNF219 [# sc-84039; 1/100] and ORC4 [# sc-20634; 1/100] were from Santa-Cruz BioTechnologies. The anti-ORC2 antibody [1/1000] was described previously (Cayrou et al. (2011) Genome Res 21:1438-49.)

### Plasmid constructs

For TAP-TAG experiments, human ORC1, ORC2 and LRWD1 were amplified by PCR using cloned cDNA as template with the following primers:
Orc1CnotS: CTGGCGGCCGCACCATGGCACACTACCCCACAAGG (SEQ ID NO: 64)
Orc1CnotAS: CTGGCGGCCGCCTCGTCTTTCAGCGCATAC (SEQ ID NO: 65)
Orc2CnotS: CTGGCGGCCGCACCATGAGTAAACCAGAATTAAAGGAAGA (SEQ ID NO: 66)
Orc2CnotAS: CTGGCGGCCGCAGCCTCCTCTTCTTCCTTTTC (SEQ ID NO: 67)
LrwdCnotS: CTGGCGGCCGCACCATGGGCCCCCTCTCGG (SEQ ID NO: 68)
LrwdCnotAS : CTGGCGGCCGCCATCCTCCCCCAGATGGC (SEQ ID NO: 69).

These primers introduced a Not I restriction enzyme site at both end of the cDNA. Using Pfu polymerase (Promega) as specified by the manufacturer: The following PCR conditions were:
1) 2 min at 95°C
2) 30 sec at 95°C
3) 30 sec at 58°C
4) 2 min/kb extension time at 73°C
5) repeat step 2-4 25 times
6) 5 min at 74°C
7) End, 4°C

PCR products were purified (Qiaquick, Qiagen) and digested with Notl. The DNA fragments were subcloned at the Not I site of pOZ-FH-C to produce a protein having an inframe C-Terminal Flag-HA tag. Restriction digestion and sequencing analysis allowed us to screen for the proper construction. For co-immunoprecipitation and ubiquitination studies, a vector expressing an N-Terminal Flag tag was constructed. The pCS2 vector was digested with BamHI/EcoRI fragment, which removes the Myc6 tag and ligated with the following annealed oligos:
FlagBamNcoEcoS:
   GATCCGCCAACATGGACTACAAGGACGACGATGACAAGTCCATGGCTGGG (SEQ ID NO: 70)
FlagBamNcoEcoAS:
   AATTCCCAGCCATGGACTTGTCATCGTCGTCCTTGTAGTCCATGTTGGCG (SEQ ID NO: 71)

This yields pCS2-Flag. Then, ORC1-6 were amplified by PCR using the cloned cDNA as template with the following template:
ORC1_XbaS: CTGTCTAGAGCCATGGCACACTACCCCACAAGGC (SEQ ID NO: 72)
ORC1_XbaAS: CTGTCTAGATTACTCGTCTTTCAGCGCATAC (SEQ ID NO: 73)
ORC2_NcoS : CTGCCATGGGAAGTAAACCAGAATTAAAGGAAGAC (SEQ ID NO: 74)
ORC2_XhoAS: CTGCTCGAGTCAAGCCTCCTCTTCTTCCTT (SEQ ID NO: 75)
ORC3_NcoS : CTGCCATGGCTACGTCCTCGAT (SEQ ID NO: 76)
ORC3_XbaAS : CTGTCTAGATTAGCAGCCTCCCCATGTTAG (SEQ ID NO: 77)
ORC4_XhoS : CTGCTCGAGAGCAGTCGTAAATCAAAGAG (SEQ ID NO: 78)
ORC4_XbaAS : CTGTCTAGATTATAACCAGCTTAGTGAGGATG (SEQ ID NO: 79)
ORC5_EcoS : CTGGAATTCTCCCCACTTGGAAAACGTGG (SEQ ID NO: 80)
ORC5_XhoAS: CTGCTCGAGTCACAAGAAATCATACAAGTATTT (SEQ ID NO: 81)
ORC6_XhoS : CTGCTCGAGGGGTCGGAGCTGATCG (SEQ ID NO: 82)
ORC6_XbaAS: CTGTCTAGATTACTCTGCTGTAGCCTTTTGA (SEQ ID NO: 83).

These primers introduced the indicated sites at both end of the cDNA. Using Pfu polymerase (Promega) as specified by the manufacturer: The following PCR conditions were:
1) 2 min at 95 °C
2) 30 sec at 95 °C
3) 30 sec at 58 °C
4) 2 min/kb extension time et 73 °C
5) repeat step 2-4 25 times
6) 5 min at 74 °C
7) End, 4 °C

PCR products were purified (Qiaquick, Qiagen) and digested with the appropriate restriction enzymes. The DNA fragments were subcloned at the relevant site in pCS2-FLAG vector. Restriction digestion and sequencing analysis allowed us to screen for the proper construction. To construct human Myc6-tagged Obi1 WT expression vectors, we amplified by PCR using the cloned cDNA as template with the following template:
Rn_1_NcoS: CTGCCATGGCTCAGACCG (SEQ ID NO: 84)
Rn_Stop_XbaAS: CTGTCTAGATTAACTTTTAGTTGCTTTTGATGGG (SEQ ID NO: 85)

For Obi1 ΔRING, the following primer were used:
Rn_54_NcoS: CTGCCATGGCTTGCAGAGTCCCCATCA (SEQ ID NO: 86)
Rn_Stop_XbaAS: CTGTCTAGATTAACTTTTAGTTGCTTTTGATGGG (SEQ ID NO: 85)

For Obi1 ΔCTD/OBD, the following primers were used:
Rn_1_NcoS: CTGCCATGGCTCAGACCG (SEQ ID NO: 84)
Rn_280XbaAS: CTGTCTAGATTATTTGCCATCTGCAGAAAGTG (SEQ ID NO: 87)

For Obi1 CC, the following primers were used:
Rn_54_NcoS: CTGCCATGGCTTGCAGAGTCCCCATCA (SEQ ID NO: 86)
Rn_280XbaAS: CTGTCTAGATTATTTGCCATCTGCAGAAAGTG (SEQ ID NO: 87)

For Obi1 CTD/OBD, the following primers were used:
Rn_280NcoS: CTGCCATGGGGAGCAAAGGCAGTGAG (SEQ ID NO: 88)
Rn_Stop_XbaAS: CTGTCTAGATTAACTTTTAGTTGCTTTTGATGGG (SEQ ID NO: 85)

These primers introduced the indicated sites at both end of the cDNA. Using Pfu polymerase (Promega) as specified by the manufacturer: The following PCR conditions were:
1) 2 min at 95 °C
2) 30 sec at 95 °C
3) 30 sec at 58 °C
4) 2 min/kb extension time et 73 °C
5) repeat step 2-4 25 times
6) 5 min at 74 °C
7) End, 4 °C

PCR products were purified (Qiaquick, Qiagen) and digested with Ncol/Xbal. The DNA fragments were subcloned at the relevant site in pCS2.

To construct ObiΔCC, the RING domain of Obi1 was amplified with the following primer:
Rn_1_NcoS: CTGCCATGGCTCAGACCG (SEQ ID NO: 84)
Rn_73NcoBAS: CTGCCATGGATCCTCCTATAATTTCTTTGCAA (SEQ ID NO: 89)
and cloned at the Ncol of pCS2-Obi1 CTD/OBD. Restriction digestion and sequencing analysis allowed us to screen for the proper construction. To construct Xenopus Myc6-tagged Obi1 WT expression vectors, we amplified by PCR using the cloned cDNA as template with the following template:
RnXenoEcoS: CTGGAATTCTATGGCGCAGACTGTTCAGAA (SEQ ID NO: 90)
RnXenoXhoAS: CTGCTCGAGTCATGGCTTAAAGGACTTTGGAG (SEQ ID NO: 91)
PCR products were purified (Qiaquick, Qiagen) and digested with EcoRI/Xhol. The DNA fragments were subcloned at the relevant site in pCS2. HA-tagged ubiquitin WT and K mutants are described (Sobhian et al. (2007) Science 316:1198-202.). For re-replication experiments, human WT Cdt1 with an C-Terminal HA-tag was generated. First, pcDNA3 (Invitrogen) was digested with EcoRI/Xbal and the following annealed oligos were cloned:
HAEcoXbaS: AATTCTATGTATGATGTTCCTGATTATGCTAGCCTCTAAT (SEQ ID NO: 92)
HAEcoXbaAS: CTAGATTAGAGGCTAGCATAATCAGGAACATCATACATAG (SEQ ID NO: 93)

To yield pcDNA3-HACT. Then, human WT Cdt1 was amplified by PCR using cloned cDNA as template with the following primers:
hCdt1-Cter-HdS: CTGAAGCTTACCATGGAGCAGCGCCGC (SEQ ID NO: 94)
hCdt1-Cter-EcoAS: CTGGAATTCAGCCCCTCCTCAGCAC (SEQ ID NO: 95).

PCR products were purified (Qiaquick, Qiagen) and digested with HindIII/EcoRI. The DNA fragments were subcloned at the relevant site in pcDNA3-HACT. pEGFP-C2 (Clontech) was used an EGFP expression vector. In chromatin fractioning experiments, Flag-tagged human MCM2 and MCM5 have been described (Tardat et al (2010) Nat Cell Biol 12:1086-93.)

### Re-replication assay

U20S cells were co-transfected with the tested constructs and an expression plasmid coding for EGFP. 60-72 h post-transfection, cells were trypsinized and fixed in 1% paraformaldehyde in PBS at RT for 15 min. Washed cells were fixed again in 70% ethanol in PBS and stored at -20°C overnight. Cells were spun at 2000 rpm for 10 min, washed in PBS and treated with 50 µg/mL RNAse A. DNA was stained with 25 µg/mL propidium iodide. Cells were analysed with a FACSCalibur flow cytometer using the CellQuestPro software.

### Protein biochemistry

For cell lysis, immunoprecipitation and western blotting were performed following standard methods. Briefly, cells were lysed in TK300 lysis buffer (20 mM Tris-HCI pH 7.9; 300 mM KCI, 5 mM MgCI2, 10% glycerol; 0.5% NP-40 plus protease inhibitors cocktail) on ice for 30 min. The protein concentration of the clarified lysates was estimated using the BCA method (Pierce). For immunoprecipitation experiments, equivalent amounts of protein samples were incubated at 4°C with anti-Flag coupled agarose beads (Sigma Aldrich) for 4h. After extensive washing with TK300 lysis buffer, bound proteins were eluted in boiling Laemmli buffer. Samples were runs on pre-cast gradient gel (NuPage Novex, Invitrogen and Mini-PROTEAN TGX, Biorad) and transferred to nitrocellulose membrane. Western blot were revealed using ECL prime (Amersham).

### In vivo ubiquitination assay

U20S cells were co-transfected with Flag-tagged ORC subunits along with HA-ubiquitin (WT or K only mutants). In some cases, Obi1 (WT or mutants) was co-transfected. In other cases, endogenous Obi1 expression was silenced by treated the cells with Obi1 siRNAs 24 hours before plasmid transfection. 48 hours after plasmid transfection, cells were lysed in Triton X-100 lysis buffer (50 mM Tris-HCI pH=7.4; 100 mM NaCl; 50 mM NaF; 5 mM EDTA; 40 mM β-glycero-phosphate; 1% Triton X-100 plus protease inhibitors cocktail) supplemented with 10 mM N-ethyl maleimide (NEM, Sigma) to inhibits deubiquitinating enzymes. Cell lysates (750 µg protein) were then incubated for 2 h at 4°C with anti-Flag coupled agarose beads (Sigma Aldrich). The bound proteins were washed 5 times in lysis buffer and resolved on gradient gels. Ubiquitin-containing conjugates were detected by immunoblotting with anti-HA antibody.

### Chromatin isolation

Chromatin and soluble fractions were obtained using the CSK procedure. Briefly, cells were lysed in CSK buffer (10 mM PIPES pH=6.8; 100 mM NaCl; 300 mM sucrose; 1 mM MgCl2; 0.5 mM DTT; 0.2% Triton X-100; 1 mM ATP plus protease inhibitor cocktail) on ice for 10 min. Insoluble material was pelleted (3000 rpm, 4°C, 3 min). Supernatants (soluble fraction) were kept, while pellets were extracted a second time in CSK buffer. The final pellets (chromatin fraction) were solubilized in Laemmli buffer.

### Foci formation assay

NIH 3T3 cells stably expressing human Obi1 or the empty vector were plated in duplicate in 60 mm plates at equivalent density. Confluent cells were cultured for 2-3 weeks and the culture medium was changed every 2-3 days. Foci were stained with crystal violet dye.

### Soft agar growth assay

105 cells of NIH 3T3 cells stably expressing human Obi1 or the empty vector were mixed with medium supplemented with 0.4% noble agarose (sigma) and placed on top of the 1% agarose layer. 1 mL medium was added to the solidified layer and changed every 2-3 days. After 3-4 weeks, colonies were counted in 10 randomly selected fields using a phase contrast microscope.

### Preparation of Nuclear Extracts (Dignam method)

Hela S3 cells (40^{∗}150 mm confluent dishes) were trypsinized to obtain a homogenous cell suspension. After a wash in PBS, cells were lyzed in a hypotonic buffer (HB buffer: 10 mM Tris pH 7.9; 1.5 mM MgCl2; 10 mM KCI; 1 mM DTT; 10 mM PMSF). Briefly, cells were washed once in ice cold HB buffer (10 times the cell pellet volume (CPV). The pelleted cell pellet was then resuspended in 2 times CPV of HB buffer. Cells were incubated on ice for 10-15 min. Swollen cells are transferred to a dounce homogenizer and cells were lyzed by 15 stroke using the tight pistol. The nuclei are pelleted by centrifugation. The supernatant is discarded and the nuclei are resuspended in 0.5 nuclear pellet volume (NPV) of Low Salt (LS) buffer (20 mM Tris pH 7.9; 1.5 mM MgCl2; 0.2 mM EDTA; 20 mM KCI; 1 mM DTT; 10 mM PMSF). Whilst gently vortexing the tube, add 0.5x NPV of High Salt (HS) buffer (20 mM Tris pH 7.9; 1.5 mM MgCl2; 0.2 mM EDTA; 1200 mM KCI; 1 mM DTT; 10 mM PMSF) drop by drop. After a 30 min incubation with agitation in the cold room, the Dignam nuclear extracts are clarified by centrifugation and freeze in liquid nitrogen. The extracts were kept at -80C

### TAP-TAG procedure

All procedures were done at 4C and on ice. Thaw Dignam extract on ice for 30-45 min. Spin (13 000 rpm, 20 min) to clarify the extract. A typical experiment involves ∼10 mL of extract. We first performed the Flag IP. We use 1 % volume/volume of anti-Flag coupled agarose beads (Sigma, ∼100 µL). Beads were washed to diminish background binding: ¼ volume beads of 0.1M Glycine pH=2.2 was added to the beads and incubate for 3 min at room temperature. ½ volume beads of Tris 1M pH=7.9 was added. The beads were washed twice with 1 mL of HA-IP Buffer (150mM KCI; 20mM Tris pH 7,5 0,05% NP40; 0,1% Tween; 10% Glycerol; 5mM MgCl₂; 1mM DTT and 10 mM PMSF). After the last centrifugation, beads were resuspended in HA-IP Buffer (1vol of Beads). Extracts were incubated with Flag beads for 4 h with agitation at 4 C. Beads were pelleted (3min at 3000rpm 4°C). Beads were washed 5 times with HA IP Buffer: Beads are pelleted (3K 3min 4°C) and the supernatant discarded. Complexes were eluted from the beads by incubating in 3 beads volume of HA IP Buffer supplemented with Flag peptide (200µg/mL, Sigma) for 1 hour at 4 C with agitation. The supernatant was kept on ice and a second elution was conducted. The eluates were pooled and subjected to an anti-HA immunoprecipitation. Before, anti-HA coupled agarose beads (SantaCruz Biotechnology) were washed with glycine buffer as described above. 10 µL of beads was used per IP. Flag eluates were incubated with anti-HA beads for 2 hours at 4 C with agitation. Beads were pelleted (3min at 3000rpm 4°C). Beads were washed 5 times with HA IP Buffer: Beads are pelleted (3K 3min 4°C) and the supernatant discarded. Complexes were eluted from the beads by incubating in 3 beads volume of HA IP Buffer supplemented with HA peptide (400 µg/mL, Roche) for 1 hour at 4 C with agitation. The supernatant was keep on ice and a second elution was conducted. HA eluates were clarified on a filter column (Pierce Spin Cups Cellulose Acetate Filter ThermoFisher): columns were rinsed with 400 µl of HA IP buffer and spun for 1 min at 7000 rpm at 4°C. The supernatant was discarded and eluates were apply to the column. After spinning, the flow-through was collected and denatured in lammeli buffer. 25% of the samples were run in precast NuPage gradient gel and stained by silver nitrate (SilverQuest, Invitrogen) as specified by the manufacturer. 75 % of samples were run on precast gel and stained with colloidal blue staining kit (Invitrogen) as specified by the manufacturer.

### Mass spectrometry analysis

Bands on colloidal blue stained gels were excised, reduced with DTT and alkylated with lodoacetamine (IAA) using standard procedure. Bands were then subjected to trypsin (Promega) digestion. Extracted peptides were collected and analysed using a nanoESI LTQ_XL Orbitrap mass spectrometer (Thermo Fisher Scientific) coupled to an Ultimate 3000 HPLC (Dionex). A gradient of 0-40% B for 30 min and 80% B for 15 min (A = 0.1% formic acid, 2% acetonitrile in water; B = 0.1 % formic acid in acetonitrile) at 300 nl/min was used to elute peptides from the capillary (0.075 mm x 150 mm) reverse-phase column (Pepmap®, Dionex). Nano-ESI was performed with a spray voltage of 2.4 kV, heated capillary temperature of 200°C, and tube lens voltage of 140 V. A cycle of one full-scan mass spectrum (400-1600 m/z) at a resolution of 30000, followed by five data-dependent MS/MS spectra was repeated continuously throughout the nanoLC separation. All MS/MS spectra were recorded using normalized collision energy and an isolation window of 2 m/z. Data were acquired using the Xcalibur software (v 2.0.7, Thermo Fisher Scientific). Data analysis was performed using MaxQuant 1.3.0.5 and standard parameters against the CPS_HUMAN database (November 2012). FDR was set at 1%.

### Example 2: Purification of the OBI1 protein

Identification of the interaction partners of a particular protein became a valuable way to gain insight into the physiological role of the protein.

The most widely used system to identify intermolecular interactive partners of a particular protein of interest is the yeast two-hybrid system. However, given that this system is designed to detect mostly binary interactions, it is not suitable for investigating the intermolecular interaction partners of a protein if the latter is a component of a multimeric protein complex.

For identification of intermolecular interaction partners, purification of the native protein complexes from the cells would be the best method. This would provide information on how the endogenous protein interacts with intermolecular interaction partners. However, in general, purification of native complexes is technically difficult and time-consuming, especially when the protein of interest is scarce. These limitations can be addressed with epitope tagging. It has been shown that stably expressed exogenous proteins with epitope tags are integrated into the complexes when the complex is synthesized de novo. Such a protein complex can be purified rapidly by immunoprecipitation with antibodies against epitopes.

The inventors took advantage of the epitope-tagging technique to develop a standardized approach to purifying multimeric protein complexes from mammalian cells. We put two different epitope tags tandemly (i.e., FLAG and HA) on the C terminus of the proteins of interest. For rapid generation of cell lines that express the epitope-tagged proteins of interest, the inventors used retroviral transduction. The cells are sorted magnetically after transduction. Once stably transduced cell lines are established, the protein complex can be purified from cells in less than 24 h.

The above steps are represented in figures 1A-B.

Results of TAP-TAG assays are reproduced on figure 2. Nuclear extract from HeLa S3 cells stably expression the indicated proteins were subjected to the Tandem-Affinity Purification (TAP)-TAG approach. Purified complexes were visualized by Silver staining and Mass Spectrometry for protein identification (shown on the left side).

These data demonstrates that Obi1/RFN219 interacts specifically with ORC1 and LRWD1, but is undetectable for ORC2.

Figure 3 summarize the ORC complex interactions.

### Example 3: OBI1 protein interacts with ORC1

In order to validate the TAP-TAG interaction, the inventors have tested the Obi1-ORC1 interaction in vitro by co-immunoprecipitation.

Results are shown in figures 4A-B.

As shown, immunoprecipitation of ORC1 co-immunoprecipitate Obi1 protein, confirming the interaction observed in TAP-TAG assay.

Further, to determine Obi1 domains involved in ORC1/Obi1 interaction, the inventors have constructed Obi1 deletion fragments of Obi1 and evaluated their ability to interact with ORC1. Constructions are shown in figure 5.

Figure 6 shows the results. In vitro, Obi1 requires its C-terminal Domain (CTD) and Coiled-coil (CC) region, but not its catalytic RING domain for ORC association.

### Example 4: OBI1 self-associates

To further identify biochemical properties of Obi1, the inventors have evaluated its ability to self-associate.

The deletion mutants described in figure 5 were co-immunoprecipitated with full length Obi1 protein.

Results are shown in figure 7.

It is to be noticed that all constructs having the Coiled-coil region can dimerize with WT Obi1.

### Example 5: OBI1 enhances licensing activity

Since ORC1 participate to DNA replication, the inventors have evaluated Obi1 function during DNA replication.

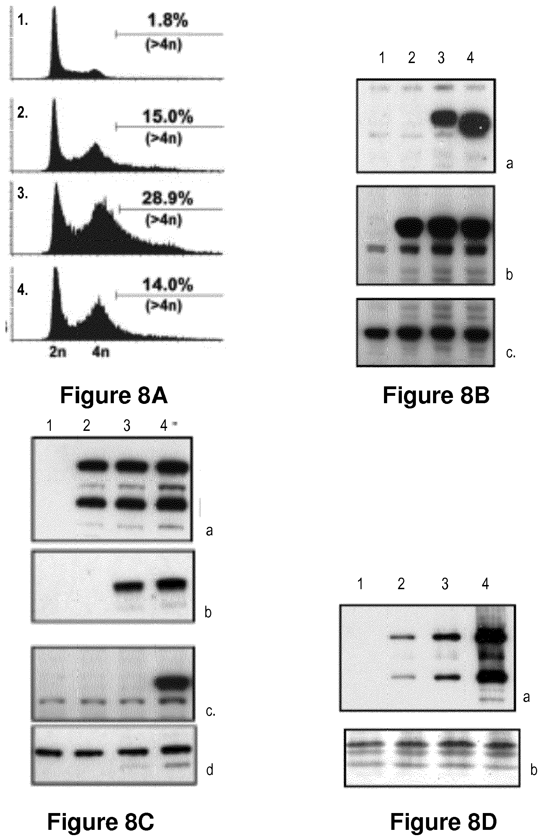

In figure 8A, it is demonstrated that Obi1 stimulates re-replication induced by Cdt1 overexpression, since the fraction of cells having DNA content >4n is enhanced when Obi1 is over expressed, said effect requiring the presence of the RING domain of Obi1. In figure 8D, it is shown that Obi1 stimulates the chromatin loading of MCM2-7 complex, as shown by the MCM2/5 amount in chromatin in cells transfected with both Obi1 and Cdt1.

### Example 6: OBI1 behaves like a proto-oncogene

Since Obi1 participate to DNA replication by inducing re-replication of DNA, the inventors have investigated the oncogenic potential of Obi1.

Figures 9 show that NIH 3T3 cells stably overexpressing Ob1 protein are able to form foci on plates and are able to form colonies when cultured in soft agar whereas the control non-transformed cells are not. Both these assays respectively demonstrate that Obi1 deregulated expression allows the loss of contact inhibition and allows the anchorage independent growth, which are hallmarks of cancer cells.

These data demonstrate that Obi1 behaves like a proto-oncogene.

### Example 7: ORC complex is polyubiquitylated via ubiquitin Lys6 linkage in vivo

Since Obi1 protein contains a RING domain, the inventors have evaluated its participation in protein ubiquitination process.

The inventors have first evaluated the ubiquitination of ORCs protein in vitro in presence of Obi1 protein. Figure 10A shows that OC1, ORC3 and ORC5 are ubiquitinated in presence of Obi1, and in particular that ORC3 and ORC5 show strong polyubiquitylation. In a second time, the inventors have demonstrated that ubiquitination is essentially carried out via Ubiquitin Lys6. Indeed, as shown in figure 10B, cells transfected with K6-only Ubiquitin mutant are note able to ubiquitinate ORC5, when transfected with Obi1.

These data demonstrate that Obi1 is a K6 polyubiquitin ligase.

In vitro experiments, shown in figure 11, demonstrate that only the full length Obi1 protein is able to polyubiquitinate ORC5.

The inventors have tested if the poly-ubiquitin ligase activity of human Obi1 protein is conserved.

As shown in figure 12, both human and xenopus Obi1, sharing 35% identity of their amino acid sequence, are able to ubiquitinate ORC5.

These results demonstrate that the activity of Obi1 on ORC polyubiquitylation is conserved toward species.

### Example 8: Obi1 is the major ORC ubiquitin ligase in vivo

To determine the importance of the Obi1 in poly-ubiquitination of ORC, the inventors have invalidated Obi1 gene by RNA interference.

Figures 13 shows that cells that does not express Obi1 are not able to poly-ubiquitinate either ORC3 or ORC5.

Obi1 is thus the major ORC ubiquitin ligase in vivo.

### Example 9 : Obi1 is required for efficient cell proliferation and efficient DNA replication.

To further evaluate the function of Obi1, the inventors have evaluated the proliferation of cells in which Obi1 expression has been inhibited by siRNAs.

Figure 14A show that cells transfected with siRNA directed against Obi1 presnt a reduced proliferation.

Moreover, figures 14C and D demonstrate that cells treated with siRNA directed against Obi1 present a large decrease of DNA synthesis, in correlation with the Obi1 interaction with the ORC complex.

### Example 10 : Obi1 is predominantly present in nucleus.

In order to determine where Obi1 protein is expressed, the inventors have transiently expressed a tagged Obi1 protein in U20S cells. Vectors comprising nucleic acid allowing the expression of a Myc-tagged Obi1 protein, or an empty vector were transiently transfected in U20S cells 48 hours post-transfection, cells were fixed in 2% paraformaldehyde/PBS, permabilized and stain using an anti-Myc mouse monoclonal antibody followed by a anti-Mouse FITC coupled antibody. Nuclei were stained using Hoescht (10 mg/ml). (Figures 15A-D) Three categories were defined: cells showing a nuclear staining with low cytoplasmic signal (N>C); cells showing an equal distribution between the nuclear and cytosolic compartment (N=C); cells having exclusive cytoplasmic signal (N<C). The inventors noted that Obi1 localisation is predominantly nuclear, with a significant proportion of cells having a strong cytoplasmic distribution (Figure 16).

### Example 11 : An artificial Obi1 protein lacking the CTD/OBD domain is a dominant negative protein.

To further understand the underlying activity of Obi1 protein during cell cycle, the inventors have developed a mutant form of Obi1 protein. Based on their findings, the inventors know that Obi1 is an homodimeric protein predominantly localized in the nucleus, where it can interact with its substrate, the ORC complex.

The inventors have developed a mutant form of Obi1 lacking the CTD/OBD domain and including a nuclear export sequence (NES), allowing the exit of the protein comprising it from the nucleus.

By fusion of the Obi1 dimerization domain (Coiled-coil) with a Nuclear Export Sequence (NES, derived from PKI), the inventors hypothesised to drive endogenous Obi1 out of the nucleus and away from its substrate. In addition, the heterodimer NES-Obi1(1-280)/Obi1 WT is inactive as it is also unable to bind to the ORC Complex. The nucleic acid molecule coding for said NES-Obi1(1-280) protein consists of the sequence as set forth in SEQ ID NO: 105. The NES-Obi1(1-280) protein consists of the amino acid sequence as set forth in SEQ ID NO : 106.

To obtain the Obi1 inhibitor Myc-NES-Obi1(1-280), a vector harboring the nuclear export sequence (NES) of the Protein Kinase A Inhibitor (PKI) was first constructed. For this, the pCS2+MT (SEQ ID NO: 107) vector was digested with NcoI/EcoRI, which removes the 6^{th} Myc tag and ligated with the following annealed oligos:
- NES_PKI_NcoBamEcoS
   CATGTCTCTGGCTCTCAAACTGGCTGGACTGGACATCGGATCCATGGTCTTG (SEQ ID NO : 108)
- NES_PKI_NcoBamEcoAS
   AATTCAAGACCATGGATCCGATGTCCAGTCCAGCCAGTTTGAGAGCCAGAGA (SEQ ID NO : 109).

This yields pCS2+MT-NES which expressed Myc-NES tagged proteins. Note that the ligated Ncol site is inactivated and a functional Ncol is reintroducted 3' of the NES sequence. Then, Obi1 ΔCTD/OBD (which is the same as Obi1(1-280)) was amplified by PCR using the cloned Obi1 cDNA as template with the following template:
Rn_1_NcoS: CTGCCATGGCTCAGACCG (SEQ ID NO: 84)
Rn_280XbaAS: CTGTCTAGATTATTTGCCATCTGCAGAAAGTG (SEQ ID NO: 87)

These primers introduced Ncol/Xbal sites at both end of the cDNA. Using Pfu polymerase (Promega) as specified by the manufacturer: The following PCR conditions were:
1) 2 min at 95 °C
2) 30 sec at 95 °C
3) 30 sec at 58 °C
4) 2 min/kb extension time at 73 °C
5) repeat step 2-4 25 times
6) 5 min at 74 °C
7) End, 4 °C

PCR products were purified (Qiaquick, Qiagen) and digested with Ncol/Xbal. The DNA fragments were subcloned into Ncol/Xbal digested pCS2+MT-NES. Restriction digestion and sequencing analysis allowed us to screen for the proper construction.

The inventors first assessed the subcellular localisation of the NES-Obi1(1-280) mutant form. U20S cells were transiently transfected with plasmids coding for Myc-tagged NES-Obi1(1-280). 24 hours post-transfection, cells were fixed in 2% paraformaldehyde/PBS, permabilized and stain using an anti-Myc mouse monoclonal antibody followed by a anti-Mouse FITC coupled antibody. Nuclei were stain using Hoescht (10 mg/ml) (Figures 17A and 17B). The position of the transfected nuclei is indicated by a white arrow in figure 17B. The localization of the ectopic protein was scored on at least 100 cells based on the nuclear:cytoplasm distribution of the signal (Figure 18). Three categories were defined: cells showing a nuclear staining with low cytoplasmic signal (N>C); cells showing an equal distribution between the nuclear and cytosolic compartment (N=C); cells having exclusive cytoplasmic signal (N<C). Note that Myc-NES-Obi1(1-280) show a strong cytoplasmic signal.

Thus, to confirm that NES-Obi1(1-280) protein still associate with endogenous Obi1 protein, the inventors have tested the interaction between the two proteins. U20S cells were transiently transfected with plasmids coding for Myc-tagged-NES-Obi1(1-280) along with Flag-Obi1 WT. 24 hours post-transfection, cells were lyzed and the soluble fraction (linput, Figure19A) was subjected to an anti-Flag immunoprecipitation (IP: Flag). Ectopic proteins were detected by western blotting (Figure 19B). Under these conditions, specific NES-Obi1(1-280) interaction with WT-Obi1 is detected in the immunoprecipitate. These results show that NES-Obi1(1-280) remains able to associate with the wild type protein.

Further, the inventors have evaluated the cellular localisation of WT-Obi1 protein when co-expressed with NES-Obi1(1-280). U20S cells were transiently transfected with plasmids coding for Flag-tagged WT Obi1 along with Myc-tagged NES-Obi1(1-280) or the empty vector. 24 hours post-transfection, cells were fixed in 2% paraformaldehyde/PBS, permabilized and stain using an anti-Flag mouse monoclonal antibody followed by a anti-Mouse FITC coupled antibody. Nuclei were stain using Hoescht (10 mg/ml). The position of the transfected nuclei is indicated by a white arrow. The localization of the ectopic protein was scored on at least 100 cells based on the nuclear:cytoplasm distribution of the signal. Three categories were defined: cells showing a nuclear staining with low cytoplasmic signal (N>C); cells showing an equal distribution between the nuclear and cytosolic compartment (N=C); cells having exclusive cytoplasmic signal (N<C). These results demonstrate that co-expression of Myc-NES-Obi1(1-280) causes Obi1 to exhibit a stronger cytoplasmic staining, indicating that Myc-NES-Obi1(1-280) acts as a dominant negative form and sequestrate WT-Obi1 protein in the cytoplasm.

Finally, the inventors evaluated the physiological effect of the Myc-NES-Obi1(1-280) expression. U20S cells were transiently transfected with plasmids coding for EGFP along with Myc-tagged NES-Obi1(1-280) or the empty vector, as indicated. 72 hours post-transfection, cells were trypsined and analyzed for fluorescence by flow cytometry (Figures 22A-C). The percentage of EGFP-posivitve (e.i. transfected) cells is indicated. These data show that expression of NES-Obi1(1-280) induces a selective loss of transfected cells, possibly by inducing cell cycle arrest or cell death.

These data also strongly suggest that a dominant negative form of Obi1 may influence cell proliferation, and in particular cell proliferation of cancer cells.

### Example 12 : Obi1 gene is mutated in patients afflicted by Meier-Gorlin syndrome.

To further evaluate the importance of Obi1 gene in regulation of DNA replication, the inventors have evaluated the possible involvement of Obi1 mutation in Meier-Gorlin syndrome patients.

Within a cohort of family having at least one member afflicted by Meier-Gorlin syndrome, the inventors have identified a patient who was heterozygous for a frameshift which lies in the last exon and would predict a truncated protein lacking the last 270 (SEQ ID NO : 100).

The mutation identified by the inventors is C.1370_1371delAA,P.GLU457VALFSX24, i.e. a deletion of the consecutive A at position 1370 and 1371 of the nucleic acid molecule as set forth in SEQ ID NO : 97, resulting in the nucleic acid molecule as set forth in SEQ ID NO: 99. The mutated mRNA expressed in the patient is the nucleic acid molecule as set forth in SEQ ID NO : 98.

The remainder of the gene is covered nicely and there doesn't appear to any second hits. The mother is a carrier for the identified mutation and there is nothing of note in the father. What is interesting is that in the patient is a query of autosomal dominant inheritance in this family, with the mother being non-penetrant, as apparently the maternal grandmother and the maternal great-grandfather look strikingly similar.

The patient's growth parameters are more mild, like Meier-Gorlin Syndrome, -3.1 Osteitis fibrosa cystica -4.4 height but with osteopenia and a few other bone abnormalities. There is nothing of note in regards to hypomorphic ears or patella.

Figures 23 represents alignment of wild-type Obi1 sequence and mutated (deleted) Obi1 sequence.

### Example 13 : Truncated Obi1 protein expressed in patient afflicted by Meier-Gorlin syndrome fails to interact with ORC1.

In order to characterize the protein expressed by the mutated allele identified in the Meier-Gorlin patient, the inventors have tested its ability to interact with the ORC complex.

U20S cells were transiently transfected with plasmids coding for Myc-tagged WT Obi1 or mutated Obi1, and also co-transfected with with plasmids coding for Flag-ORC1. 48 hours post-transfection, cells were lyzed and the soluble fraction was subjected to an anti-Flag immunoprecipitation (IP:Flag - Figure 24B). Ectopic proteins were detected by western blotting. Under these conditions, specific Obi1/ORC1 complex is detected in the immunoprecipitate. In contrast, no association is observed between ORC1 and the mutated Obi1 protein (Figure 24B).

These results demonstrate that truncated Obi1 protein does not interact with ORC, which leads to a modification in DNA replication metabolism.

The natural mutant observed in Meier-Gorlin syndrome appears to act in a similar way than the artificial dominant negative mutant NES-Obi1(1-280) described above, by interfering with the endogenous Obi1 protein activity.

## Claims

1. Composition comprising at least one of the following compounds:
- the protein consisting of the amino acid sequence as set forth in SEQ ID NO: 1,
- a protein derived from the protein as set forth in SEQ ID NO:1 by substitution of at least one nucleotide provided that said protein retains ubiquitin ligase activity and/or participates to DNA replication, said protein consisting of the amino acid sequence as set forth in SEQ ID NO: 2-27,
- a nucleic acid molecule coding for said proteins,
- a compound affecting the expression of said protein, said compound being a small interfering molecule comprising the nucleic acid sequence as set forth in SEQ ID NO: 32 to 55,
- a compound affecting the activity of said protein, said compound being
∘ a fragment of said protein provided that said fragment retains properties to interact with the origin recognition complex, said fragment consisting of the amino acid sequence SEQ ID NO: 59, SEQ ID NO: 29, SEQ ID NO: 30, SEQ ID NO: 60, SEQ ID NO: 61, SEQ ID NO: 62, SEQ ID NO: 63,SEQ ID NO: 100 or SEQ ID NO : 106, or
∘ a nucleic acid molecule coding for said fragment,
a salt or a solvate thereof,
possibly in association with a pharmaceutically acceptable carrier,
for its use for the treatment or the prevention of a human or animal pathology linked to the DNA replication.

2. Composition for its use according to claim 1, wherein said pathology linked to DNA replication is chosen among Meier-Gorlin syndrome, Seckel syndrome and Majewski osteodysplastic primordial dwarfism and wherein said composition comprises
- the protein consisting of the amino acid sequence as set forth in SEQ ID NO: 1,
- a protein derived from the protein as set forth in SEQ ID NO:1 by substitution of at least one nucleotide provided that said protein retains ubiquitin ligase activity and/or participates to DNA replication, said protein consisting of the amino acid sequence as set forth in SEQ ID NO: 2- 27, or
- a nucleic acid molecule coding for said proteins.

3. Composition for its use according to claim 1, wherein said pathology is a neoplastic disease or cancer, and wherein said composition comprises a compound inhibiting the expression or the activity of said protein as defined in claim 1.

4. Composition for its use according to claims 1 or 2, wherein said nucleic acid molecule comprises the nucleic acid sequence SEQ ID NO: 31, said nucleic acid molecule being possibly contained in a vector, said vector comprising means allowing the expression of said nucleic acid molecule.

5. Nucleic acid molecule inhibiting the expression of the OBI1 gene coding for a protein as set forth in SEQ ID NO: 1 by RNA interference comprising one of the following sequences: SEQ ID NO : 32 to 55, or a combination of said nucleic acid molecule, for its use for inhibiting *in vitro* cell proliferation.

6. Fragment of the OBI1 protein consisting of the sequence as set forth in SEQ ID NO
: 1, **characterized in that** it consists of the amino acid sequence: SEQ ID NO: 29, SEQ ID NO 30, SEQ ID NO: 59, SEQ ID NO: 60, SEQ ID NO: 61, SEQ ID NO: 62, SEQ ID NO: 63, SEQ ID NO: 100 and SEQ ID NO: 106, in particular for inhibiting *in vitro* cell proliferation.

7. Use of a protein consisting of an amino acid sequence as set forth in SEQ ID NO: 1, or a protein derived from the protein as set forth in SEQ ID NO:1 by substitution of at least one nucleotide provided that said protein retains ubiquitin ligase activity and/or participates to DNA replication, said protein consisting of the amino acid sequence as set forth in SEQ ID NO: 2- 27, as Lys6ubiquitine ligase enzyme.

8. Process for purifying a protein consisting of the amino acid sequence SEQ ID NO: 1, or a protein derived from the protein as set forth in SEQ ID NO:1 by substitution, deletion, or insertion of at least one nucleotide provided that this protein retains ubiquitin ligase activity and/or participate to DNA replication, said protein consisting of the amino acid sequence as set forth in SEQ ID NO: 2- 27, said process comprising :
- a step of purifying either the ORC1 protein or the LRWD1 protein, and
- a step of isolating the OBI1 protein.

9. Method for diagnosing *in vitro* or *ex vivo* Meier-Gorlin syndrome comprising
a. a step of identifying the sequence of the Obi1 gene coding for a protein as set forth in SEQ ID NO: 1, in cells originating from an individual,
b. a step of comparing said sequence with a reference sequence as set forth in SEQ ID NO: 97 to determine if a mutation has occurred in the sequence of said Obi1 gene in said cells originating from said individual, and
c. a step of determining if said mutation affects the biological function of the protein coded by said Obi1 gene in said cells originating from said individual.

## Patentansprüche

1. Zusammensetzung, mindestens eine der folgenden Verbindungen umfassend:
- das Protein bestehend aus der Aminosäuresequenz, wie in SEQ ID NO: 1 dargelegt,
- ein Protein, das von dem in SEQ ID NO:1 dargelegten Protein durch Substitution von mindestens einem Nukleotid abgeleitet ist, vorausgesetzt, dass das besagte Protein Ubiquitin-Ligase-Aktivität beibehält und/oder an der DNA-Replikation beteiligt ist, wobei das besagte Protein aus der Aminosäuresequenz wie in SEQ ID NO: 2-27 dargelegt, besteht,
- ein Nukleinsäuremolekül, das für die besagten Proteine kodiert,
- eine Verbindung, welche die Expression des besagten Proteins beeinflusst, wobei die besagte Verbindung ein kleines, interferierendes Molekül ist, umfassend die Nukleinsäuresequenz, wie in SEQ ID NO: 32 bis 55 dargelegt,
- eine Verbindung, welche die Aktivität des besagten Proteins beeinflusst, wobei die besagte Verbindung
o ein Fragment des besagten Proteins ist, vorausgesetzt, dass das besagte Fragment Eigenschaften beibehält, um mit dem Ursprungserkennungskomplex zu interagieren, wobei das Fragment aus der Aminosäuresequenz SEQ ID NO: 59, SEQ ID NO: 29, SEQ ID NO: 30. SEQ ID NO: 60, SEQ ID NO: 61, SEQ ID NO: 62, SEQ ID NO: 63, SEQ ID NO: 100 oder SEQ ID NO: 106 besteht oder
o ein Nukleinsäuremolekül ist, das für das besagte Fragment kodiert,
ein Salz oder Solvat davon,
möglicherweise in Verbindung mit einem pharmazeutisch annehmbaren Träger, zur Verwendung zur Behandlung oder Vorbeugung einer humanen oder tierischen Pathologie, die mit der DNA-Replikation verbunden ist.

2. Zusammensetzung zur Verwendung gemäß Anspruch 1, wobei die besagte Pathologie, die mit der DNA-Replikation verbunden ist, aus dem Meier-Gorlin-Syndrom, dem Seckel-Syndrom und dem Majewski osteodysplastischen primordialen Kleinwuchs ausgewählt ist und wobei die Zusammensetzung umfasst
- das Protein, bestehend aus der Aminosäuresequenz wie in SEQ ID NO: 1 dargelegt,
- ein Protein, das von dem in SEQ ID NO:1 dargelegten Protein durch Substitution von mindestens einem Nukleotid abgeleitet ist, vorausgesetzt, dass das besagte Protein Ubiquitin-Ligase-Aktivität beibehält und/oder an der DNA-Replikation beteiligt ist, wobei das besagte Protein aus der Aminosäuresequenz besteht, wie in SEQ ID NO: 2- 27 dargelegt, oder
- ein Nukleinsäuremolekül, das für die besagten Proteine kodiert.

3. Zusammensetzung zur Verwendung gemäß Anspruch 1, wobei die besagte Pathologie eine neoplastische Erkrankung oder Krebs ist, und wobei die besagte Zusammensetzung eine Verbindung umfasst, welche die Expression oder die Aktivität des besagten Proteins wie in Anspruch 1 definiert, inhibiert.

4. Zusammensetzung zur Verwendung gemäß Anspruch 1 oder 2, wobei das besagte Nukleinsäuremolekül die Nukleinsäuresequenz SEQ ID NO: 31 umfasst, wobei das besagte Nukleinsäuremolekül möglicherweise in einem Vektor enthalten ist, wobei der besagte Vektor Mittel umfasst, welche die Expression des besagten Nukleinsäuremoleküls ermöglichen.

5. Nukleinsäuremolekül, das die Expression des OBI1-Gens, das für ein Protein, wie in SEQ ID NO: 1 dargelegt, kodiert, durch RNA-Interferenz hemmt, umfassend eine der folgenden Sequenzen: SEQ ID NO: 32 bis 55, oder eine Kombination des besagten Nukleinsäuremolekül, zur Verwendung zur Inhibition der *in vitro* Zellproliferation.

6. Fragment des OBI1-Proteins, bestehend aus der Sequenz, wie in SEQ ID NO : 1 dargelegt, **dadurch gekennzeichnet, dass** es aus der Aminosäuresequenz besteht: SEQ ID NO: 29, SEQ ID NO 30, SEQ ID NO: 59, SEQ ID NO: 60, SEQ ID NO: 61, SEQ ID NO: 62, SEQ ID NO: 63, SEQ ID NO: 100 und SEQ ID NO: 106, insbesondere zur Inhibition der *in vitro* Zellproliferation.

7. Verwendung eines Proteins, das aus einer Aminosäuresequenz besteht, wie in SEQ ID NO: 1 dargelegt, oder eines Proteins, das von dem in SEQ ID NO:1 dargelegten Protein durch Substitution von mindestens einem Nukleotid abgeleitet ist, vorausgesetzt, dass das besagte Protein Ubiquitin-Ligase-Aktivität beibehält, und/oder an der DNA-Replikation beteiligt ist, wobei das Protein aus der Aminosäuresequenz besteht, wie in SEQ ID NO: 2- 27 dargelegt, als Lys6-Ubiquitin-Ligase-Enzym.

8. Verfahren zur Aufreinigung eines Proteins, bestehend aus der Aminosäuresequenz SEQ ID NO: 1 oder eines Proteins, das von dem in SEQ ID NO: 1 dargelegten Protein durch Substitution, Deletion oder Insertion mindestens eines Nukleotids abgeleitet ist, vorausgesetzt, dass dieses Protein Ubiquitin-Ligase-Aktivität beibehält und/oder an der DNA-Replikation beteiligt ist, das besagte Protein aus der Aminosäuresequenz, wie in SEQ ID NO: 2- 27 dargelegt, besteht, das besagte Verfahren umfassend:
- einen Schritt zur Aufreinigung entweder des ORC1-Proteins oder des LRWD1-Proteins, und
- einen Schritt zur Isolierung des OBI1-Proteins.

9. Verfahren zur *in vitro* oder *ex vivo* Diagnose des Meier-Gorlin-Syndroms, umfassend
a. einen Schritt des Identifizierens der Sequenz des Obi1-Gens, das für ein Protein wie in SEQ ID NO: 1 dargelegt kodiert in Zellen, die von einem Individuum stammen,
b. einen Schritt des Vergleichens der besagten Sequenz mit einer Referenzsequenz, wie in SEQ ID NO: 97 dargelegt, um zu bestimmen, ob eine Mutation in der Sequenz des besagten Obi1-Gens in den besagten Zellen, die von dem besagten Individuum stammen aufgetreten ist, und
c. einen Schritt des Bestimmens, ob die besagte Mutation die biologische Funktion des Proteins, das durch das besagte Obi1-Gen kodiert wird, die in den besagten Zellen von dem besagten Individuum stammen, beeinträchtigt.

## Revendications

1. Composition comprenant au moins l'un des composés suivants :
- La protéine consistant en la séquence d'acides aminés SEQ ID NO : 1,
- une protéine dérivée de la protéine SEQ ID NO:1 par substitution d'au moins un by substitution of at least one acide aminés sous réserve que ladite protéine conserve une activité ubiquitine ligase et/ou participe à la réplication de l'ADN, ladite protéine consistant en la séquence d'acides aminés SEQ ID NO : 2 à 27,
- une molécule d'acides nucléiques codant lesdites protéines,
- un compose affectant l'expression desdites protéines, ledit compose étant une petite molécule interférente comprenant la séquence d'acides nucléiques SEQ ID NO: 32 à 55,
- un composé affectant l'activité de ladite protéine, ladite protéine étant :
o un fragment de ladite protéine sous réserve que ledit fragment conserve des propriétés d'interaction avec le complexe de reconnaissance des origines, ledit fragment consistant en la séquence d'acide SEQ ID NO : 59, SEQ ID NO : 29, SEQ ID NO : 30, SEQ ID NO : 60, SEQ ID NO : 61, SEQ ID NO : 62, SEQ ID NO : 63,SEQ ID NO : 100 ou SEQ ID NO : 106, ou
o une molécule d'acides nucléiques codant ledit fragment,
un sel ou un solvate de ceux-ci,
éventuellement en association avec un véhicule pharmaceutiquement acceptable,
pour son utilisation pour le traitement ou la prévention d'une pathologie humaine ou animale liée à la réplication de l'ADN.

2. Composition pour son utilisation selon la revendication 1, où ladite pathologie liée à la réplication de l'ADN est choisie parmi le syndrome de Meier-Gorlin, le syndrome de Seckel et le nanisme ostéodysplastic primordial de Majewski et ou ladite composition comprend
- la protéine consistant en la séquence d'acides aminés SEQ ID NO : 1,
- une protéine dérivée de la protéine SEQ ID NO :1 par substitution d'au moins acide aminé sous réserve que ladite protéine conserve une activité ubiquitine ligase et/ou participe à la réplication de l'ADN, ladite protéine consistant en la séquence d'acides aminés SEQ ID NO : 2 à 27, ou
- une molécule d'acides nucléiques codant lesdites protéines.

3. Composition pour son utilisation selon la revendication 1, ou ladite pathologie est une maladie néoplasique ou un cancer, et ou ladite composition comprend un compose inhibant l'expression ou l'activité de la protéine telle que définie dans la revendication 1.

4. Composition pour son utilisation selon la revendication 1 ou 2, ou ladite molécule d'acides nucléiques comprend la séquence d'acides nucléiques SEQ ID NO : 31, ladite séquence d'acides nucléiques étant éventuellement contenue dans un vecteur, ledit vecteur comprenant des moyens permettant l'expression de ladite molécule d'acides nucléiques.

5. Molécule d'acides nucléiques inhibant l'expression du gène OBI1 codant la protéine EQ ID NO : 1 par interférence à ARN comprenant une des séquences suivantes SEQ ID NO : 32 to 55, ou une combinaison desdites molécules d'acid nucléiques, pour son utilisation pour l'inhibition in vitro de la prolifération cellulaire.

6. Fragment de la protéine OBI1 consistant en la séquence SEQ ID NO : 1, **caractérisé en ce qu'**il consiste en la séquence d'acides aminés SEQ ID NO : 29, SEQ ID NO : 30, SEQ ID NO : 59, SEQ ID NO : 60, SEQ ID NO : 61, SEQ ID NO : 62, SEQ ID NO : 63, SEQ ID NO : 100 et SEQ ID NO : 106, en particulier pour son utilisation pour inhiber in vitro la prolifération cellulaire.

7. Utilisation d'une protéine consistant en la séquence d'acides aminés SEQ ID NO : 1, ou une protéine dérivée de la protéine SEQ ID NO :1 par substitution d'au moins acide aminé sous réserve que ladite protéine conservé une activité ubiquitine ligase et/ou participe à la réplication de l'ADN, ladite protéine consistant en la séquence d'acides SEQ ID NO : 2 à 27, en tant qu'enzyme Lys6ubiquitine ligase.

8. Procédé de purification d'une protéine consistant en la séquence d'acides aminés SEQ ID NO : 1, ou une protéine dérivée de la protéine SEQ ID NO:1 par substitution d'au moins acide aminé sous réserve que ladite protéine conservé une activité ubiquitine ligase et/ou participe à la réplication de l'ADN, ladite protéine consistant en la séquence d'acides SEQ ID NO : 2 à 27, ledit procédé comprenant:
- Une étape de purification soit de la protéine ORC1 ou la protéine LRWD1, et
- Une étape d'isolement de la protéine OBI1.

9. Méthode de diagnostic in vitro ou ex vivo du syndrome de Meier-Gorlin comprenant :
a. Une étape d'identification de la séquence du gène Obi1 codant la protéine SEQ ID NO : 1, dans des cellules issues d'un individu,
b. Une étape de comparaison de ladite séquence avec une séquence de référence SEQ ID NO : 97 afin de déterminer si une mutation est survenue dans la séquence du gène Obi1 dans lesdites cellules issues dudit individu, et
c. Une étape de détermination si ladite mutation affecte la fonction biologique de la protéine codée par le gène dans lesdites cellules issues dudit individu.
